# EUROPEAN PATENT APPLICATION

(11) **EP 4 759 323 A2**
(43) Date of publication of application: **17.06.2026**
(21) Application number: 26176049.0
(22) Date of filing: 17.05.2022
(51) Int. Cl.: A61K 38/16

(54) **VASOACTIVE INTESTINAL PEPTIDE (VIP) RECEPTOR ANTAGONISTS**

(30) Priority: 17.05.2021 US 202163189507 P
(62) Divisional of application: 22805312.0
(71) Applicant: Emory University, Atlanta, GA 30322 (US); Cambium Oncology LLC, Atlanta, Georgia 30306-3602 (US)
(72) Inventor: WALLER, Edmund K., Atlanta, Georgia, 30322 (US); LI, Jian-Ming, Atlanta, Georgia, 30340 (US); FNU, Passang Tenzin, Atlanta, Georgia, 30322 (US); RAVINDRANATHAN, Sruthi, Atlanta, Georgia, 30322 (US); MAJUMDAR, Anish Sen, Atlanta, Georgia, 30322 (US)
(74) Representative: Dehns

(57) **Abstract**

Disclosed are VIP-R antagonists for uses in managing the treatment or prevention of cancer and viral infections. In certain embodiments, this disclosure relates to chimeric variants of VIP-R antagonists, as peptides disclosed herein, and pharmaceutical composition comprising the same. In certain embodiments, this disclosure contemplates methods of treating subjects with cancer or infection with VIP-R antagonists or stimulating immune cells to target cancer by mixing immune cells in vitro with peptides disclosed herein and further administering an effective amount of stimulated immune cells to a subject in need of cancer treatment.

## Description

### I. CROSS REFERENCE TO RELATED APPLICATIONS

1. This application claims the priority benefit of U.S. Provisional Application No. 63/189,507, filed May 17, 2021, which is expressly incorporated herein by reference in its entirety.

### II. BACKGROUND

2. Vasoactive intestinal peptide (VIP) is produced in a variety of cells, including immune cells, neurons, and endocrine cells in the central nervous system. Endogenous VIP is present in nerves innervating smooth muscles of airway and pulmonary vessels within the lung, and VIP functions as a bronchodilator in this organ. VIP also can alter cellular proliferation and the production of inflammatory signals through the VIP receptors VPAC1 and VPAC2. A chimeric peptide, referred to as VIPhyb, was developed with the six N-terminal amino acids of native VIP replaced with six highly polar N-terminal six amino acids from the sequence of the neurotensin peptide followed by the C-terminal 22 amino acid sequence of VIP. With changes in the N-terminal amino acids, VIPhyb has altered biological activity compared with VIP and acts as an antagonist to the VIP receptor (VIP-R) by competitively binding to the receptor but not signaling. .

3. Cancer treatments typically utilize surgery, chemotherapy, and radiation therapy. However, alternative methods of treatment that strengthen the immune system to attack cancerous cells are reported. These methods include collecting, amplifying, and altering T cells in order to target and stimulate the immune system to aggressively eliminate cancerous cells. In chimeric antigen receptor (CAR) T cell therapy, isolated T cell are engineered to express chimeric protein and are administered back into the patient. However, there is a need to identify therapies that improve the functional properties of T cells.

### III. SUMMARY

4. Disclosed are methods and compositions related to vasoactive intestinal peptide (VIP) receptor antagonists for uses in managing the treatment or prevention of cancer and viral infections. In certain embodiments, this disclosure relates to chimeric variants of VIP-R antagonists, as peptides disclosed herein, and pharmaceutical composition comprising the same. In certain embodiments, this disclosure contemplates methods of stimulating immune cells to target cancer by mixing immune cells in vitro with peptides disclosed herein and further administering an effective amount of stimulated immune cells to a subject in need of cancer treatment.

5. In one aspect disclosed herein are vasoactive intestinal peptide (VIP) receptor antagonists comprising the amino acid sequence KPRRPYX¹X²X³X⁴TX⁵LRKQX⁶AVX⁷X⁸KYLX⁹X¹⁰ILN (SEQ ID NO: 3), wherein X¹ is T or A; X² is D, V, or S; X³ is N or D; X⁴ is Y or C; X⁵ R or S; X⁶ M or I; X⁷ is K or N; X⁸ is K or absent, X⁹ is N or M; and X¹⁰ is S or L; and provided that the peptide is not KPRRPYTDNYTRLRKQMAVKKYLNSILN (SEQ ID NO: 1) or the combination wherein X¹ is T, X² is D, X³ is N; X⁴ is Y, X⁵ is R, X⁶ is M, X⁷ is K, X⁹ is N, and X¹⁰ is S. In one aspect disclosed herein are vasoactive intestinal peptide (VIP) receptor antagonists comprising the amino acid sequence KPRRPYX¹X²X³X⁴TX⁵LRKQX⁶AVX⁷KYLX⁸X⁹ILN (SEQ ID NO: 21), wherein X¹ is T or A; X² is D, V, or S; X³ is N or D; X⁴ is Y or C; X⁵ R or S; X⁶ M or I; X⁷ is K or N; X⁸ is N or M; and X⁹ is S or L; and provided that the peptide is not KPRRPYTDNYTRLRKQMAVKKYLNSILN (SEQ ID NO: 1) or the combination wherein X¹ is T, X² is D, X³ is N; X⁴ is Y, X⁵ is R, X⁶ is M, X⁷ is K, X⁸ is N, and X⁹ is S. For example, disclosed herein are VIP-R antagonist comprising the amino acid sequence KPRRPYADNYTRLRKQMAVNKYLNLILN (SEQ ID NO: 6), KPRRPYAVNYTRLRKQIAVKKYLMSILN (SEQ ID NO: 7), KPRRPYAVNYTRLRKQMAVNKYLMSILN (SEQ ID NO: 8), KPRRPYADNCTRLRKQIAVNKKYLNSILN (SEQ ID NO: 9), KPRRPYTVNYTSLRKQIAVKKYLMLILN (SEQ ID NO: 10), KPRRPYTDNCTSLRKQIAVNKYLNLILN (SEQ ID NO: 11), KPRRPYAVNCTSLRKQIAVNKYLNSILN (SEQ ID NO: 12), KPRRPYAVNCTSLRKQIAVKKYLMSILN (SEQ ID NO: 13), KPRRPYTVNCTSLRKQIAVKKYLMLILN (SEQ ID NO: 14), KPRRPYTSDYTRLRKQMAVKKYLNSILN (SEQ ID NO: 15), KPRRPYTSDYTRLRKQMAVKKYLNLILN (SEQ ID NO: 16), a fragment thereof, or an analog thereof.

6. Also, disclosed herein are VIP-R antagonists of any preceding aspect, wherein an amino, carboxyl, hydroxyl, or thiol group in the VIP-R antagonist is substituted.

7. In some aspects, the VIP-R antagonist is conjugated to and/or encapsulated within a nanoparticle.

8. Also disclosed herein are VIP-R antagonists of any preceding aspect wherein the VIP-R antagonist further comprises a label, e.g., fluorescent or radioactive.

9. In one aspect, disclosed herein are pharmaceutical compositions comprising the VIP-R antagonist of any preceding aspect and a pharmaceutically acceptable carrier.

10. In one aspect, disclosed herein are nucleic acids encoding the VIP-R antagonists of any preceding aspect. Also disclosed herein are recombinant vectors comprising said nucleic acids. In one aspect, disclosed herein are expression systems or cells comprising a recombinant vector of any preceding aspect.

11. Also disclosed herein are methods of treating, decreasing, inhibiting, reducing, ameliorating, and/or preventing a cancer and/or metastasis in a subject or enhancing the immune response to cancer and/or metastasis in a subject comprising administering to the subject a therapeutically effective amount of the VIP-R antagonist (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or pharmaceutical composition of any preceding aspect. In certain embodiments, the VIP-R antagonist or the pharmaceutical composition is administered in combination with another anti-cancer agent. In some aspect, the method can further comprise exposing the subject to radiation and/or transplanting allogeneic hematopoietic stem cells into the subject and/or other adoptive cellular therapies (such as, for example, administration of CAR T cells, TCR Modified T Cells, CAR NK cells, TILs, TINKs, and/or MILs). In certain embodiments, the method further comprises administering to the subject a therapeutically effective amount of a phosphatidylinositol 3-kinase (PI3K) inhibitor (including, for example, a PI3Kα inhibitor, a PI3Kβ inhibitor, a PI3Kδ inhibitor, or a PI3Kγ inhibitor). In certain embodiments, the method further comprises administering to the subject a therapeutically effective amount of an immune checkpoint blockade. In some embodiments, the immune checkpoint blockade is a PD-1 inhibitor, a PD-L1 inhibitor, or a CTLA-4 inhibitor.

12. In certain embodiments, this disclosure relates to methods of *ex vivo* augmenting T cell activation and/or expansion comprising mixing T cells with a VIP-R antagonist of any preceding aspect (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof). In certain embodiments, mixing T cells is in combination with an anti-CD3 antibody and/or anti-CD28 antibody. In certain embodiments, the mixing T cells is in combination with a phosphatidylinositol 3-kinase (PI3K) inhibitor (including, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib)). In certain embodiments, the mixing T cells is in combination with an immune checkpoint blockade. In some embodiments, the immune checkpoint blockade is a PD-1 inhibitor, a PD-L1 inhibitor, or a CTLA-4 inhibitor.

13. Also disclosed herein is a kit comprising the VIP-R antagonist of any preceding aspect (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or the pharmaceutical composition of any preceding aspect and an anti-CD3 antibody and/or anti-CD28 antibody. In certain embodiments, the kit further comprises a phosphatidylinositol 3-kinase (PI3K) inhibitor (including, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib)). In certain embodiments, the kit further comprises an immune checkpoint blockade. In some embodiments, the immune checkpoint blockade is a PD-1 inhibitor, a PD-L1 inhibitor, or a CTLA-4 inhibitor.

14. Also disclosed herein is an *in vitro* cell culture composition, comprising one or more T cells and the VIP-R antagonist of any preceding aspect (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or the pharmaceutical composition of any preceding aspect. In some embodiments, the *in vitro* cell culture composition further comprises an anti-CD3 antibody and/or anti-CD28 antibody. In certain embodiments, the *in vitro* cell culture composition further comprises a phosphatidylinositol 3-kinase (PI3K) inhibitor (including, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib)). In certain embodiments, the *in vitro* cell culture composition further comprises an immune checkpoint blockade. In some embodiments, the immune checkpoint blockade is a PD-1 inhibitor, a PD-L1 inhibitor, or a CTLA-4 inhibitor.

15. In certain embodiments, this disclosure relates to methods of treating or preventing graft versus host disease in a subject comprising administering an effective amount of any VIP-R antagonist of any preceding aspect (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, or a fragment thereof) to a subject that is to receive or received transplanted allogeneic tissue or cells.

16. In certain embodiments, this disclosure relates to methods of treating, reducing, inhibiting, decreasing, ameliorating, managing, and/or preventing a microbial infection (including, but not limited to viral, bacterial, fungal, and/or parasitic infections) comprising administering to a subject infected with a microbe or at risk for a microbial infection a therapeutically effective amount of the VIP-R antagonist of any preceding aspect (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or the pharmaceutical composition of any preceding aspect.

17. Also disclosed herein is a method of treating a cancer or a chronic infection in a subject in need, comprising providing one or more T cells; mixing the one or more T cells with the VIP-R antagonist (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or the pharmaceutical composition of any preceding aspect thereby expanding the one or more T cells; and administering a therapeutically effective amount of the expanded T cells to the subject

18. In some embodiments, the method of any preceding aspect comprises mixing the one or more T cells is in combination with an anti-CD3 antibody and/or an anti-CD28 antibody. In some embodiments, the method of any preceding aspect comprises mixing the one or more T cells is in combination with an immune checkpoint blockade. In some embodiment, the method of any preceding aspect comprises mixing one or more T cells in combination with a phosphatidylinositol 3-kinase (PI3K) inhibitor (including, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib)).

19. In some embodiments, the method of any preceding aspect further comprises administering to the subject a PI3 kinase inhibitor, a VIP receptor antagonist, or an immune checkpoint blockade, or a combination thereof before, during, or after administering the expanded T cells. In some embodiments, the one or more T cells are derived from the subject. In some embodiments, the one or more T cells are engineered T cells. In some embodiments, the one or more T cells comprises a chimeric antigen receptor.

### IV. BRIEF DESCRIPTION OF THE DRAWINGS

20. The accompanying drawings, which are incorporated in and constitute a part of this specification, illustrate several embodiments and together with the description illustrate the disclosed compositions and methods.

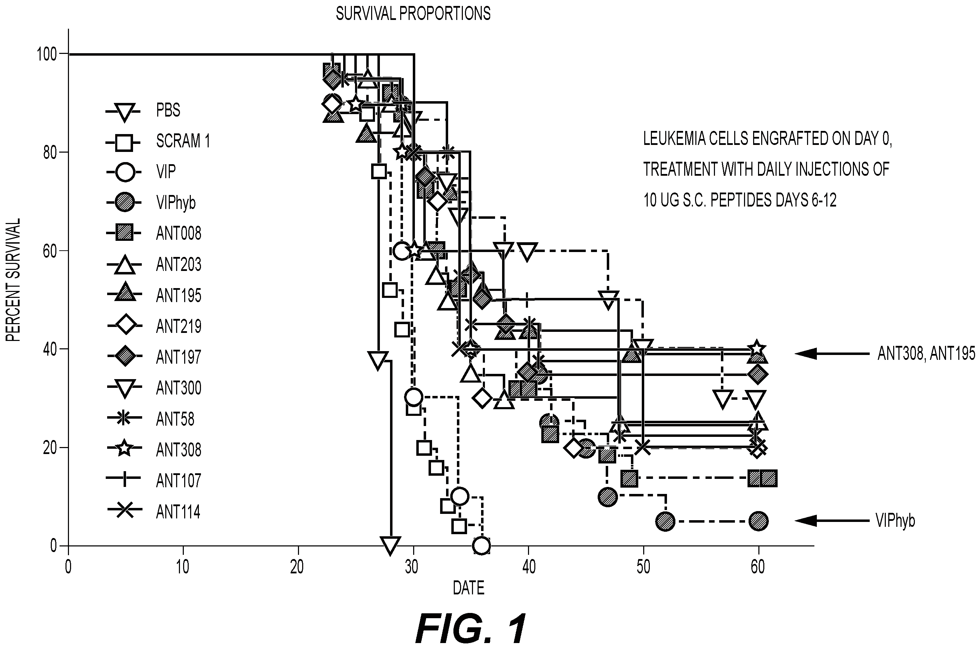

21. Figure 1 shows treatment with the VIP-derived novel peptides prolonged survival of mice engrafted with acute myeloid leukemia. B6 (CD45.2, H-2K^{b}) mice were administered C1498 1 x 10⁶ /mouse through tail vein. VIP novel peptides were subcutaneously injected 10 microgram per mouse daily from day 6 following leukemia administration, totally 7 doses. Survival of the mice was observed daily. Data were pooled from 3 replicated experiments.

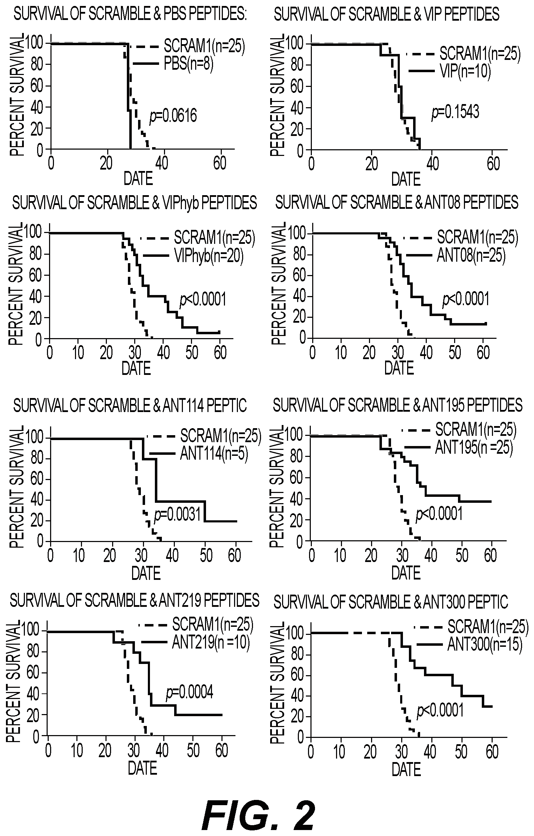

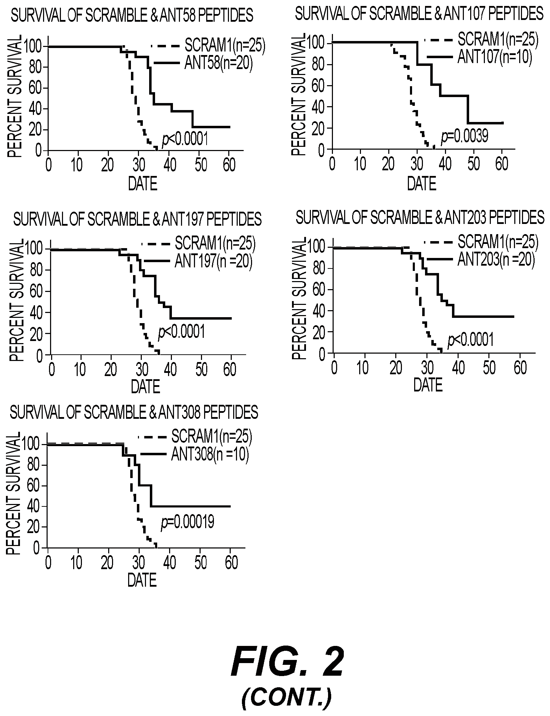

22. Figure 2 shows treatment with the VIP-derived novel peptides prolonged survival of the leukemic mice. B6 (CD45.2, H-2K^{b}) mice were administered C1498 1 x 10⁶ /mouse through tail vein. VIP novel peptides were subcutaneously injected 10 microgram per mouse daily from day6 following leukemia administration, totally 7 doses. Survival of the mice was observed daily. Survival of the mice were pooled from 3 replicated experiments and analyzed by log-rank test compared with the survival of mice treated with scrambled peptide.

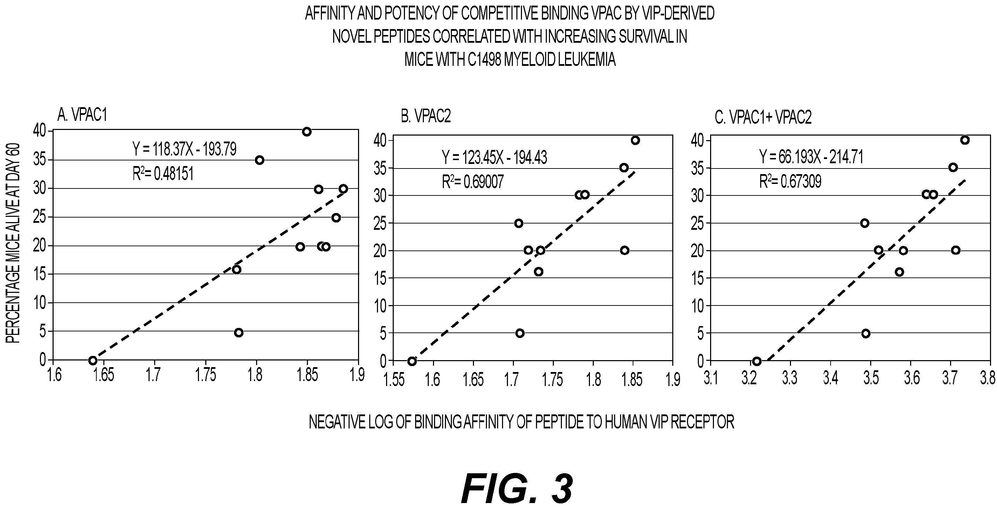

23. Figure 3 shows predicted affinity and potency of competitive binding VPAC by VIP-derived novel peptides correlated with increasing percentages of survival in leukemia-bearing mice treated with VIP-derived novel peptides. We make a logarithm of the sum of the absolute values of the predicted binding affinity to human VPAC1 and VPAC2 for each of the distinct VIP novel peptides and then plot logarithm of absolute value of binding affinity against respective percentages of survival. Panel A: Correlation of survival with the logarithm of the absolute value of the predicted binding affinity to human VPAC1, (logarithm of absolute value of binding affinity for VPAC1), [percentage of survival]. ANT293(scramb) (1.64) [0], ANT08 (1.78) [16], VIPhyb (1.782) [5], ANT197 (1.803) [35], ANT219 (1.841)[20], ANT195 (1.848)[40], ANT107 (1.864) [20], ANT114 (1.867) [20], ANT203 (1.877)[25]. ANT58 (1.883)[30), Panel B: Correlation of survival with the logarithm of the absolute value of the predicted binding affinity to human VPAC2. (logarithm of absolute value of binding affinity for VPAC2) [percentage of survival]. ANT293(scramb) (1.573)[0], ANT203 (1.707)[25], VIPhyb (1.708) [5], ANT107 (1.719)[20], ANT08 (1.732)[16], ANT114 (1.734)[20], ANT58 (1.782) [30], ANT197 (1.839) [35], ANT219 (1.839)[20], ANT195 1.854 [40] Panel C: shows the logarithms of the absolute value of the sum of binding affinities to VPAC1 and VPAC2 plotted against respective survival percentages in leukemic mice treated with each VIP-derived novel peptide.

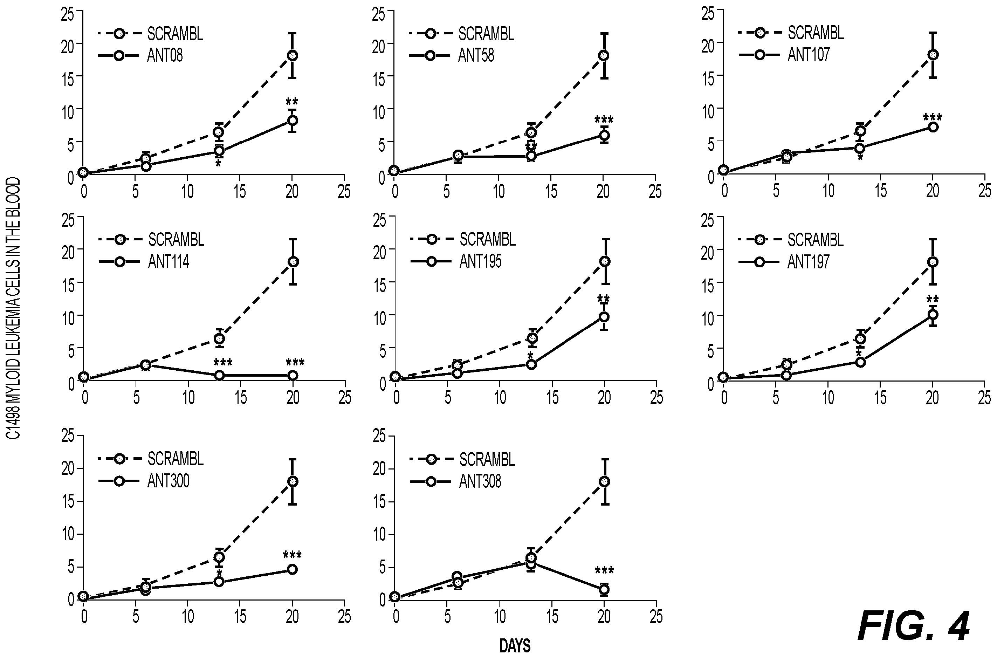

24. Figure 4 shows treatment with VIP derived novel peptides reduced levels of leukemia cells in blood of the leukemic mice. B6 SJL (CD45.1, H-2K^{b}) mice were administered C1498 (CD45.2 H-2K^{b})1 x 10⁶ /mouse through tail vein. VIP novel peptides were subcutaneously injected 10 microgram per mouse daily from day6 following leukemia administration, totally 7 doses. The mice were bled weekly from the submandibular vein, starting from day 6, prior to the administration of peptides. Antibodies to CD45.2 identified myeloid leukemia cells and antibodies to CD45.1 identified murine leukocytes of the host.

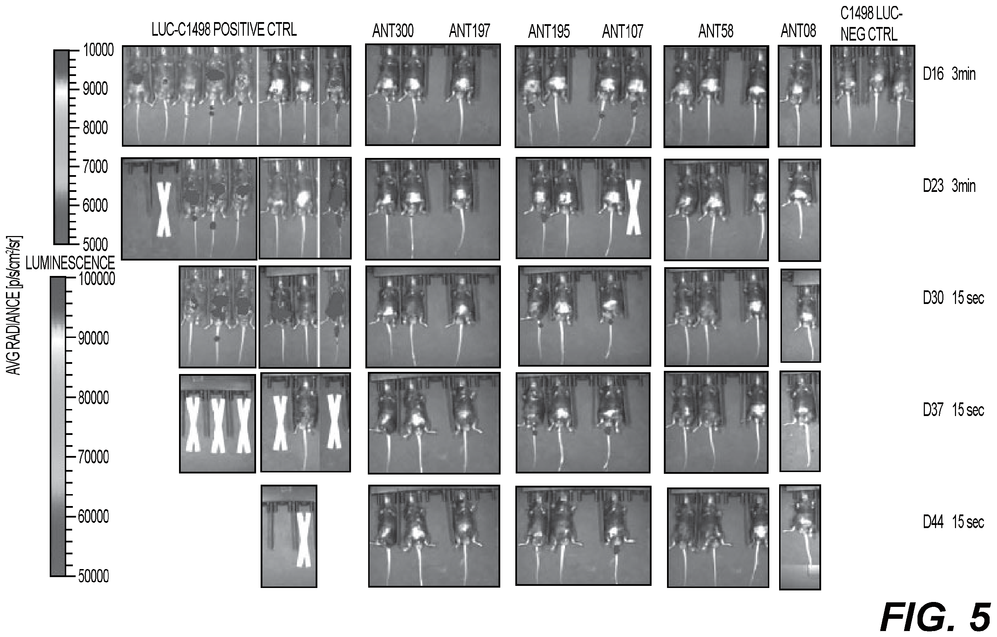

25. Figure 5 shows that mice treated with the VIP-derived novel peptides had a prolonged survival from re-challenged with myeloid leukemia cells. B6 mice were administered either Luc-1498 1 x 10⁶ /mouse for Luc-C198 positive control or C1498 1 x 10⁶ /mouse for C1498 (luciferase) negative control through tail vein. Tumor-free residual survival mice after day 68 were re-challenged with Luc-1498 1 x 10⁶ /mouse through tail vein. 16 days following leukemia re-challenge, the mice were luminescence-imaged bi-weekly. The image exposure time were 15 seconds except 3 minutes at day16 and day23. Data were showed with Average Radiance [p/s/cm²/sr]. Dead mice are signified by the white X.

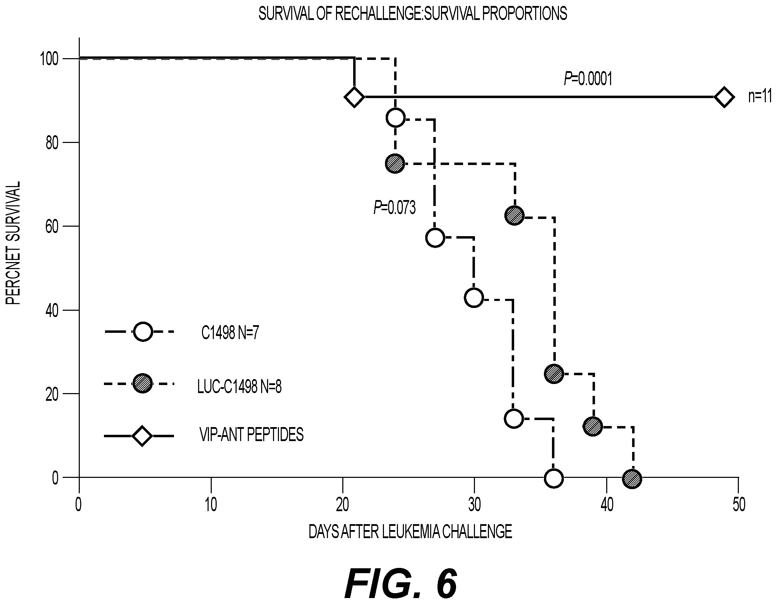

26. Figure 6 shows aggregate data from mice treated with the VIP-derived novel peptides had a prolonged survival from re-challenge with acute myeloid leukemia. B6 mice were administered either Luc-1498 1 x 10⁶ /mouse for Luc-C198 positive control or C1498 1 x 10⁶ /mouse for C1498 (luciferase-negative) positive-control through the tail vein. Tumor-free mice that had been previously inoculated with C1498, treated with novel VIP-R antagonist peptides and remained cancer-free for more than 60 days, were re-challenged with Luc-C1498. Survival of the mice was measured daily. The eleven mice that had survived following initial inoculation with C1498 leukemia and then treatment with a VIP-derived novel peptide were pooled as follows: ANT08 one mouse, ANT58 three mice, ANT107 two mice, ANT195 two mice, ANT197 one mouse, ANT300 two mice. Luciferase positive C1498 was administered to eight control mice that had not previously been exposed to leukemia or VIP-derived novel peptides (Luc-C1498 control), and luciferase-negative C1498 was administered to seven control mice that had not previously been exposed to leukemia or VIP-derived novel peptides (C1498 control).

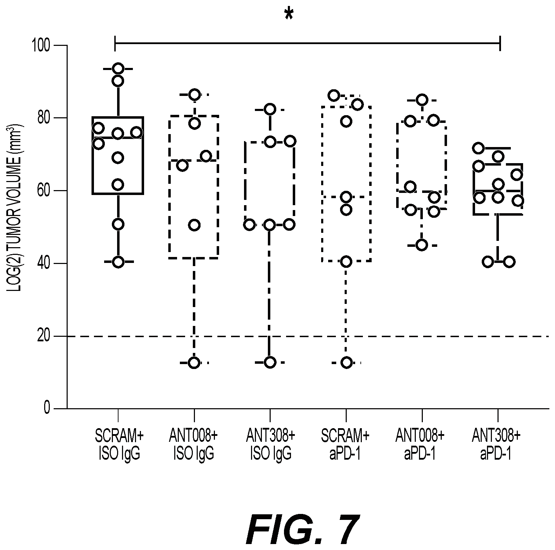

27. Figure 7 shows decreased tumor volume upon ANT308+aPD-1 treatment of C57BL/6 mice with subcutaneously implanted KPC tumors. Day 22 after tumor implantation, 3 days after completing a 10-day course of treatment. *p<0.05 Wilcoxon signed rank test

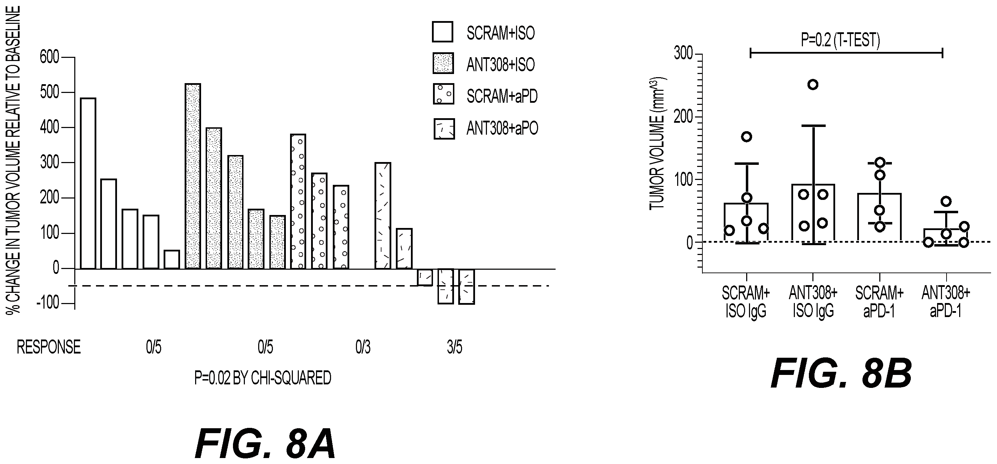

28. Figures 8A shows relative changes differences in the volume of Panc02 tumors growing as a sub-cutaneous tumor, from the time that treatment was initiated on day 10 post-implantation to euthanasia on day 22 post-implantation, following 10 days of treatment with daily injections of ANT308 or scrambled peptide and injection every 3 days of anti-PD1 monoclonal antibody or iso-type matched antibody, from day 10 through day 19 post-implantation. Mice were euthanized and tumor volume measured Day 22 after tumor implantation, and 3 days after completing a 10-day course of treatment. Figures 8B shows actual tumor volume of the Panc02 tumors that had grown sub-cutaneously following 10 days of treatment with daily injections of ANT308 or scrambled peptide and every 3 days injection of anti-PD1 monoclonal antibody or iso-type matched antibody from day 10 through day 19 post-implantation. Mice were euthanized and subjected to necropsy on day 22 after tumor implantation, and 3 days after completing a 10-day course of treatment.

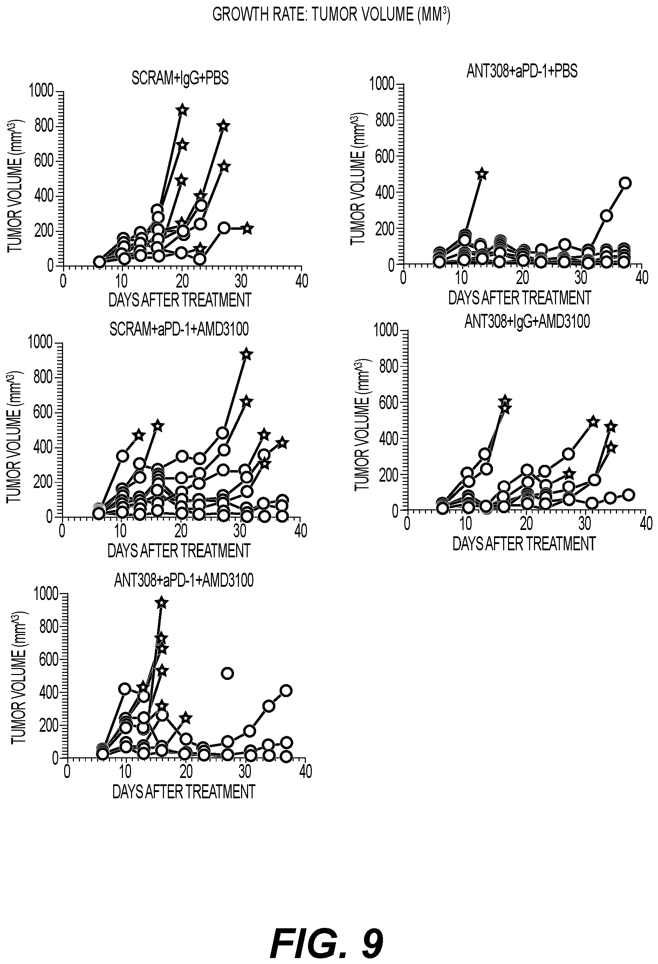

29. Figure 9 shows tumor size and growth rates at 37 days following sub-cutaneous KPC tumor implantation. Shown are scrambled peptide plus IgG, Ant308 plus anti-PD1, scrambled peptide plus anti-PD-1, Ant308 plus IgG, and Ant308 plus AMD3100. Tumor volume was measured with calipers. 'Star' (*) indicates mice euthanized either due to large tumor volume (>500mm³) or ulceration of the skin overlying the tumor.

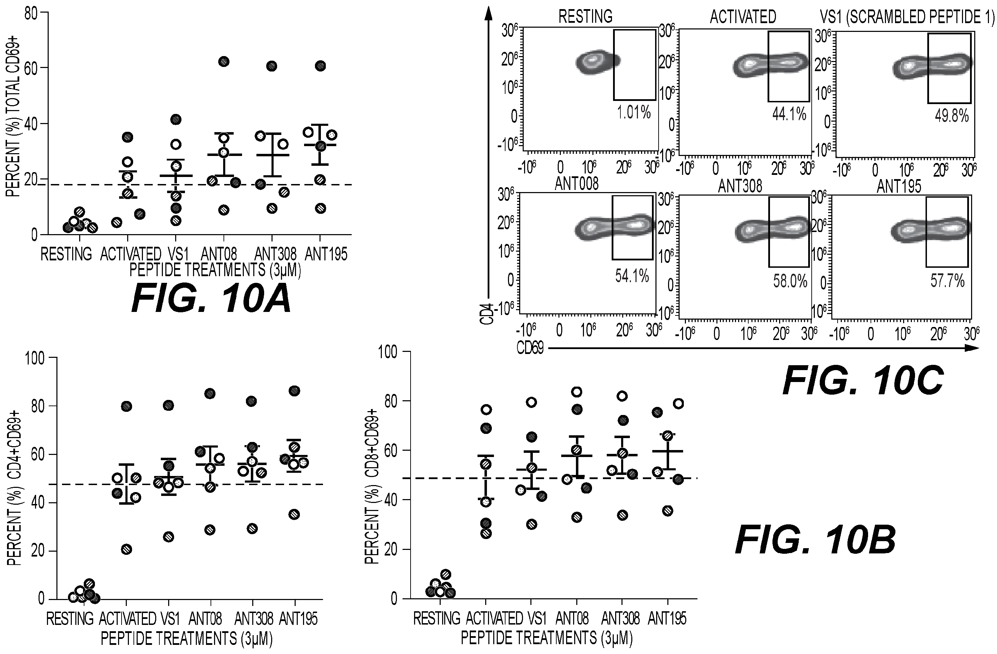

30. Figures 10A, 10B, and 10C show that treatment of human T cells with VIP-R antagonists (Ant08, Ant308, Ant195) augments T cell activation, as measured through CD69 expression. Figure 0A shows the total percent of T cells positive for CD69 expression at 6hrs with respective peptide treatments at 3uM. Figure 10B shows the total percent of CD4+ and CD8+ subset T cells positive for CD69 expression at 24hrs with respective peptide treatments. 'Resting' represents group maintained in culture for 6 or 24 hours without activation. 'Activated' reflects T cells activated with CD3 antibody coated plates with no corresponding peptide treatment. 'VS1' denotes group activated in the presence of VIP scrambled 1 peptide as a peptide control. Mean value from 'Activated' group shown with dotted line (---) for comparison. Responses from same healthy donors are shown with same color dots. Error bars are computed as the standard error of the mean (SEM) from 6 healthy donor samples. Figure 10C shows a representative flow plot showing higher proportion of CD4+CD69+ T cells in antagonist-treated group relative to control groups (row 1) at 24hrs from one donor (blue dots).

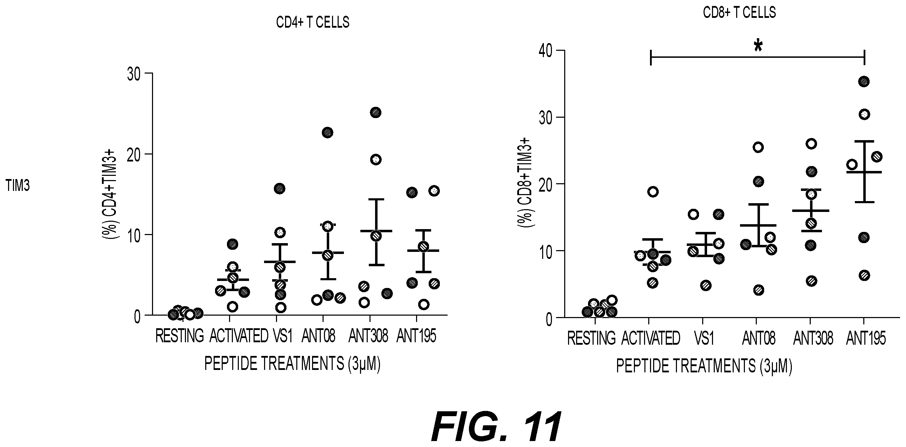

31. Figure 11 shows the expression of TIM3 in CD4+ and CD8+ T cells at 24 hours post treatment.

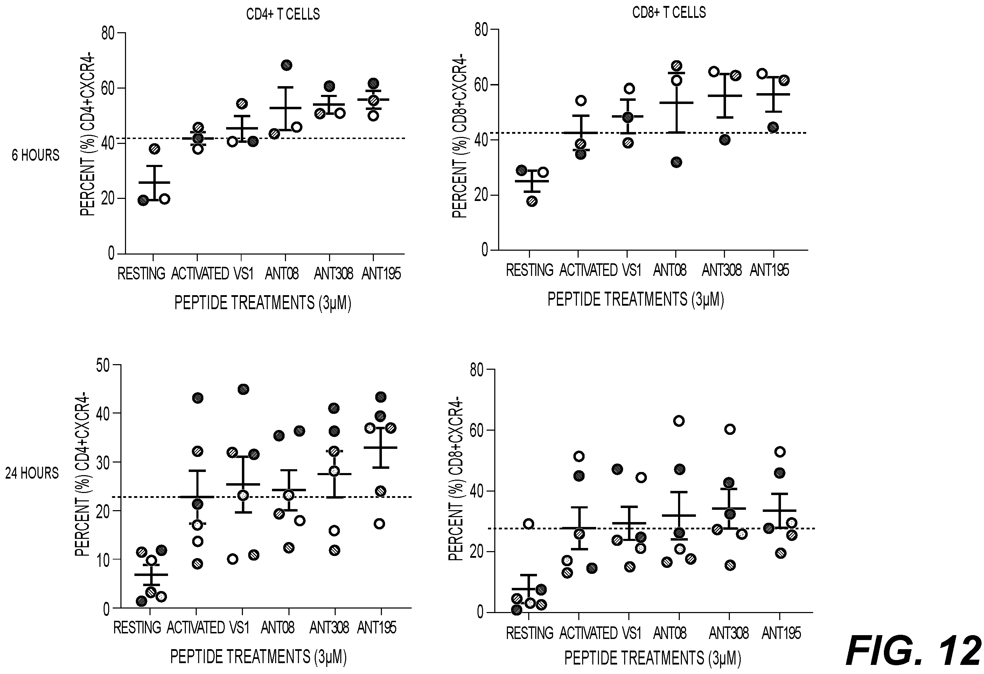

32. Figure 12 shows the expression of CXCR4 in CD4+ and CD8+ T cells at 6 and 24 hours post treatment.

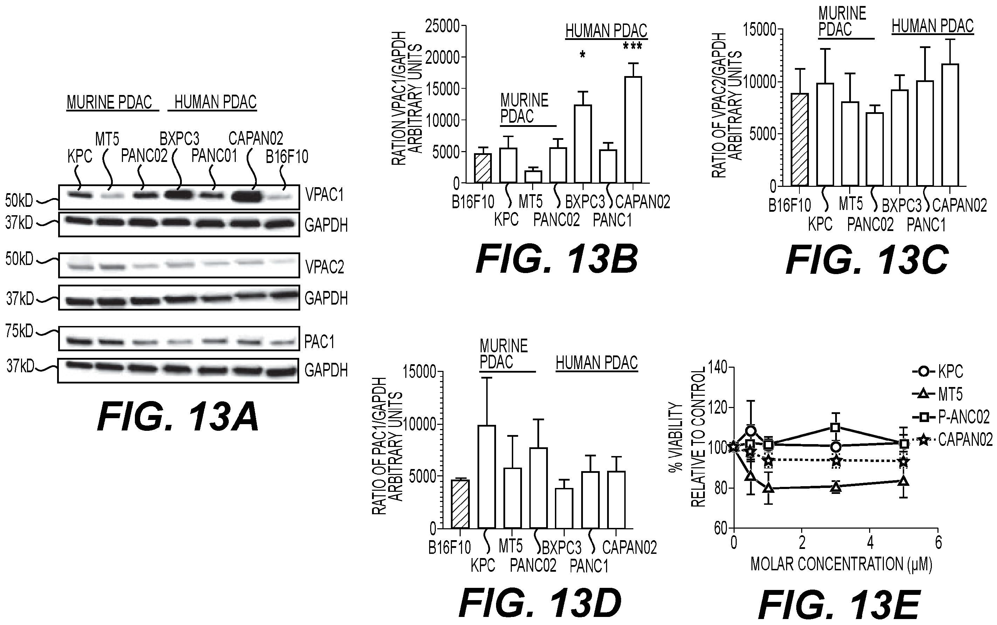

33. Figures 13A, 13B, 13C, 13D, and 13E show that mouse pancreatic cancer cells (KPC) express VIP receptors but their growth is not affected by treatment with VIP-R antagonist. Figure 13A shows a western blot showing expression of VPAC1, VPAC2, and PAC1 in human and mouse pancreatic cancer cells. Figures 13B, 13C, and 13D show the expression ratio of VPAC1 (Figure 13B), VPAC2 (Figure 13C), and PAC1 (Figure 13D) relative to GAPDH in each cell line. Figure 13E shows the viability of the cells relative to a control with increasing concentration of a CIP antagonist.

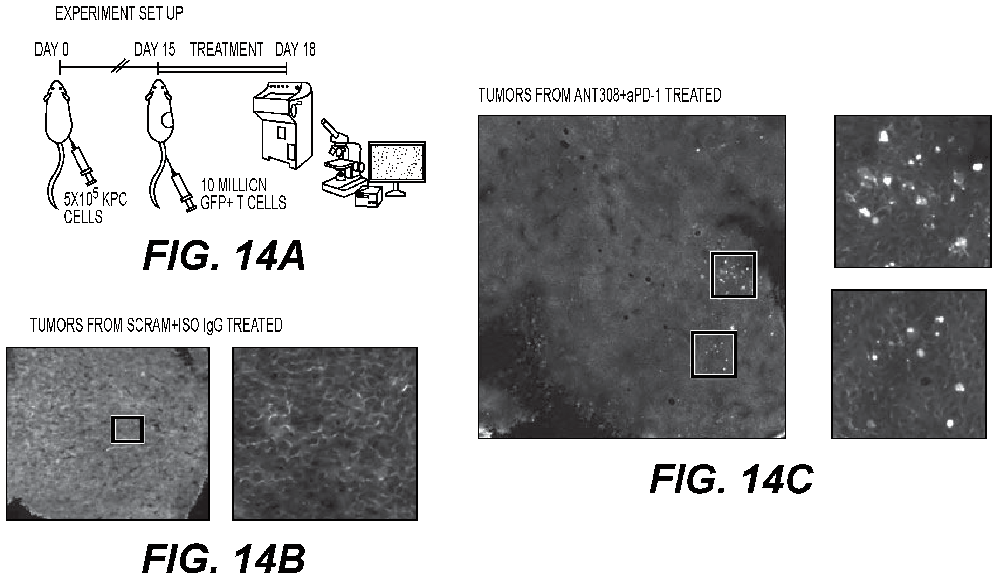

34. Figures 14A, 14B, and 14C show that the VIP-R antagonist ANT308 plus anti-PD1 antibody treatment promoted infiltration of adoptively transferred T cells into pancreatic tumors. Figure 14A shows the experimental scheme. Figures 14B and 14C show immunohistochemistry images from tumors in untreated (Figure 14B) and treated (Figure 14C) mice.

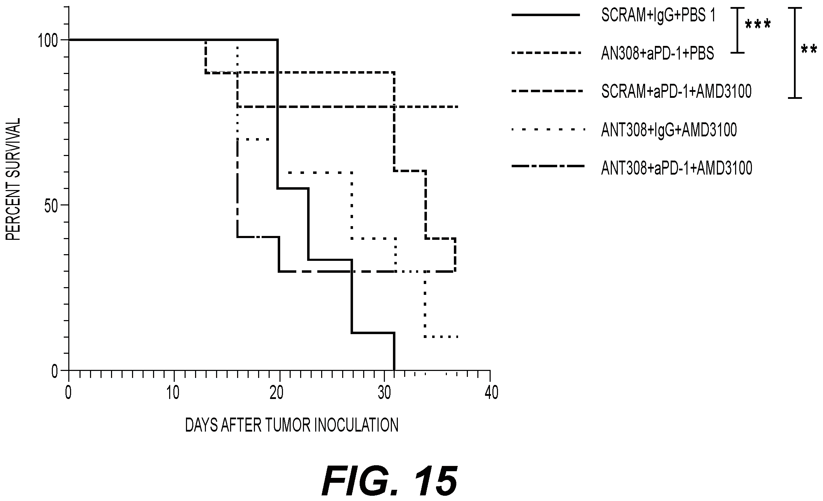

35. Figure 15 shows a survival curve in mice with and without treatment following implantation of pancreatic cancer (KPC) tumors.

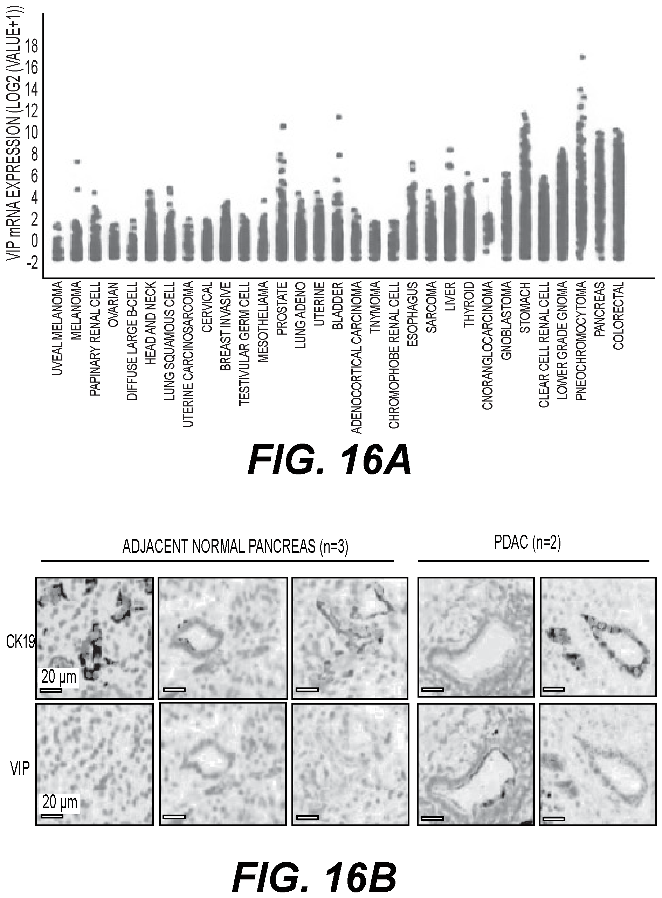

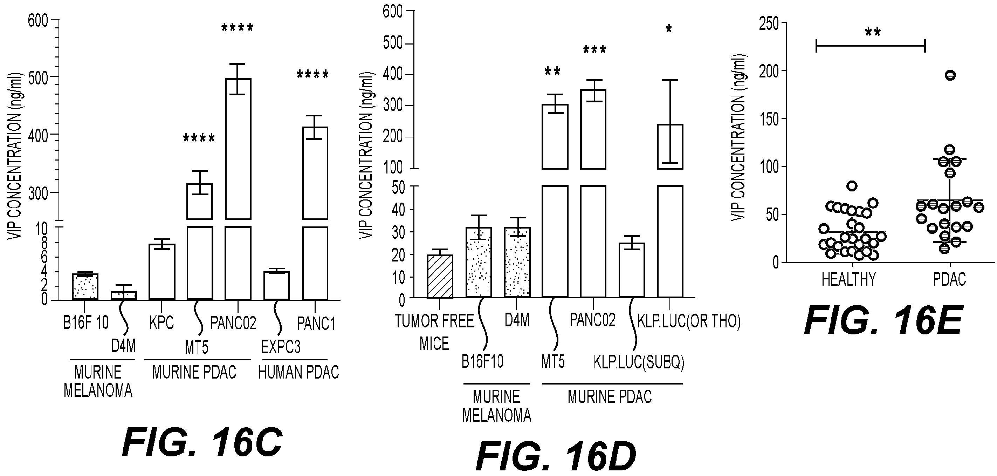

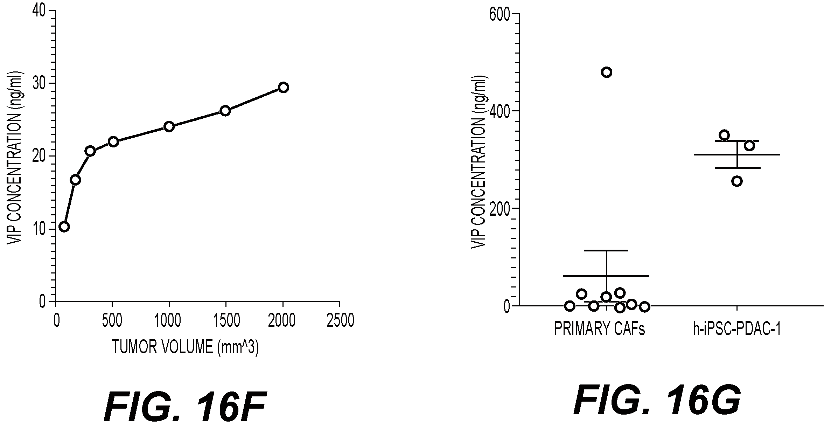

36. Figures 16A, 16B, 16C, 16D, 16E, 16F, and 16G shows that VIP is over-expressed by PDAC. (Figure 16A) VIP mRNA expression levels in various solid malignancies, as obtained from TCGA. (Figure 16B) Representative images of human PDAC tumor stained with antibodies to VIP (green) and CK19 (red), showing higher VIP expression in cancer epithelial cells compared to adjacent normal epithelial cells. Scale bars represent 20µm. Levels of VIP in (Figure 16C) culture supernatants collected from murine and human PDAC cell lines cultured for 24 hours (n=3 per cell line) were compared to culture supernatants from B16F10 and D4M melanoma cells; (Figure 16D) plasma of mice bearing melanoma or PDAC tumors (n=5) compared to plasma of non-tumor- bearing mice; (Figure 16E) plasma of PDAC patients (n=19) compared to that from healthy volunteers (n=26); (Figure 16F) plasma from one C57BL/6 mice bearing subcutaneous KPC.Luc tumor isolated at different tumor volumes; and (Figure 16G) culture supernatants from primary CAFs isolated from human PDAC tumors (n=9) and PSCL-12 cell line (n=3). p values in Figures 16C and 16D were calculated using ANOVA and Dunnett's post-hoc test, where the means were compared to B16F10. p values in e were calculated by student t-test. Error bars show mean ± SEM. **p<0.01, ***p<0.001 and ****p<0.0001.

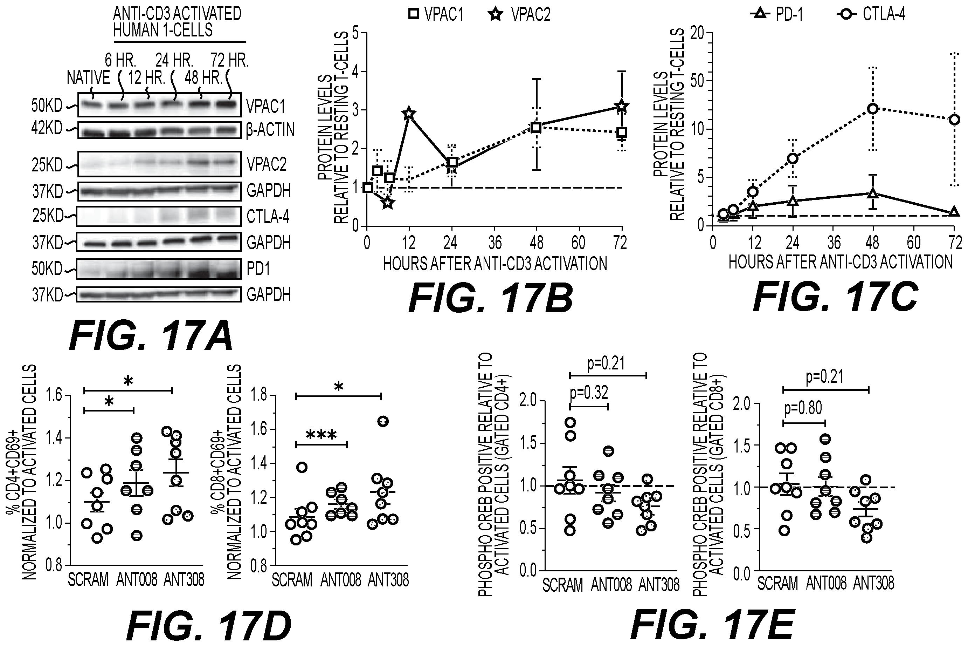

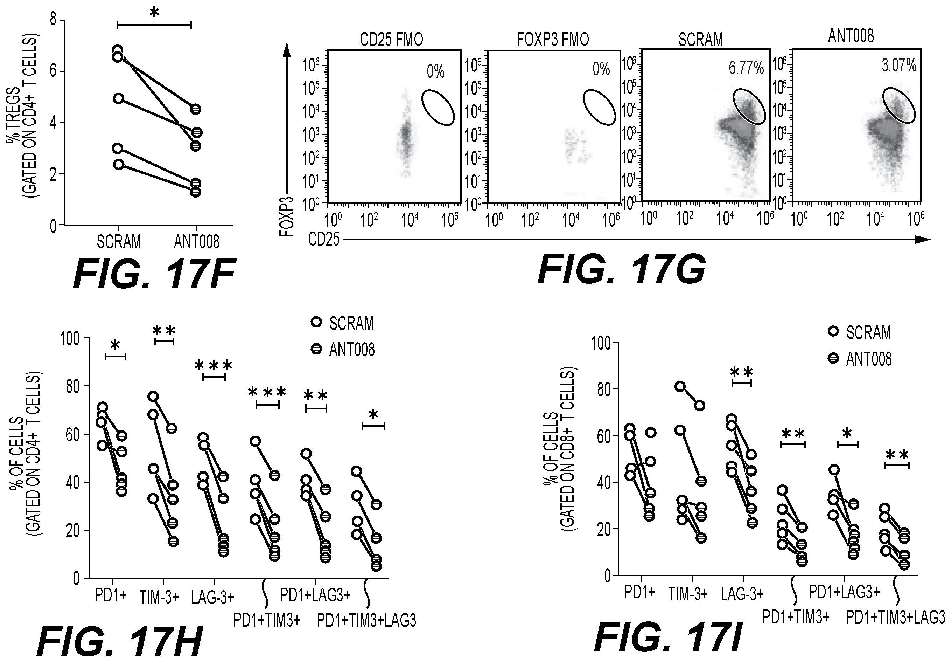

37. Figures 17A, 17B, 17C, 17D, 17E, 17F, 17G, 17H, and 17I show that inhibition of VIP-R signaling decreases T cell exhaustion marker expression in cultured human T cell. (Figure 17A) Representative western blot and (Figure 17B) quantified expression levels of VPAC1 and VPAC2; and (Figure 17C) PD-1 and CTLA-4 in lysates of healthy human T cells expanded with plate bound human anti-CD3 antibodies for 0, 3, 6, 12, 24, 48 and 72 hours. Lower molecular weight band of 25kD shown for VPAC2-specific expression as confirmed from our VPAC2 KO model. Percentage of (Figure 17D) CD69 expression at 24 h post activation (Figure 17E) phosphorylation of CREB (phospho-CREB) downstream of VPAC1/2 receptor at 6 h and in CD4+ and CD8+ T cells normalized to levels in control with no peptide. PDAC patient peripheral blood T cells were expanded for 9 days with plate bound human anti-CD3 antibodies +/- ANT008 and the (Figure 17F) percentage of Tregs was quantified using the (Figure 17G) gating strategy shown. Percentage of PD1+, Tim- 3+, Lag3+, PD1+Tim-3+, PD1+Lag3+ and PD1+Tim-3+Lag-3+(triple positive) in (Figure 17H) CD4+ and (Figure 17I) CD8+ subsets is shown. Statistical differences in Figures 17D and 17E were calculated via repeated measures ANOVA followed by Dunnett's post-test where each sample in the treatment group was compared to the matched sample in the control group (scrambled treated). Statistical differences in Figures 17F, 17H, and 17I were calculated via paired student T-test. Error bars show mean ± SEM. *p<0.50, **p<0.01 and ***p<0.001.

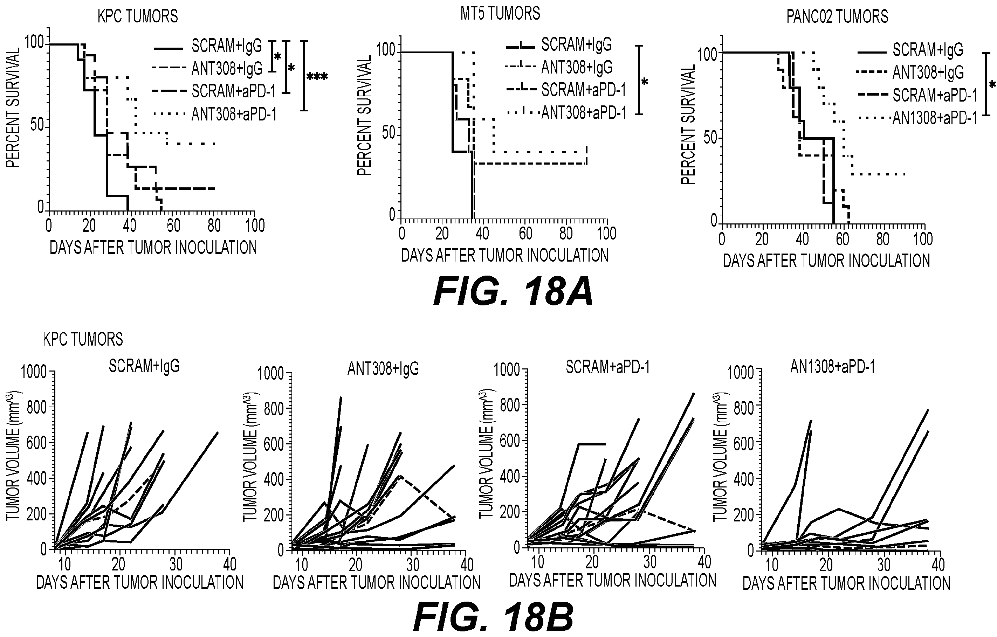

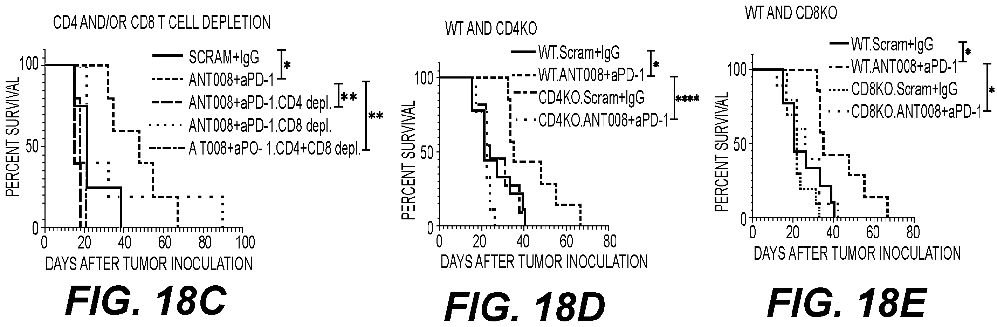

38. Figures 18A, 18B, 18C, 18D, and 18E show improved survival in PDAC-bearing mice treated with the combination of VIP-R antagonists and anti-PD-1 is T cell dependent. (Figure 18A) Kaplan-Meier survival plots of C57BL/6 mice with subcutaneously implanted KPC.Luc, MT5 or Panc02 tumors stratified by treatment. (Figure 18B) Spider plots for KPC.Luc corresponding to results in a. as measured by Vernier calipers following subcutaneous tumor implantation in 4 different treatment groups. Median tumor volumes represented as dashed gray line (----). In a and b, tumor cells were implanted in female or male mice with males receiving 20µg of ANT308 due to higher body weight compared to female mice. Kaplan-Meier survival plots of (Figure 18C) C57BL/6 mice receiving monoclonal CD4 and/or CD8 monoclonal antibodies (Figure 18D) CD4KO or (Figure 18E) CD8KO mice compared to wild-type CD57BL/6 mice with subcutaneously implanted KPC.Luc tumors, stratified by treatment. Statistical differences for Kaplan-Meier curves are calculated via Log-rank test. *p<0.05, **p<0.01 and ***p<0.001, ****p<0.0001.

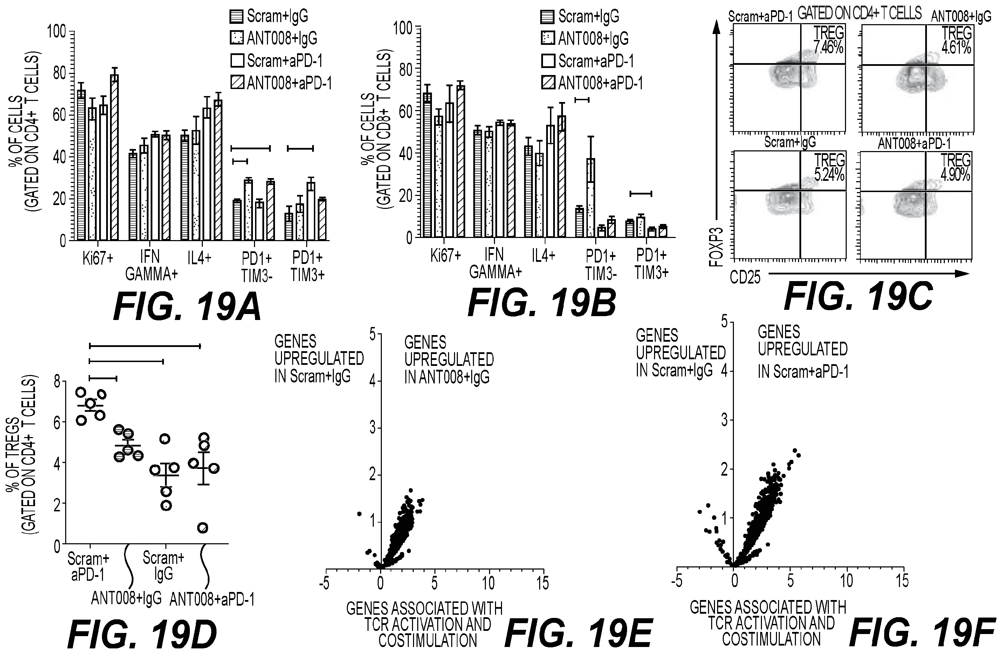

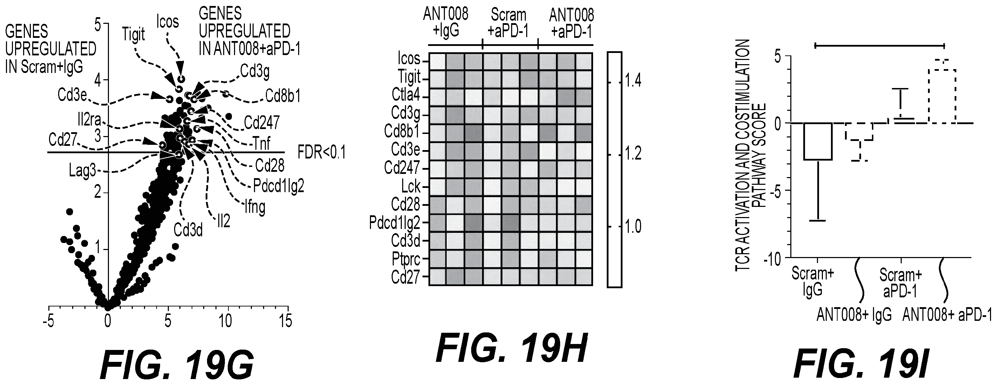

39. Figures 19A, 19B, 19C, 19D, 19E, 19F, 19G, 19H and 19I show increased T cell activation and reduced frequency of Tregs in KPC.Luc tumors treated with a combination of VIP-R antagonist and anti-PD-1. Subcutaneous KPC.Luc tumors in C57BL/6 mice treated with ANT008 and/or anti-PD-1 (n=5 per treatment group), were analyzed via flow cytometry 10 days after treatment for proportion of (Figure 19A) CD4+ and (Figure 19B) CD8+ T cells expressing Ki67, IFN gamma, IL-4, PD-1 and Tim-3. (19C) Representative flow plots showing the gating strategy used to quantify CD25+ FoxP3+ Tregs. (Figure 19D) Percentage of Tregs in tumors of the different treatment groups (n=5 per treatment group). Volcano plot showing differential expression of genes in T cells from (Figure 19E) ANT008+ isotype IgG (IgG) vs scrambled peptide (Scram) + isotype IgG, (Figure 19F) scrambled peptide +anti-PD-1 vs scrambled peptide + isotype IgG and (Figure 19G) ANT008+anti-PD-1 vs scrambled peptide + isotype IgG (n=3 mice per treatment group). Horizontal black line represents false discovery rate (FDR) < 0.1. Genes that are associated with TCR activation and co-stimulation and are at levels significantly higher when compared to Scram+ isotype IgG (FDR<0.1) are labeled in red. (Figure 19H) Heat map showing gene expression changes in genes associated with TCR activation and co-stimulation. (Figure 19I) TCR activation and co-stimulation pathway score between the T cells in tumors of mice from the different treatment groups. Statistical differences in Figure 19A, 19B, 19D and 19I were calculated via ANOVA followed by Dunnett's post-test. Error bars show mean ± SEM *p<0.05, **p<0.001, ***p<0.0001.

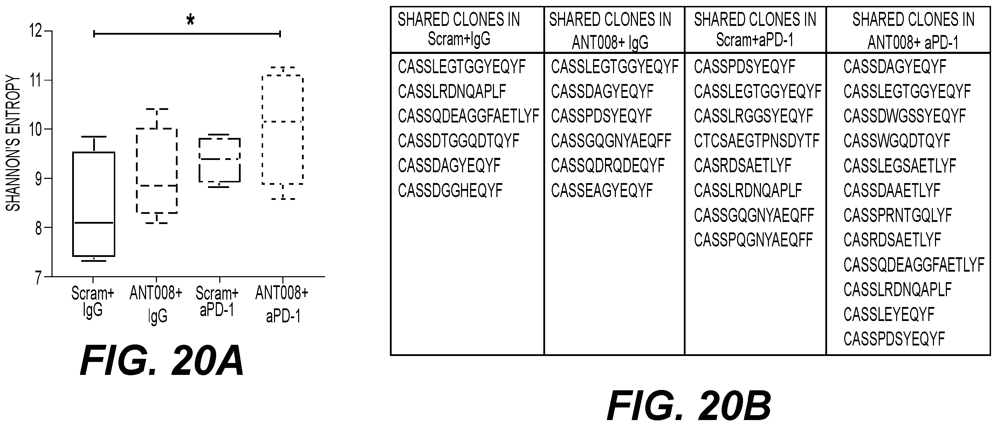

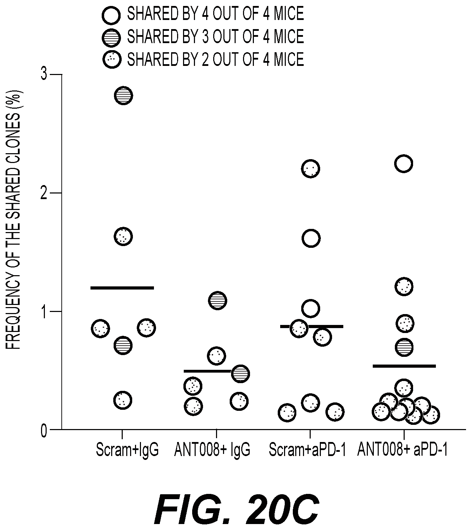

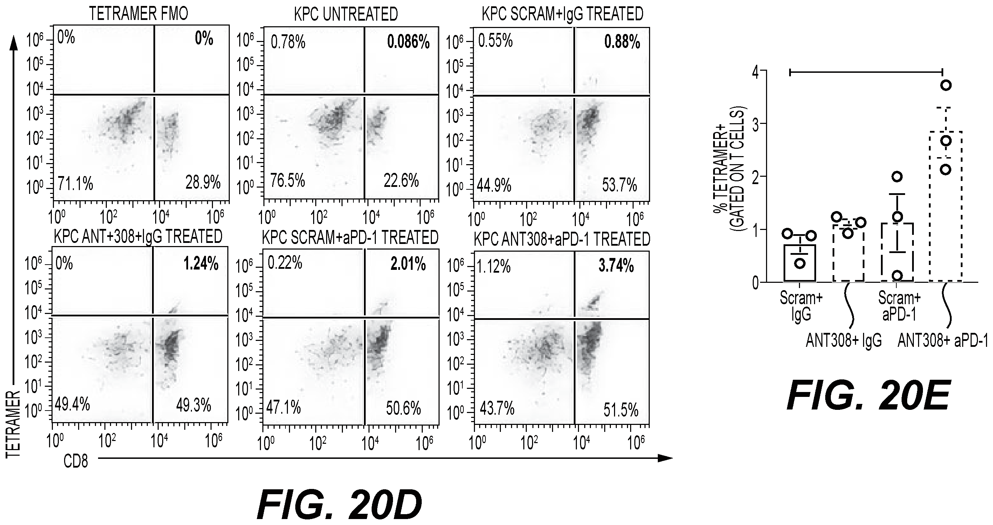

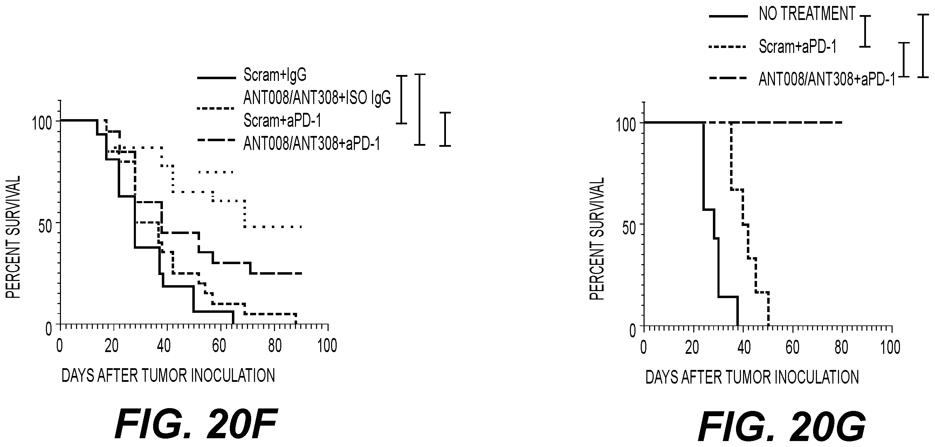

40. Figures 20A, 20B, 20C, 20D, 20E, 20F, and 20G show that combination therapy with VIP-R antagonist and anti-PD-1 increased frequency of tetramer+, CD8+ T cells within the tumor and provide protective immunity to tumor re- challenge. (Figure 20A) Box and whiskers plot showing Shannon's Entropy in T cells of KPC.Luc tumors in each treatment group. (Figure 20B) List showing TCR-β amino acid sequences shared between samples of each treatment group and (Figure 20C) the frequencies of the shared clones in each treatment group. Sequences are color coded to represent number of mice per group (n=4) that share the specific TCR-β clone. CD8+ in subcutaneous KPC.Luc tumors were stained with MuLV p15E-H2Kb tetramer after 10 days of treatment with ANT308 and/or anti-PD-1 (n=3 per treatment group) using the (Figure 20D) gating strategy and (Figure 20E) quantified. (Figure 20F) Kaplan-Meier survival curves of subcutaneous KPC.Luc bearing mice treated with ANT008/ANT308 and/or anti-PD-1 from day 3-12 after tumor implantation (n=16 per scrambled peptide + isotype IgG, n=20 in ANT008/ANT308+ isotype IgG and in scrambled peptide + anti-PD-1 treatment groups; n=23 in ANT008/ANT308 + anti-PD-1 treatment group). (Figure 20G) Kaplan-Meier survival curves of tumor free mice from Figure 20F that were rechallenged with KPC.Luc tumors on the opposite flank (n=6 in scrambled peptide + anti-PD-1 treatment group; n=8 in ANT008/ANT308+anti-PD-1 treatment group). Naive C57BL/6 mice were inoculated with tumor cells at the same time of rechallenge (n=7). Statistical differences in a and e were calculated via ANOVA followed by Dunnett's post-test and in Figure 20F and Figure 20G were calculated using Log-rank test. Error bars show mean ± SEM *p<0.05, **p<0.01, ****p<0.0001.

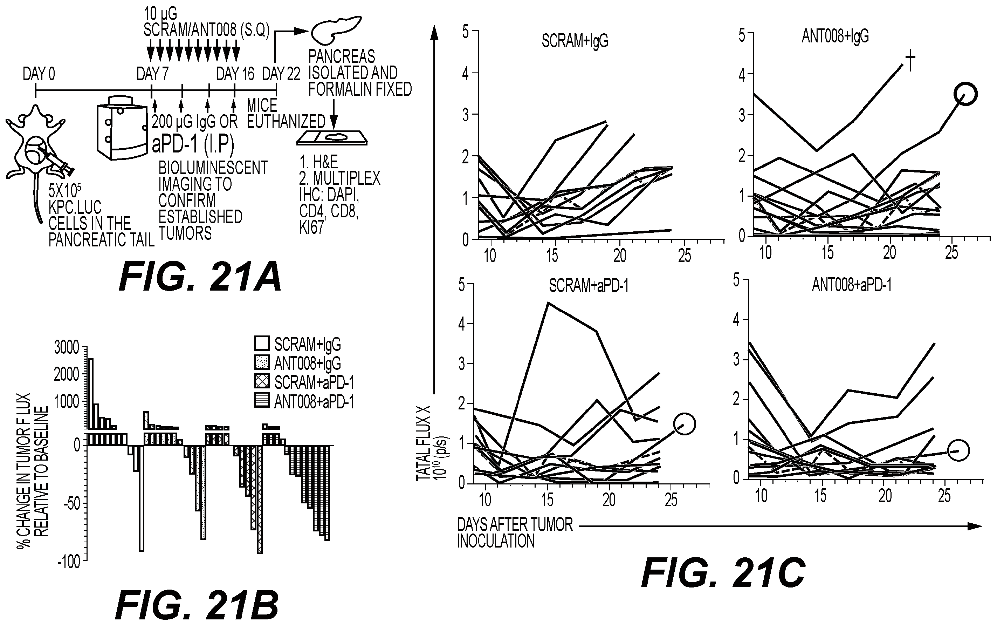

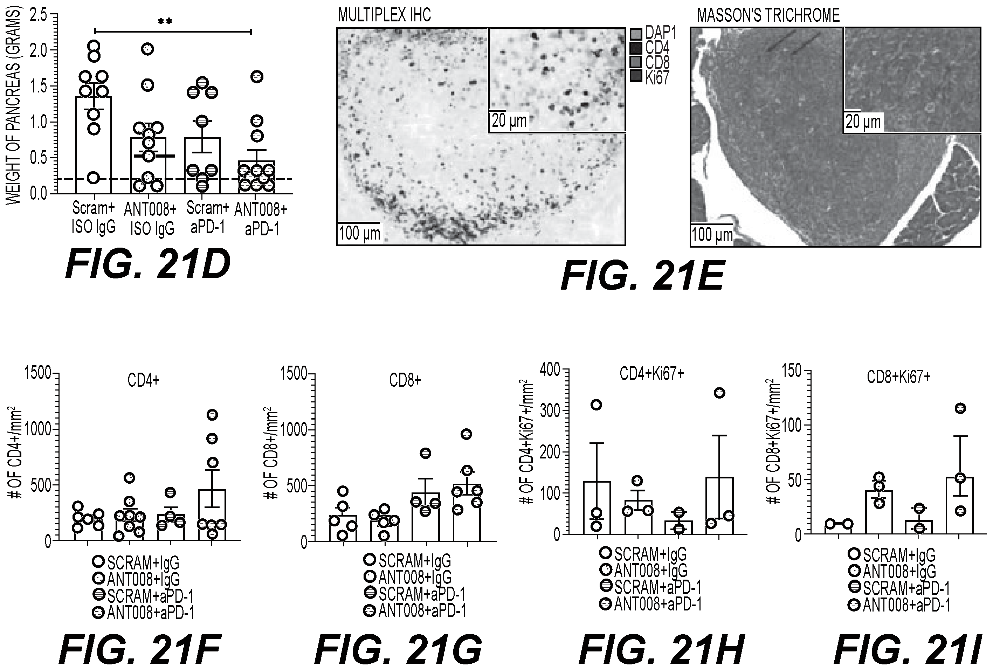

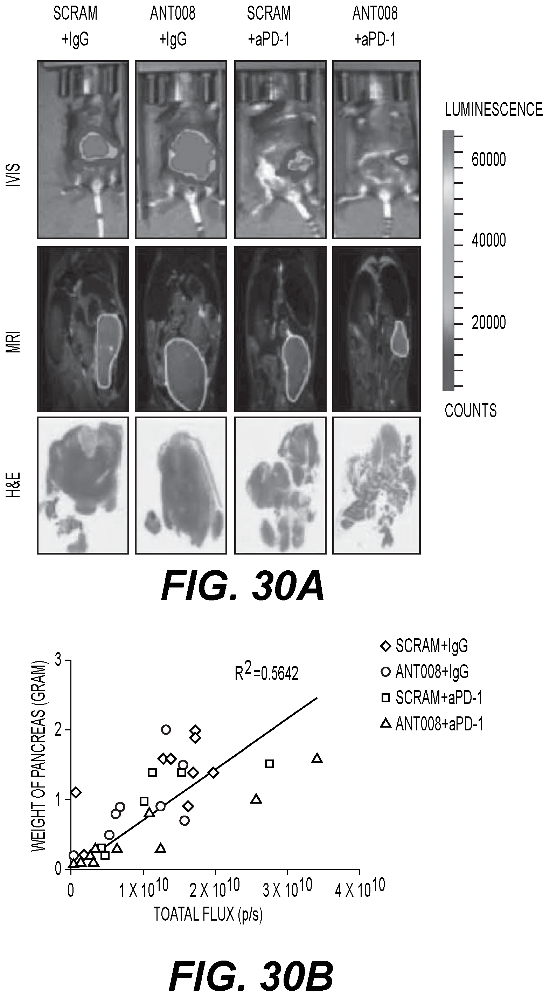

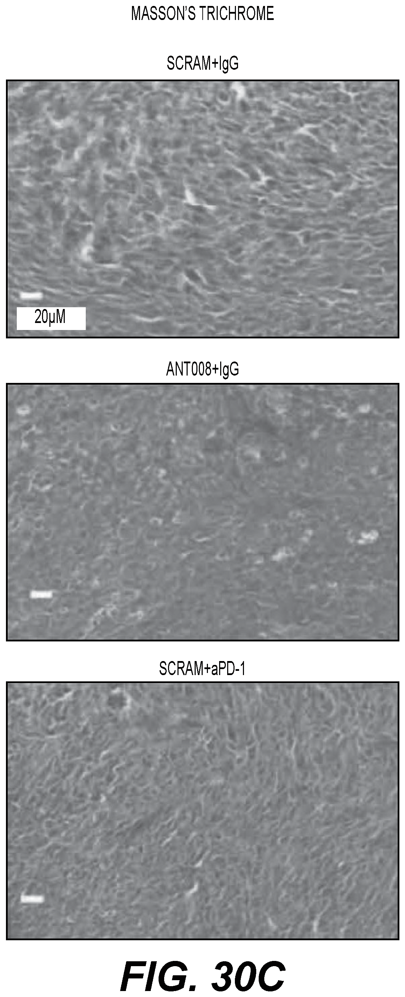

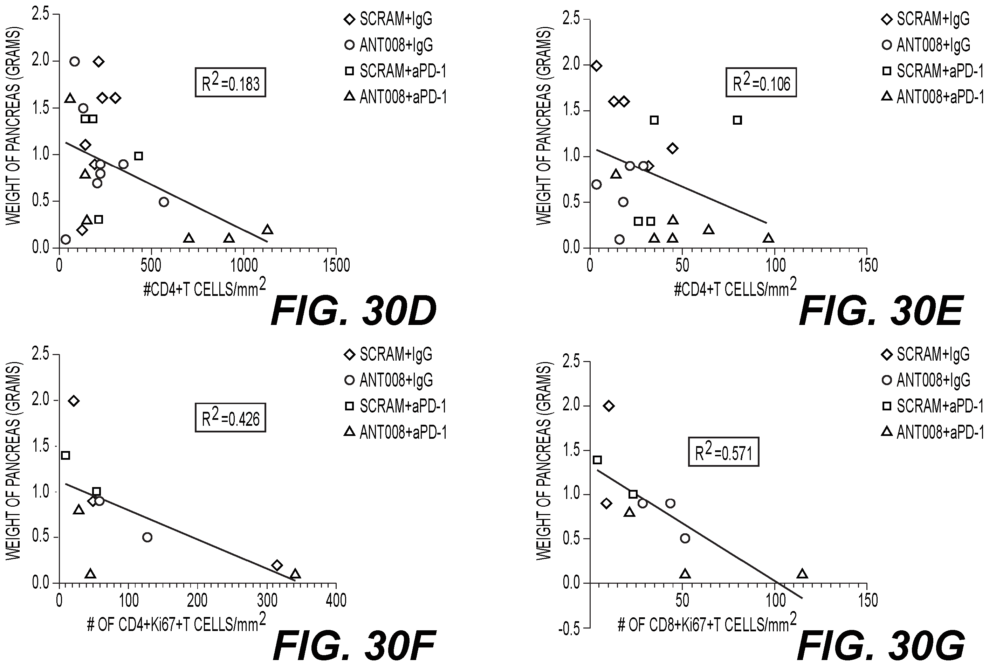

41. Figures 21A, 21B, 21C, 21D, 21E, 21F, 21G, 21H and 21I show that synergism between ANT008 and anti-PD-1 increases T cell infiltration and proliferation and decreases tumor burden in orthotopic KPC.Luc murine PDAC. KPC.Luc cells were orthotopically implanted in the tail of the pancreas of C57BL/6 mice and treated with ANT008 and/or anti-PD-1 with n=9, 10, 8 and 11 in scrambled+IgG, ANT008+IgG, scrambled+anti-PD-1 and ANT008+anti-PD-1, respectively. (Figure 21A) Schematic showing orthotopic implantation of KPC.Luc cells and treatment strategy with ANT008 and/or anti-PD-1. (Figure 21B) Waterfall plot showing % change in tumor flux on day 22 relative to day 7 prior to start of treatment. (Figure 21C) Total flux as measured by IVIS bioluminescent imaging in the different treatment groups. Isoflurane was used for anesthesia for bioluminescent imaging. Median flux represented as dashed gray line (----). Cross symbol (+) represents mice that were euthanized before day 25 due to ulceration of the tumor and circle symbol(o) represent mouse that were imaged on day 26 via MRI imaging shown in Figure 30. (Figure 21D) Bar graph showing weight of pancreas on day 25 when the mice were euthanized. 'Star' shaped (*) data points indicate tumor free mice and dotted horizontal line (----) represents the average weight of healthy pancreas from naive mice. (Figure 21E) Representative multiplex IHC images (right) showing pancreatic tumors stained for DAPI (blue), CD4 (yellow), CD8 (red) and Ki67 (cyan) and trichrome staining (left) with black arrows showing blue collagen stain in the tissue. Bar plot showing number of (Figure 21F) CD4+ or (21G) CD8+ T cells/mm²; and (Figure 21H) Ki67+ CD4+ or (Figure 21I) Ki67+ CD8+ T cells/mm². P values in Figure 21D were calculated using student ANOVA followed by Dunnett's post hoc test (comparing each treatment group with Scram+IgG). Error bars show mean ± SEM. *p<0.05, **p<0.01.

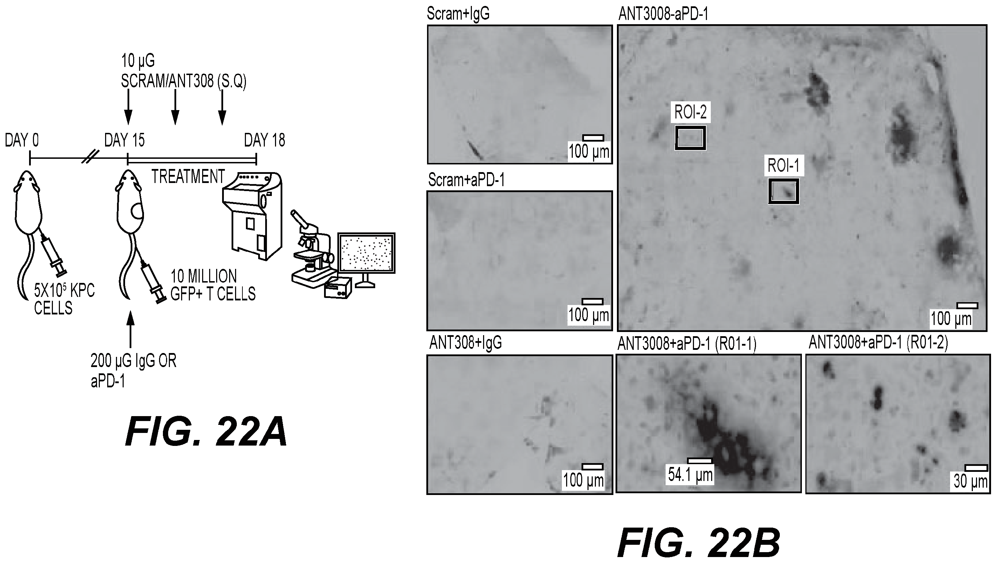

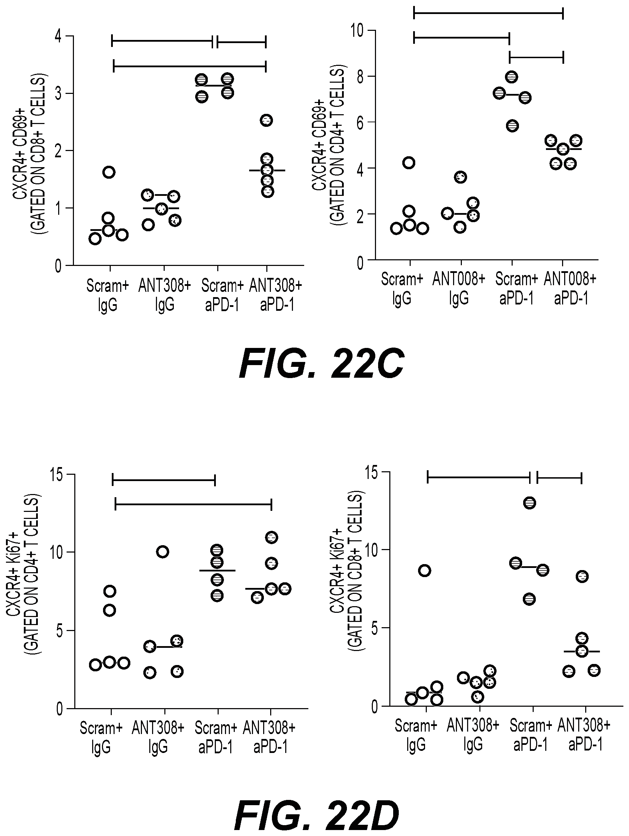

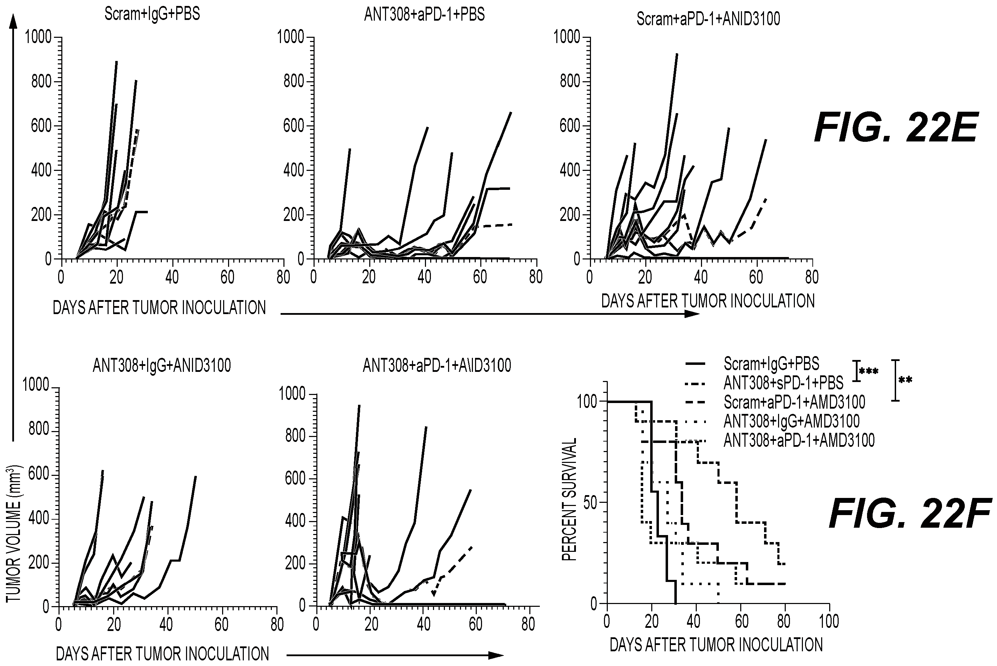

42. Figures 22A, 22B, 22C, 22E, 22F show that combination therapy with VIP-R antagonist and anti-PD-1 promotes intratumoral T cell infiltration and decreases CXCR4 expression on T cells in tumor draining lymph nodes. KPC.Luc tumors were subcutaneously implanted in C57BL/6 mice. On day 15 after tumor implantation, GFP+ T cells were adoptively transferred (via tail vein injections) treated with ANT308+/- aPD-1 for 3 days. (Figure 22A) Schematic showing GFP+ T cell transfer and treatment strategy in mice with subcutaneous KPC.Luc tumors. (Figure 22B) Representative Hoescht (blue for nucleus) stained tumor tissues from tumors of each treatment group. Zoom in of two regions of interest (RO1) labelled as R0I-1 and ROI-2 in the original image of tumors of mice treated with ANT308+aPD- is also shown. Percentage of (Figure 22C) CXCR4+CD69+ and (Figure 22D) CXCR4+Ki67+ cells in CD4+ (left) and CD8+ (right) subsets of T cells. (Figure 22E) Tumor growth rate and (Figure 22F) survival curves generated from mice with subcutaneous KPC.Luc tumors that were treated with scrambled peptide, IgG and PBS or ANT308 and aPD-1 or AMD3100 and aPD-1 or ANT308 and AMD3100 or a combination of ANT308, aPD-1 and AMD3100. Median tumor volume represented as dashed gray line (----). Statistical differences in c and d were determined via repeated measures ANOVA and Dunnett's post-test with n=4-5 mice per group. Statistical differences in 22E were determined via Log-rank test (n = 9-10 mice per group). Straight lines in c and d show mean. *p<0.05, **p<0.01, **p<0.001, p<0.0001.

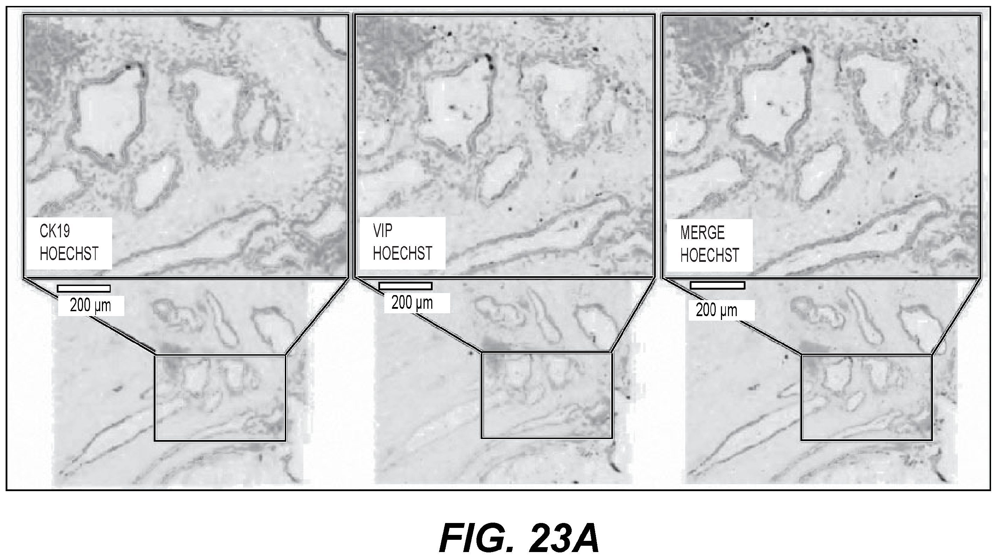

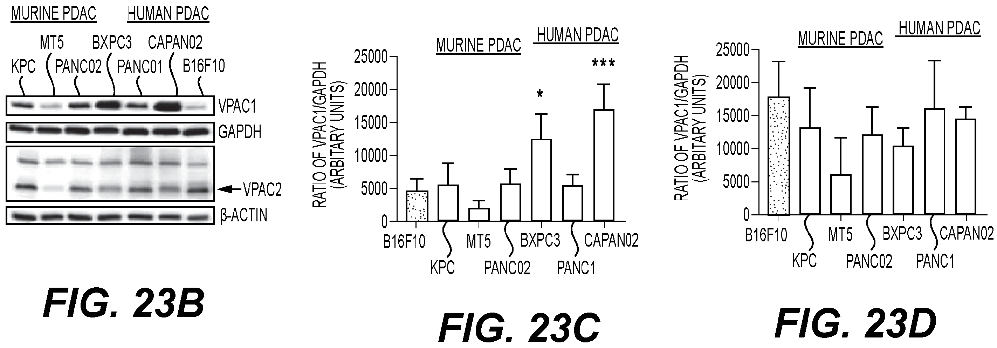

43. Figures 23A, 23B, 23C, and 23D show that PDAC cell lines and human PDAC tissues express VIP and receptors for VIP (Figure 23A) Representative images of one human PDAC tumor stained with antibodies to VIP (green), CK19 (red) and merged (yellow) showing VIP expression in cancer epithelial cells. Scale bars represent 200µm. (Figure 23B) Representative western blot of lysates from murine melanoma; and murine and human PDAC cell lines probed for VPAC1, VPAC2 and GAPDH as control. (Figure 23C) VPAC1, (Figure 23D) VPAC2 protein bands from western blot were analyzed by densitometric analysis and normalized against the intensity of GAPDH. Results are the mean ± SEM of three independent experiments. P values in Figure 23C were determined by ANOVA followed by Dunnett's post-test. *p<0.05, ***p<0.001.

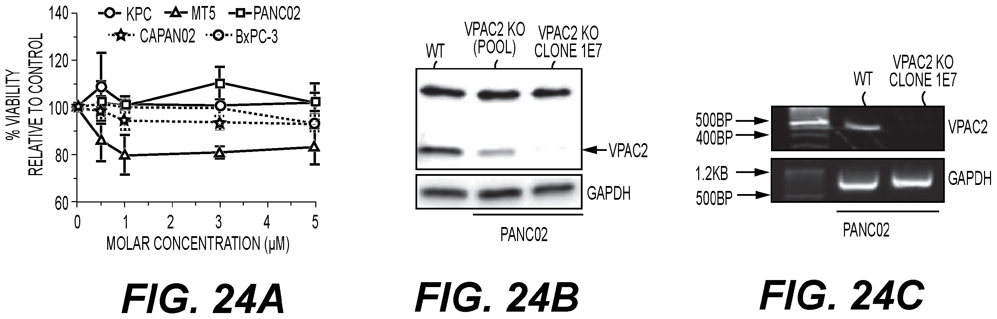

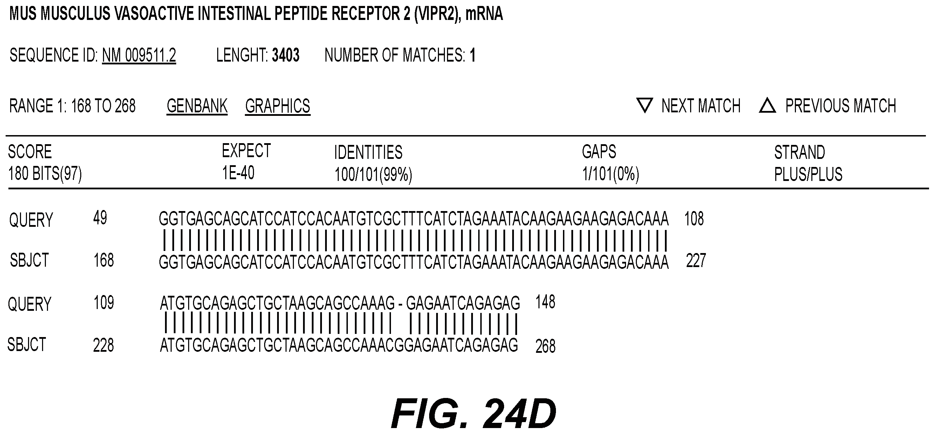

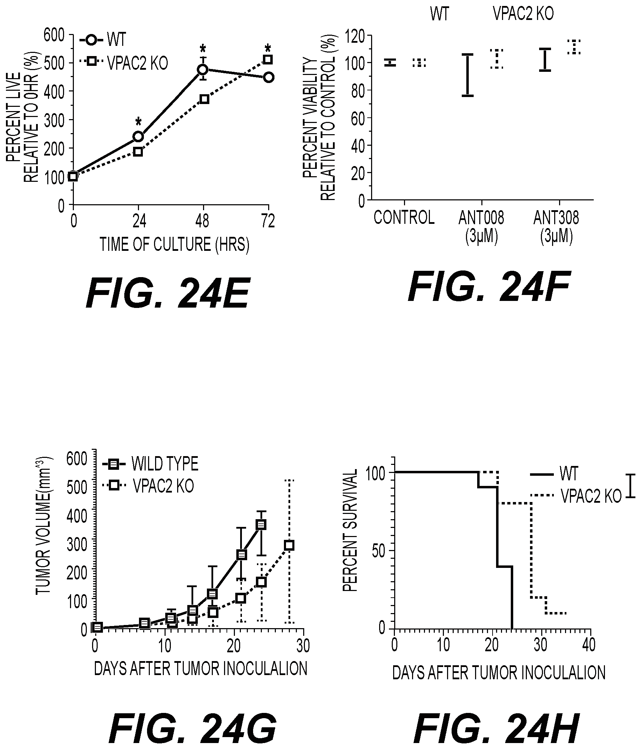

44. Figures 24A, 24B, 24C, 24D, 24E, 24F, 24G, and 24H show that absence of VPAC2 receptor on PDAC cells confer limited autocrine effect on the growth of cancer cells *in vitro* and *in vivo.* (Figure 24A) Percentage viability of murine (MT5, KPC.Luc, Panc02) and human (Capan02, BxPC3) PDAC cell lines cultured in the presence of different concentrations ranging from 0-5µM of ANT008 for 72 hours is plotted. Confirmation of CRISPR-Cas9 KO of VIPR2 encoding VPAC2 receptor via (Figure 24B) western blot; (Figure 24C) RT-PCR using primers targeting exon 9-12 downstream of targeted site and (Figure 24D) Sanger Sequencing showing the validation of in-del mutation in exon 2. *In vitro* MTT assay showing (Figure 24E) Proliferation of WT and KO cells over 72 hours; (Figure 24F) Percent viability of wild type (WT) and VPAC2 KO (KO) Panc02 cells treated with ANT008 and ANT308 at 3µM for 72 hours. (Figure 24G) Tumor growth curve of WT versus KO Panc02 cells in C57BL/6 mice following subcutaneous tumor implantation. Values represent median tumor volume ± 95% confidence interval. (Figure 24H) Kaplan-Meier survival plots corresponding to results in Figure 24G. Median survival time for WT is 21 days and 28 days for VPAC2 KO. Error bars represent mean and standard deviation. *p<0.05, **p<0.01.

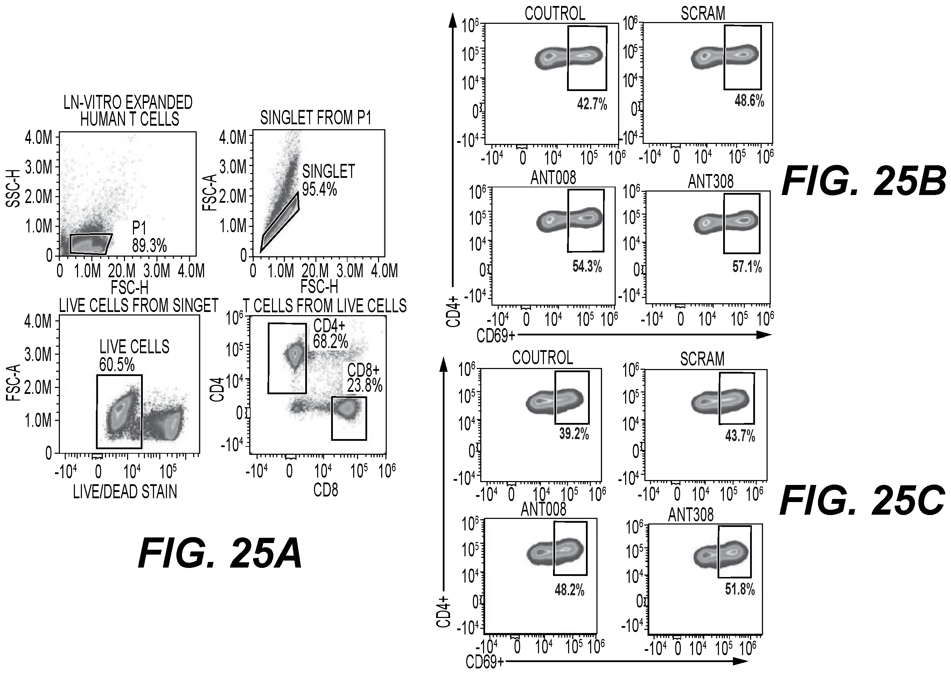

45. Figures 25A, 25B, and 25C show gating strategy for flow cytometric analysis of healthy human T cells. (Figure 25A) Cells were gated as 'P1' by plotting forward scatter height (FSC-H) and side scatter height (SSC-H). Singlet from P1 was selected by gating along the diagonal on forward scatter height (FSC-H) versus forward scatter area (FSC-A) plot. Live cells from singlets were selected by plotting live/dead versus FSC-A. The live cells were then plotted on CD4 versus CD8 plot, to identify CD4+ and CD8+ T cells. CD69 expressing T cells in (Figure 25B) CD4+ and (Figure 25C) CD8+ subsets were then identified by plotting each subset on CD4/CD8 versus CD69 flow plots. Percentage of CD69+ T cells within each subset is shown in shown in red.

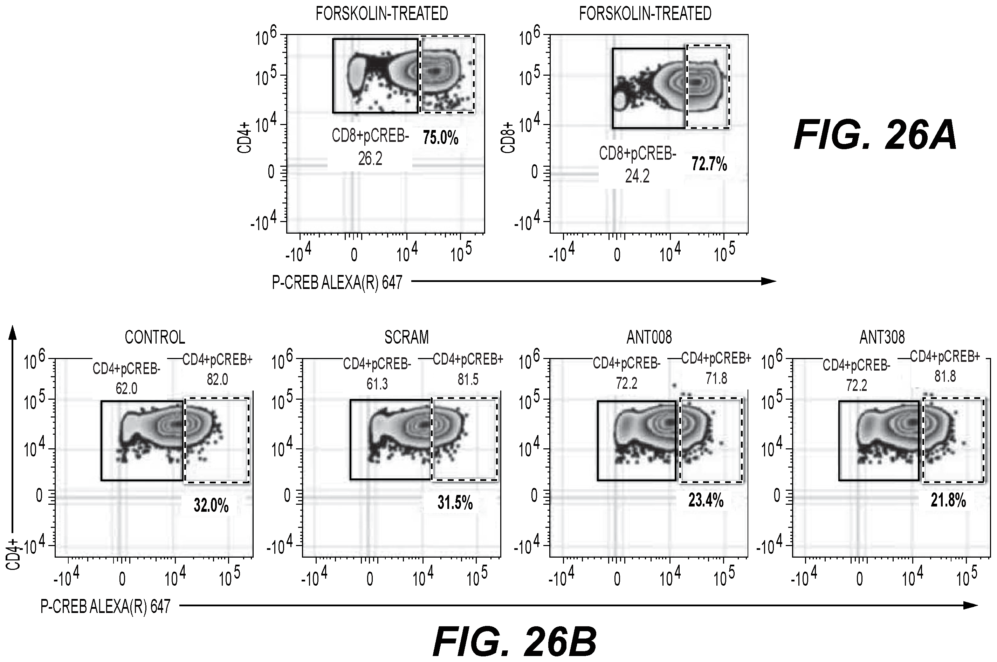

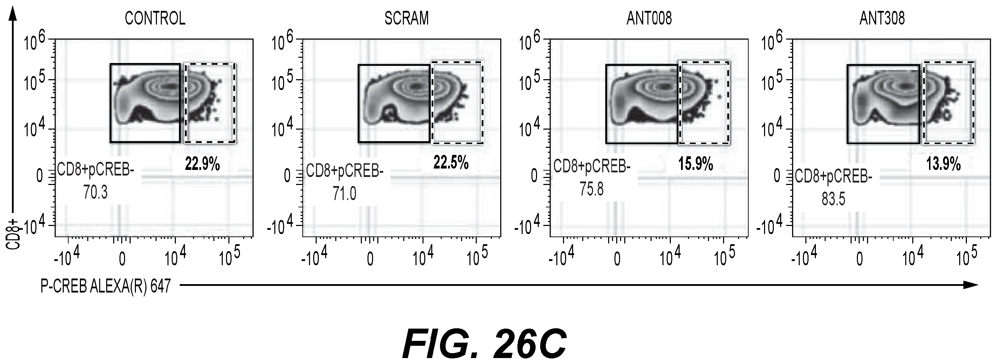

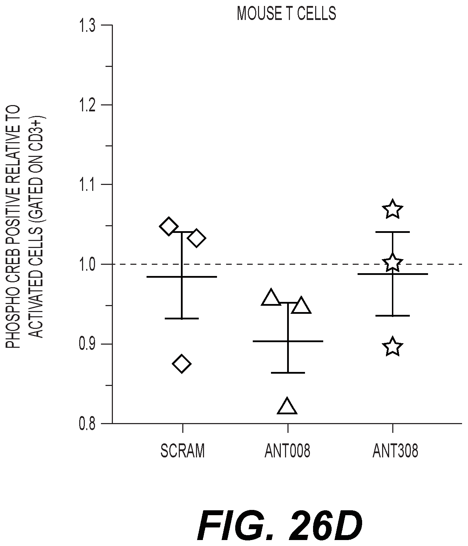

46. Figures 26A, 26B, 26C, and 26D show gating strategy for flow cytometric analysis of CREB phosphorylation in T cells. (Figure 26A) Plots for Forskolin-treated human T cells used as positive control for gating phospho-CREB positive cells. T cells were treated with forskolin at 30µM on ice for 30 mins and stained for the surface expression of CD4 and CD8, followed by intracellular staining with anti-phospho-CREB (S133) antibody. Representative plots for phospho-CREB expression in (Figure 26B) CD4+ and (Figure 26C) CD8+ human T cells when treated with scrambled peptide (Scram), ANT008 and ANT308 at 3µM for 6h (Figure 26D) Percentage of CD3+phospho-CREB+ in murine T cells under similar conditions as in Figure 26C.

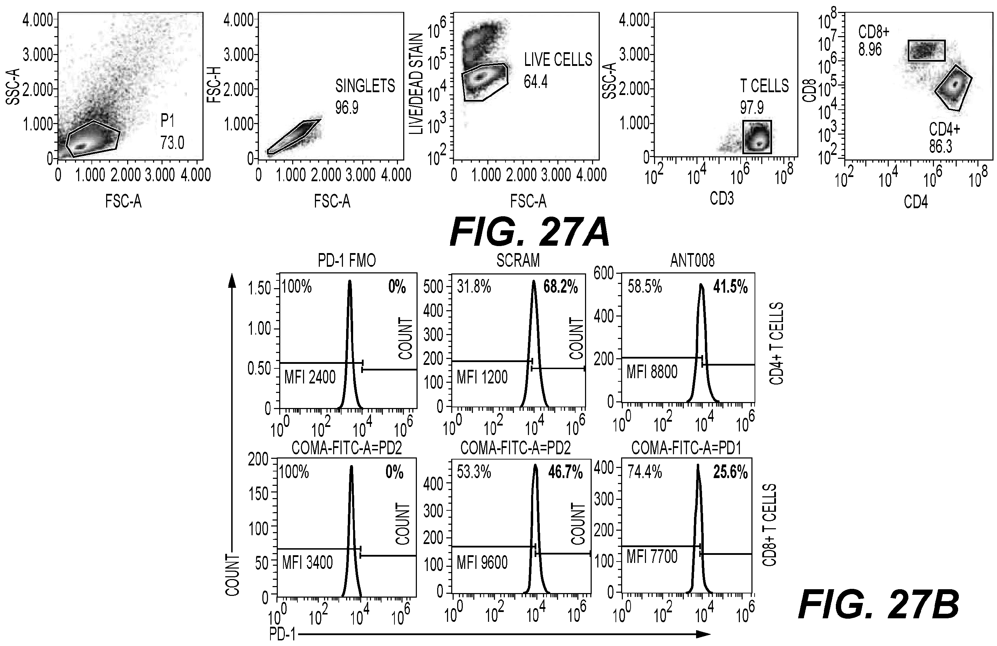

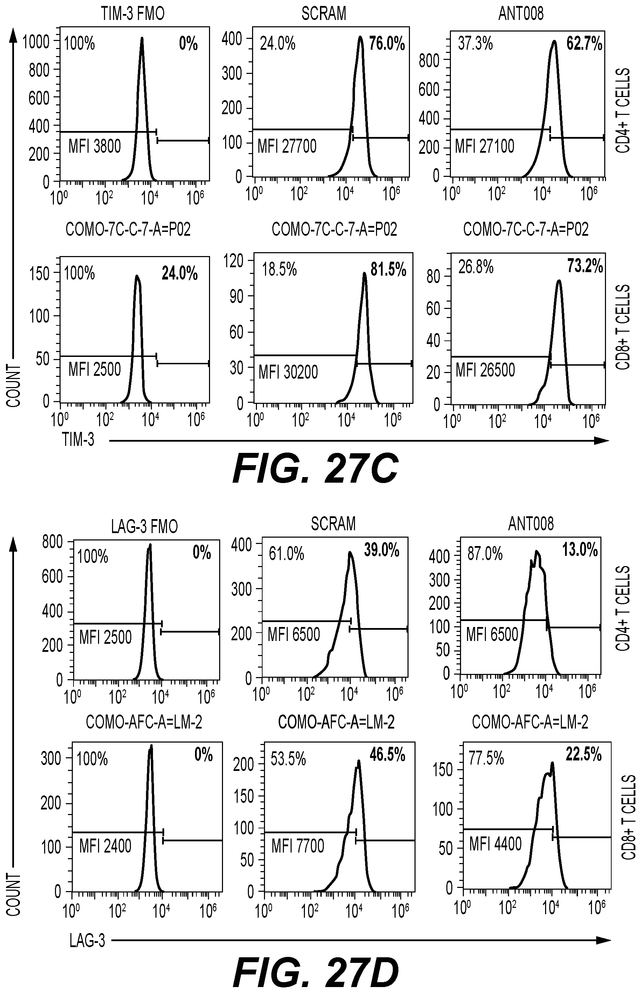

47. Figure 27A, 27B, 27C, and 27D show gating strategy for PD-1, Tim-3 or Lag-3 expression on PDAC patient CD4+ or CD8+ T cells expanded ex-vivo over 9 days. (Figure 27A) Cells were gated as 'P1' by plotting forward scatter area (FSC-A) and side scatter area (SSC-A). Singlet from P1 was selected by gating along the diagonal on forward scatter height (FSC-H) versus forward scatter area (FSC-A) plot. Live cells from singlets were selected by plotting live/dead versus FSC-A. The live cells were then plotted on CD3 versus FSC-A plot, and the cells that are positive for CD3 were gated as T cells. CD4+ and CD8+ T cells were then discriminated by plotting T cells on CD4 versus CD8 plot. (Figure 27B) PD-1+, (Figure 27C) Tim-3+ and (Figure 27D) Lag-3+ cells were gated on CD4+ (top) or CD8+ (bottom) T cells based on FMO controls.

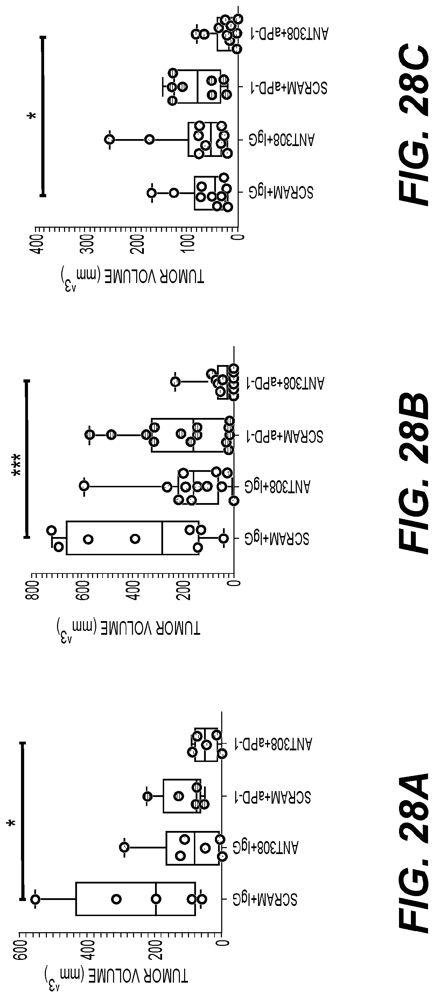

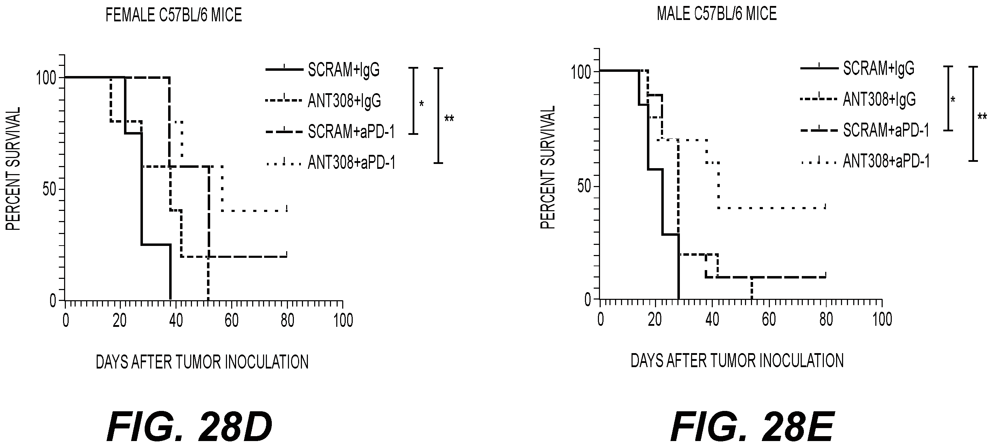

48. Figures 28A, 28B, 28C, 28D, and 28E show that combination therapy with VIP-R antagonist and anti-PD-1 reduces tumor burden and improves survival in male and female C57BL/6 mice with KPC tumors. Boxplot showing tumor volumes of MT5 (Figure 28A); KPC-Luc (Figure 28B) and Panc02 (Figure 28C) tumor volumes as measured by Vernier calipers on day 22 for MT5 and day 22 for KPC and Panc02 after subcutaneous tumor implantation. Kaplan-Meir survival curve of (Figure 28D) female or (Figure 28E) male C57BL/6 mice subcutaneously implanted with KPC.Luc tumors and treated with ANT308 (female: 10µg, male: 20µg) and/or anti-PD-1. Statistical differences in a-c were calculated by ANOVA followed by Dunnett's post-test. Solid line shows median with in each treatment group. Statistical differences in d and e are calculated via Log-rank test. *p<0.05, **p<0.01 and ***p<0.001.

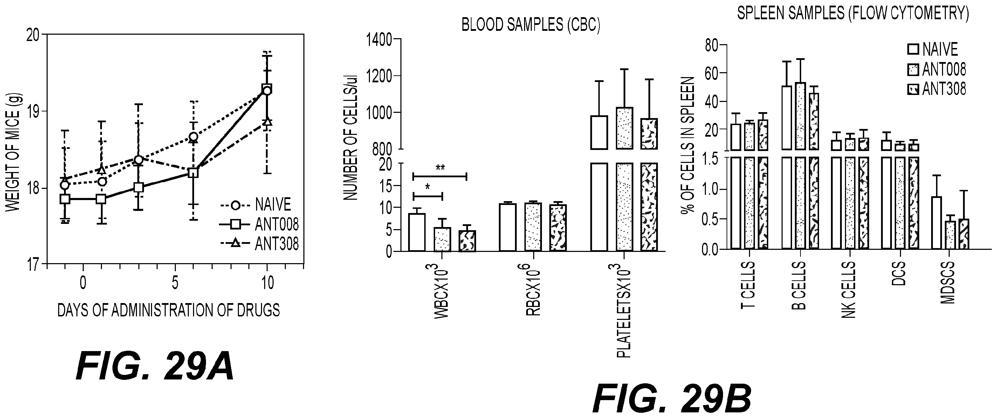

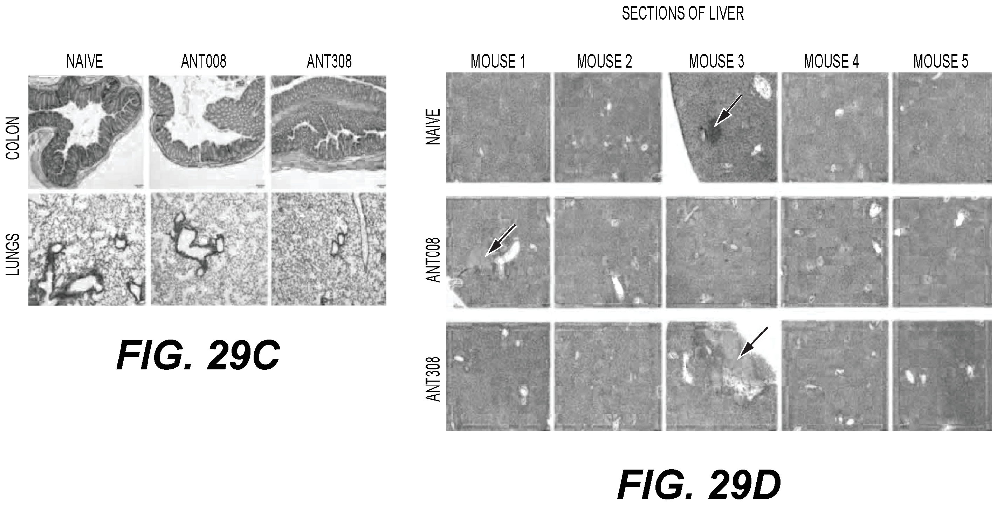

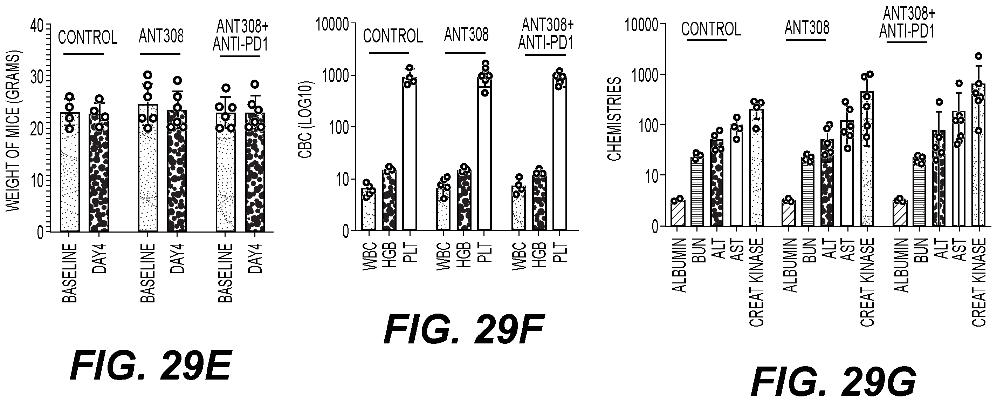

49. Figures 29A, 29B, 29C, 29D, 29E, 29F, and 29G show that administration of ANT008 or ANT308 showed no adverse toxicity in C57BL/6 mice. C57BL/6 mice received daily subcutaneous injection of ANT008 or ANT308 for 10 days (n=5 per group) and analyzed for evidence of toxicity on day 11. (Figure 29A) Body weight in grams during the duration of drug administration; (Figure 29B) Number of WBCs, RBCs and platelets in blood as per complete blood count (left) proportions of T cells, B cells, NK cells, DCs and MDSCs in spleen as identified by flow cytometry (right) are plotted. Representative H&E stained sections of (Figure 29C) colon (top), lungs (bottom) and (Figure 29D) liver are shown. Arrows in Figure 29D show the focal hepatic lesions in liver. The focal hepatic necrosis that was observed in one of five mice in each group is not considered as drug-related toxicity, as these lesions are commonly observed in several in-bred mice strains at the Jackson Laboratory. C57BL/6 mice received daily subcutaneous injection of 30ug of ANT308 (n=6) or a combination of 30ug of ANT308 daily along with 200ug of anti-PD1 every 3 days (n=6), for a duration of 4 days. Mice receiving scrambled peptide and isotype IgG served as control (n=4). (Figure 29E) Weight of the mice, (Figure 29F) complete blood count (CBC) and (Figure 29G) serum chemistries after 4 days of treatment are plotted. P values in Figures 29B, 29E, 29F, and 29G were calculated by ANOVA followed by Dunnett's post-test. Error bars represent mean and standard deviation. *p<0.05, **p<0.01.

*50.* Figures 30A, 30B, 30C, 30D, 30E, 30F, and 30G show that bioluminescent signal from orthotopically implanted KPC.Luc tumors positively correlated with tumor burden and demonstrates histologic desmoplasia. (Figure 30A) On day 26 after orthotopic KPC.Luc tumor implantation in C57BL/6 mice, tumor burden in representative mice indicated by 'circle' symbol in Figure 21C, were compared via bioluminescent imaging, IVIS imaging and H&E staining of formalin fixed pancreas isolated after euthanasia. For bioluminescent imaging, isoflurane was used for anesthesia. (Figure 30B) Total flux (p/s) as measured by bioluminescent imaging on day 26 after tumor implantation was plotted with respect to weight of the isolated pancreas after euthanasia. Data points are color coded to represent mice in different treatment groups with n=9, 10, 8 and 11 in scrambled+IgG, ANT008+IgG, scrambled+anti-PD-1 and ANT008+anti-PD-1, respectively. (Figure 30C) Trichrome staining showing blue collagen stains in the tissue for orthotopically implanted KPC.Luc tumors in all treatment groups. Representative images for scrambled+IgG, ANT008+IgG, scrambled+anti-PD-1 shown; ANT008+anti-PD-1 shown in Figure 21E. XY plot showing the correlation between number of (Figure 30D) CD4+ or (Figure 30E) CD8+ T cells/mm²; and (Figure 30F) Ki67+ CD4+ or (Figure 30G) Ki67+ CD8+ T cells/mm² with weight of the pancreas with n = 4 to 6 mice per group.

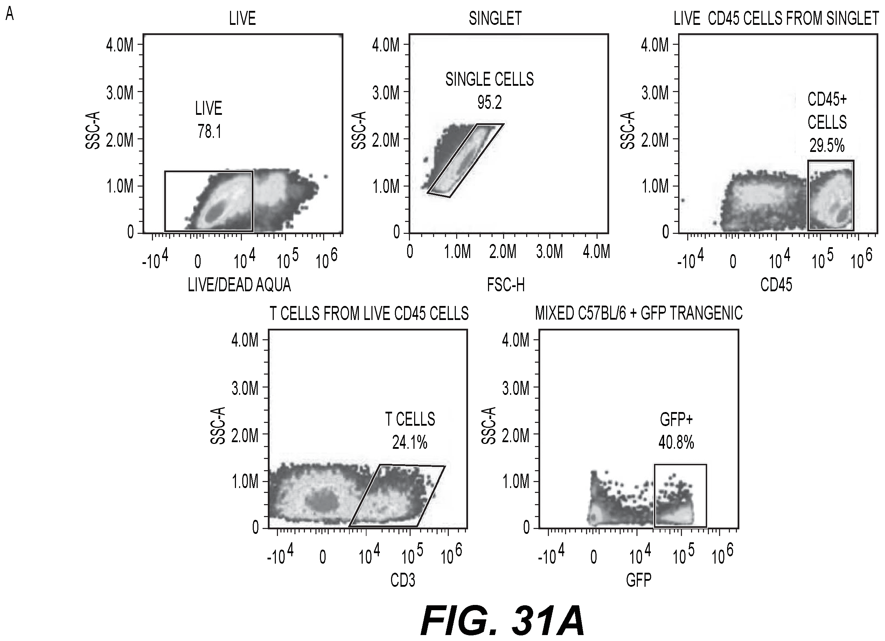

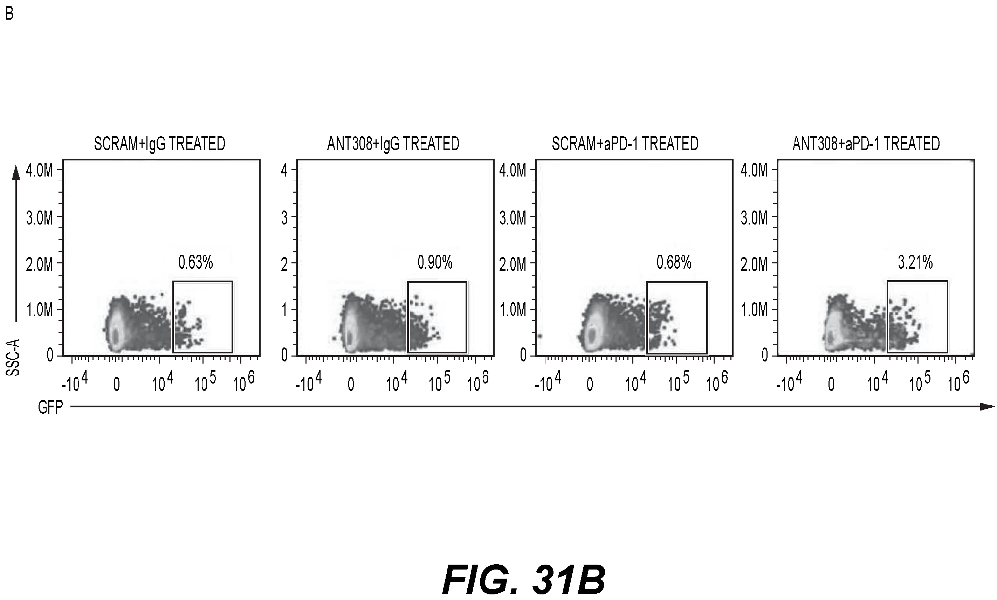

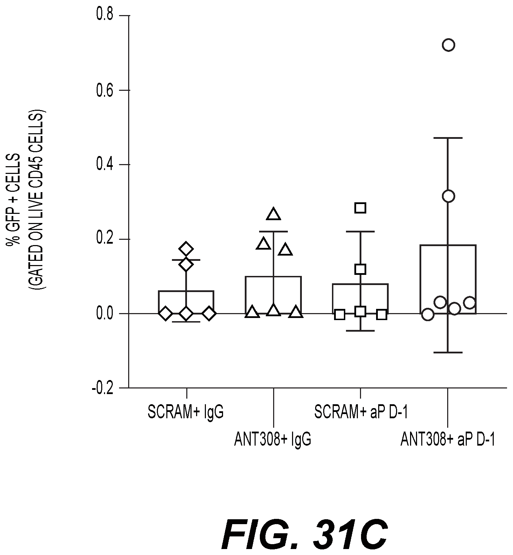

51. Figures 31A, 31B, and 31C show increased frequency of GFP+ T cells in tumors of mice treated with the combination of VIP-R antagonist and anti-PD-1 as confirmed by flow cytometry. (Figure 31A) Singlet from single cell suspensions prepared from tumors of mice from Figure 22A were gated by plotting forward scatter area (FSC-A) versus forward scatter height (FSC-H). Live CD45+ cells were gated by selecting CD45 positive, followed by gating for CD3 positive cells in CD3 versus SSC-A plots. GFP+ cells were then selected by gating GFP positive cells based on mixed population of unstained splenocytes from naive C57BL/6 mice and spleen samples from GFP transgenic mice. (Figure 31B) Representative plot for CD3+GFP+ cells for four treatment groups (Scram+IgG, ANT308+IgG, Scram+anti-PD1, ANT308+ant-PD1) (Figure 31C) Summary data from b showing percentage of GFP+ T cells over live CD45+ T cells. Percent GFP+ T cells were computed as percentage of total CD3+GFP+ events divided by total live CD45+ events enumerated from FlowJo.

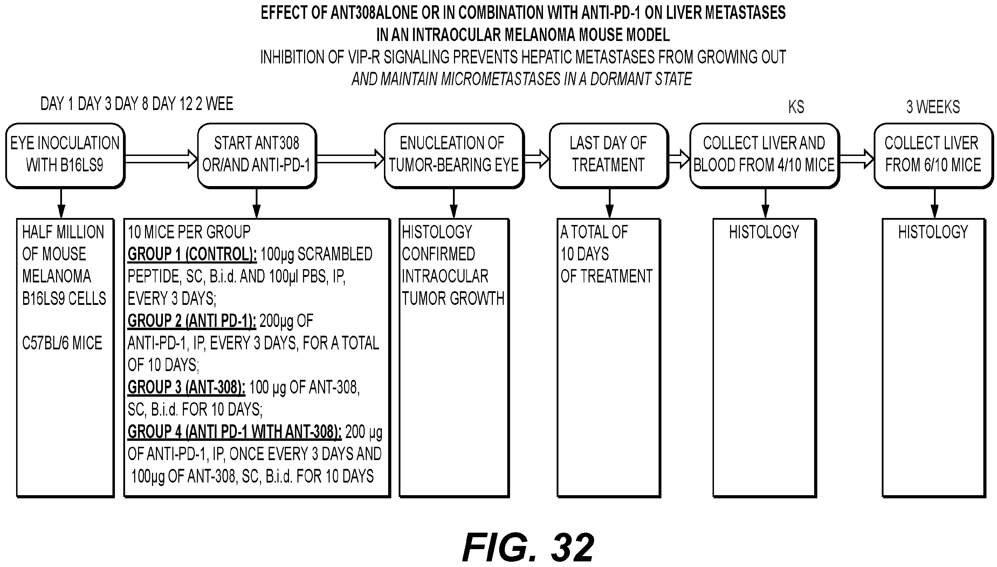

52. Figure 32 shows the experiment design of testing the effect of ANT308 alone or in combination with anti-PD-1 on liver metastases in an intraocular melanoma mouse model.

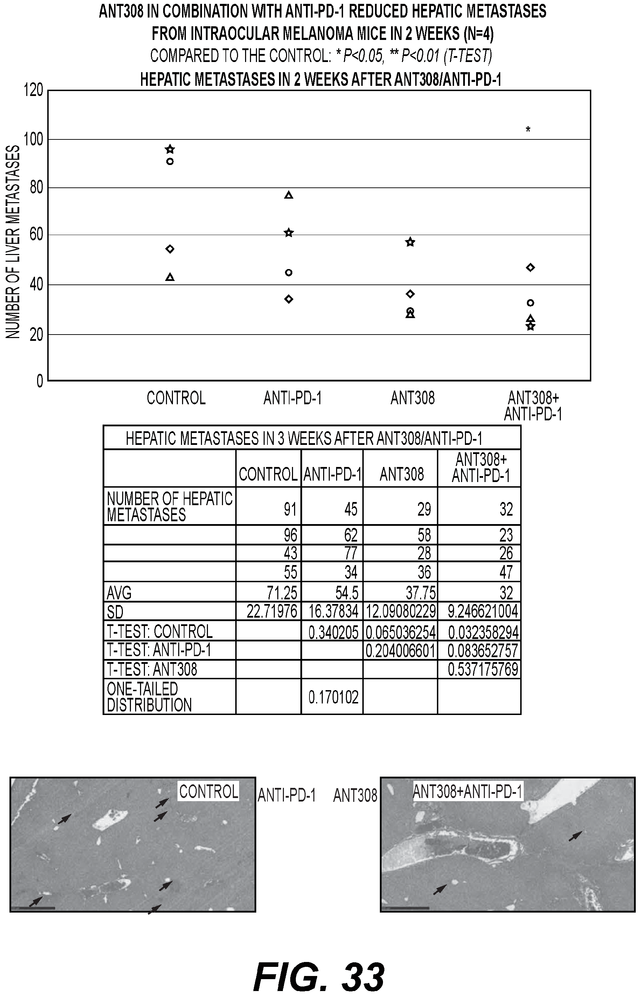

53. Figure 33 shows that ANT308 in combination with anti-PD-1 reduced hepatic metastases from intraocular melanoma mice in 2 weeks (n=4).

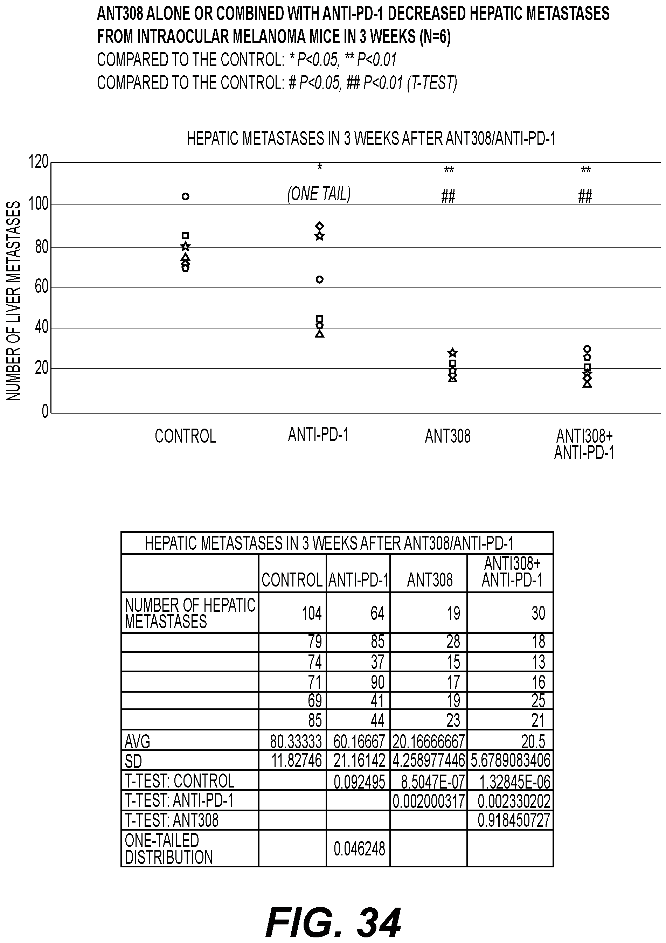

54. Figure 34 shows that ANT308 alone or combined with anti-PD-1 decreased hepatic metastases from intraocular melanoma mice in 3 weeks (n=6).

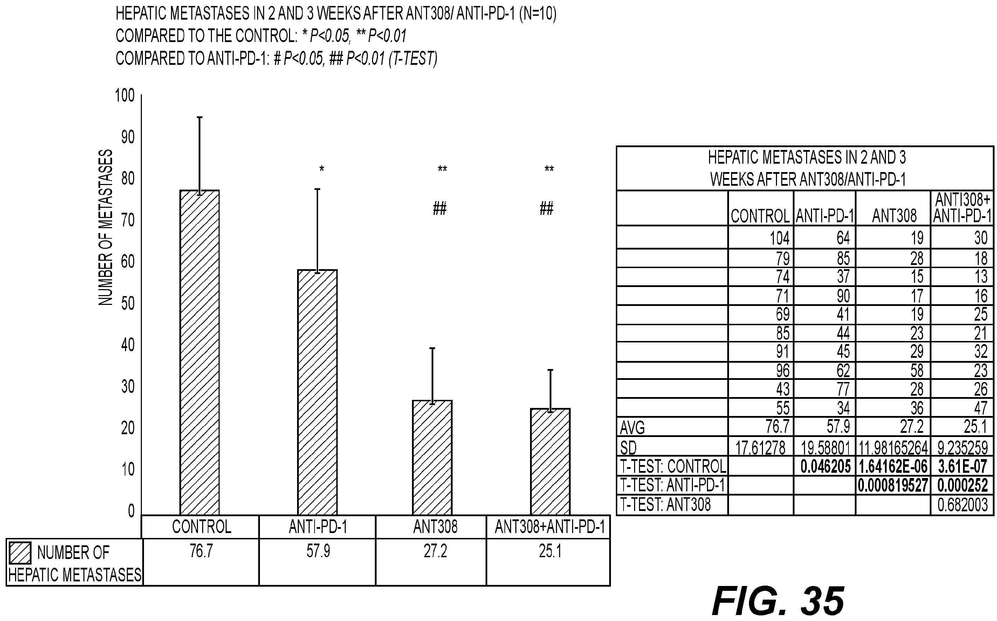

55. Figure 35 shows hepatic metastases in 2 and 3 weeks after ANT308/anti-PD-1 (n=10).

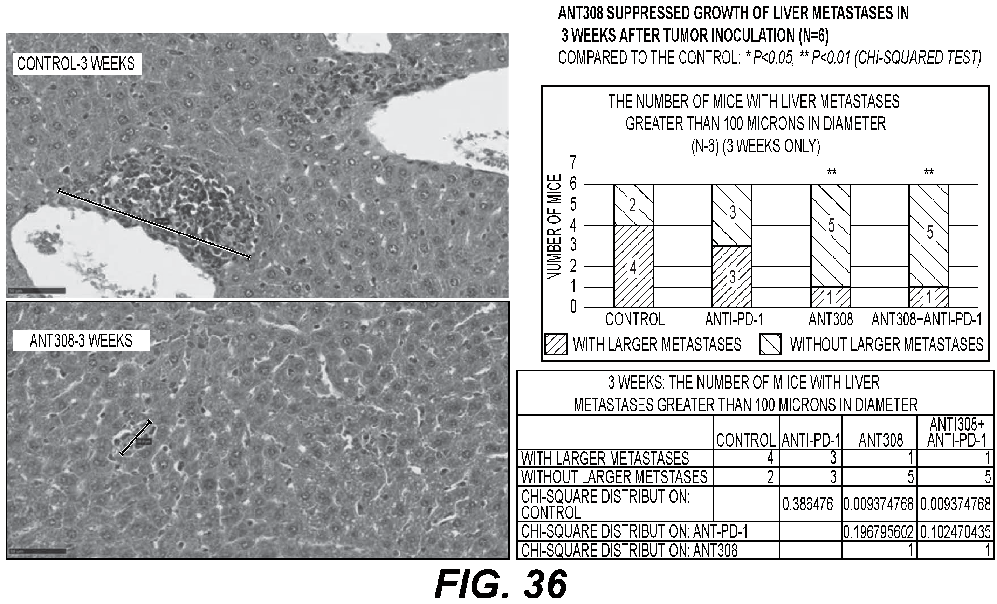

56. Figure 36 shows that ANT308 suppressed growth of liver metastases in 3 weeks after tumor inoculation (N=6).

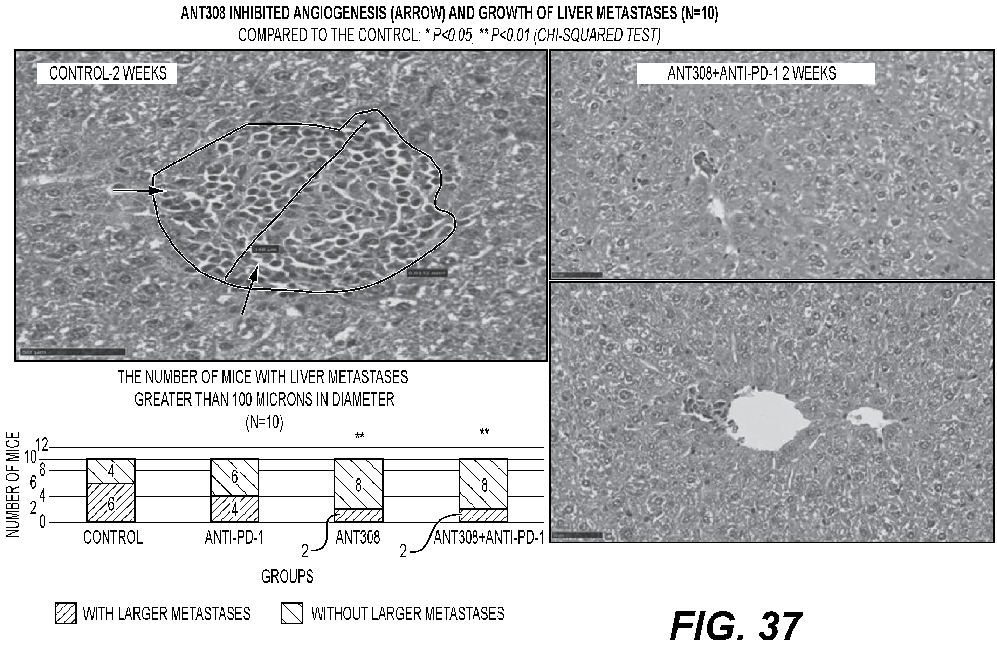

57. Figure 37 shows that ANT308 inhibited angiogenesis (arrow) and growth of liver metastases (N=10).

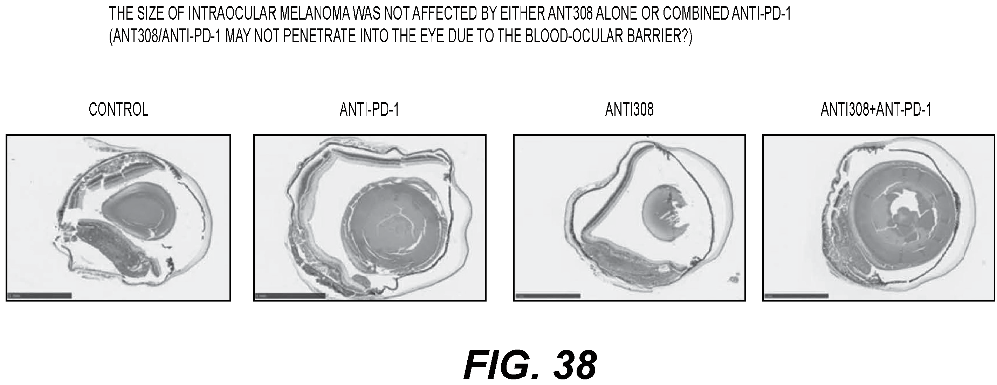

58. Figure 38 shows that the size of intraocular melanoma was not affected by either ANT308 alone or combined anti-PD-1.

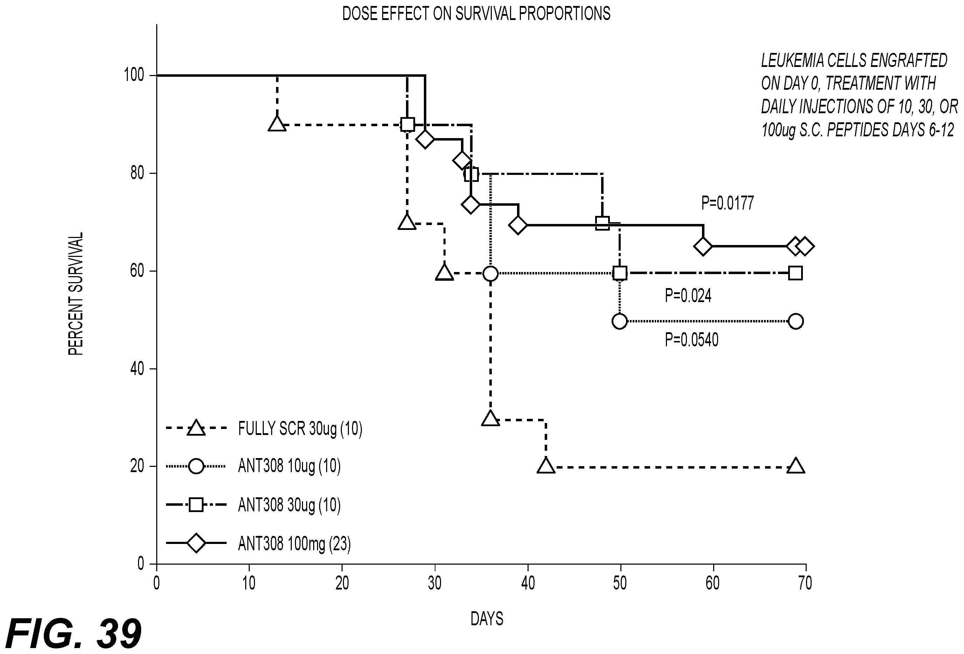

59. Figure 39 shows that VIP-R antagonist ANT308 induced dose-dependent clearance of C1498 leukemia and long-term survival in mice with AML.

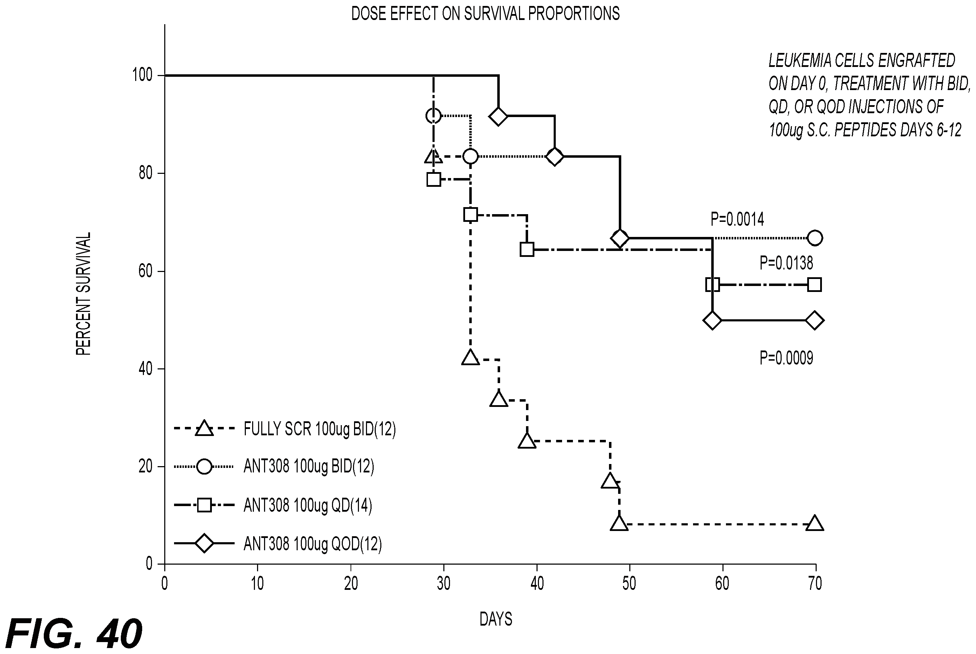

60. Figure 40 shows that VIP-R antagonist ANT308 induced schedule-dependent clearance of C1498 leukemia and long-term survival in mice.

### V. DETAILED DESCRIPTION

61. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### A. Definitions

62. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

63. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

64. In this specification and in the claims which follow, reference will be made to a number of terms which shall be defined to have the following meanings:

65. "Administration" to a subject or "administering" includes any route of introducing or delivering to a subject an agent. Administration can be carried out by any suitable route, including intravenous, intraperitoneal, and the like. Administration includes self-administration and the administration by another. "Administration" to a subject includes any route of introducing or delivering to a subject an agent. Administration can be carried out by any suitable route, including oral, topical, intravenous, subcutaneous, transcutaneous, transdermal, intramuscular, intra-joint, parenteral, intra-arteriole, intradermal, intraventricular, intracranial, intraperitoneal, intralesional, intranasal, rectal, vaginal, by inhalation, via an implanted reservoir, or via a transdermal patch, and the like. Administration includes self-administration and the administration by another.

66. "Optional" or "optionally" means that the subsequently described event or circumstance may or may not occur, and that the description includes instances where said event or circumstance occurs and instances where it does not.

67. The term "comprising" in reference to a peptide having an amino acid sequence refers a peptide that may contain additional N-terminal (amine end) or C-terminal (carboxylic acid end) amino acids, i.e., the term is intended to include the amino acid sequence within a larger peptide. The term "consisting of" in reference to a peptide having an amino acid sequence refers a peptide having the exact number of amino acids in the sequence and not more or having not more than a rage of amino acids expressly specified in the claim. In certain embodiments, the disclosure contemplates that the "N-terminus of a peptide may consist of an amino acid sequence," which refers to the N-terminus of the peptide having the exact number of amino acids in the sequence and not more or having not more than a rage of amino acids specified in the claim however the C-terminus may be connected to additional amino acids, e.g., as part of a larger peptide. Similarly, the disclosure contemplates that the "C-terminus of a peptide may consist of an amino acid sequence," which refers to the C-terminus of the peptide having the exact number of amino acids in the sequence and not more or having not more than a rage of amino acids specified in the claim however the N-terminus may be connected to additional amino acids, e.g., as part of a larger peptide.

68. An "increase" can refer to any change that results in a greater amount of a symptom, disease, composition, condition or activity. An increase can be any individual, median, or average increase in a condition, symptom, activity, composition in a statistically significant amount. Thus, the increase can be a 1, 2, 3, 4, 5, 6, 7, 8, 9, 10, 15, 20, 25, 30, 35, 40, 45, 50, 55, 60, 65, 70, 75, 80, 85, 90, 95, or 100% increase so long as the increase is statistically significant.

69. A "decrease" can refer to any change that results in a smaller amount of a symptom, disease, composition, condition, or activity. A substance is also understood to decrease the genetic output of a gene when the genetic output of the gene product with the substance is less relative to the output of the gene product without the substance. Also, for example, a decrease can be a change in the symptoms of a disorder such that the symptoms are less than previously observed. A decrease can be any individual, median, or average decrease in a condition, symptom, activity, composition in a statistically significant amount. Thus, the decrease can be a 1, 2, 3, 4, 5, 6, 7, 8, 9, 10, 15, 20, 25, 30, 35, 40, 45, 50, 55, 60, 65, 70, 75, 80, 85, 90, 95, or 100% decrease so long as the decrease is statistically significant.

70. "Inhibit," "inhibiting," and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10, 20, 30, 40, 50, 60, 70, 80, 90, 100%, or any amount of reduction in between as compared to native or control levels.

71. "Inhibitors" or "antagonists" of expression or of activity are used to refer to inhibitory molecules, respectively, identified using *in vitro* and *in vivo* assays for expression or activity of a described target protein, e.g., ligands, antagonists, and their homologs and mimetics. Inhibitors are agents that, e.g., inhibit expression or bind to, partially or totally block stimulation or enzymatic activity, decrease, prevent, delay activation, inactivate, desensitize, or down regulate the activity of the described target protein, e.g., antagonists. A control sample (untreated with inhibitors) are assigned a relative activity value of 100%. Inhibition of a described target protein is achieved when the activity value relative to the control is about 80%, optionally 50% or 25, 10%, 5% or 1%. As used herein, the terms "VIP antagonist" or "VIP receptor antagonist" are used interchangeably.

72. By "reduce" or other forms of the word, such as "reducing" or "reduction," is meant lowering of an event or characteristic (e.g., tumor growth). It is understood that this is typically in relation to some standard or expected value, in other words it is relative, but that it is not always necessary for the standard or relative value to be referred to. For example, "reduces tumor growth" means reducing the rate of growth of a tumor relative to a standard or a control.

73. By "prevent" or other forms of the word, such as "preventing" or "prevention," is meant to stop a particular event or characteristic, to stabilize or delay the development or progression of a particular event or characteristic, or to minimize the chances that a particular event or characteristic will occur. Prevent does not require comparison to a control as it is typically more absolute than, for example, reduce. As used herein, something could be reduced but not prevented, but something that is reduced could also be prevented. Likewise, something could be prevented but not reduced, but something that is prevented could also be reduced. It is understood that where reduce or prevent are used, unless specifically indicated otherwise, the use of the other word is also expressly disclosed.

74. The term "subject" refers to any individual who is the target of administration or treatment. The subject can be a vertebrate, for example, a mammal. In one aspect, the subject can be human, non-human primate, bovine, equine, porcine, canine, or feline. The subject can also be a guinea pig, rat, hamster, rabbit, mouse, or mole. Thus, the subject can be a human or veterinary patient. The term "patient" refers to a subject under the treatment of a clinician, e.g., physician.

75. The term "therapeutically effective amount" refers to the amount of the composition used is of sufficient quantity to ameliorate one or more causes or symptoms of a disease or disorder. Such amelioration only requires a reduction or alteration, not necessarily elimination.

76. The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

77. "Biocompatible" generally refers to a material and any metabolites or degradation products thereof that are generally non-toxic to the recipient and do not cause significant adverse effects to the subject.

78. "Comprising" is intended to mean that the compositions, methods, etc. include the recited elements, but do not exclude others. "Consisting essentially of" when used to define compositions and methods, shall mean including the recited elements, but excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of" shall mean excluding more than trace elements of other ingredients and substantial method steps for administering the compositions provided and/or claimed in this disclosure. Embodiments defined by each of these transition terms are within the scope of this disclosure.

79. "Composition" refers to any agent that has a beneficial biological effect. Beneficial biological effects include both therapeutic effects, e.g., treatment of a disorder or other undesirable physiological condition, and prophylactic effects, e.g., prevention of a disorder or other undesirable physiological condition. The terms also encompass pharmaceutically acceptable, pharmacologically active derivatives of beneficial agents specifically mentioned herein, including, but not limited to, a vector, polynucleotide, cells, salts, esters, amides, proagents, active metabolites, isomers, fragments, analogs, and the like. When the term "composition" is used, then, or when a particular composition is specifically identified, it is to be understood that the term includes the composition per se as well as pharmaceutically acceptable, pharmacologically active vector, polynucleotide, salts, esters, amides, proagents, conjugates, active metabolites, isomers, fragments, analogs, etc.

80. A "control" is an alternative subject or sample used in an experiment for comparison purposes. A control can be "positive" or "negative."

81. "Effective amount" of an agent refers to a sufficient amount of an agent to provide a desired effect. The amount of agent that is "effective" will vary from subject to subject, depending on many factors such as the age and general condition of the subject, the particular agent or agents, and the like. Thus, it is not always possible to specify a quantified "effective amount." However, an appropriate "effective amount" in any subject case may be determined by one of ordinary skill in the art using routine experimentation. Also, as used herein, and unless specifically stated otherwise, an "effective amount" of an agent can also refer to an amount covering both therapeutically effective amounts and prophylactically effective amounts. An "effective amount" of an agent necessary to achieve a therapeutic effect may vary according to factors such as the age, sex, and weight of the subject. Dosage regimens can be adjusted to provide the optimum therapeutic response. For example, several divided doses may be administered daily or the dose may be proportionally reduced as indicated by the exigencies of the therapeutic situation.

82. A "pharmaceutically acceptable" component can refer to a component that is not biologically or otherwise undesirable, i.e., the component may be incorporated into a pharmaceutical formulation provided by the disclosure and administered to a subject as described herein without causing significant undesirable biological effects or interacting in a deleterious manner with any of the other components of the formulation in which it is contained. When used in reference to administration to a human, the term generally implies the component has met the required standards of toxicological and manufacturing testing or that it is included on the Inactive Ingredient Guide prepared by the U.S. Food and Drug Administration.

83. "Pharmaceutically acceptable carrier" (sometimes referred to as a "carrier") means a carrier or excipient that is useful in preparing a pharmaceutical or therapeutic composition that is generally safe and non-toxic and includes a carrier that is acceptable for veterinary and/or human pharmaceutical or therapeutic use. The terms "carrier" or "pharmaceutically acceptable carrier" can include, but are not limited to, phosphate buffered saline solution, water, emulsions (such as an oil/water or water/oil emulsion) and/or various types of wetting agents. As used herein, the term "carrier" encompasses, but is not limited to, any excipient, diluent, filler, salt, buffer, stabilizer, solubilizer, lipid, stabilizer, or other material well known in the art for use in pharmaceutical formulations and as described further herein.

84. "Pharmacologically active" (or simply "active"), as in a "pharmacologically active" derivative or analog, can refer to a derivative or analog (e.g., a salt, ester, amide, conjugate, metabolite, isomer, fragment, etc.) having the same type of pharmacological activity as the parent compound and approximately equivalent in degree.

85. "Therapeutic agent" refers to any composition that has a beneficial biological effect. Beneficial biological effects include both therapeutic effects, e.g., treatment of a disorder or other undesirable physiological condition, and prophylactic effects, e.g., prevention of a disorder or other undesirable physiological condition (e.g., a non-immunogenic cancer). The terms also encompass pharmaceutically acceptable, pharmacologically active derivatives of beneficial agents specifically mentioned herein, including, but not limited to, salts, esters, amides, proagents, active metabolites, isomers, fragments, analogs, and the like. When the terms "therapeutic agent" is used, then, or when a particular agent is specifically identified, it is to be understood that the term includes the agent per se as well as pharmaceutically acceptable, pharmacologically active salts, esters, amides, proagents, conjugates, active metabolites, isomers, fragments, analogs, etc.

86. The term "prodrug" refers to an agent that is converted into a biologically active form in vivo. Prodrugs are often useful because, in some situations, they may be easier to administer than the parent compound. The prodrug may also have improved solubility in pharmaceutical compositions over the parent drug. A prodrug may be converted into the parent drug by various mechanisms, including enzymatic processes and metabolic hydrolysis. Typical prodrugs are pharmaceutically acceptable esters. Prodrugs include compounds wherein a hydroxy, amino or mercapto (thiol) group is bonded to any group that, when the prodrug of the active compound is administered to a subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound and the like.

87. "Therapeutically effective amount" or "therapeutically effective dose" of a composition (e.g. a composition comprising an agent) refers to an amount that is effective to achieve a desired therapeutic result. In some embodiments, a desired therapeutic result is the control of tumor growth. In some embodiments, a desired therapeutic result is the control of metastasis. In some embodiments, a desired therapeutic result is prevention of relapse. Therapeutically effective amounts of a given therapeutic agent will typically vary with respect to factors such as the type and severity of the disorder or disease being treated and the age, gender, and weight of the subject. The term can also refer to an amount of a therapeutic agent, or a rate of delivery of a therapeutic agent (e.g., amount over time), effective to facilitate a desired therapeutic effect, such as pain relief. The precise desired therapeutic effect will vary according to the condition to be treated, the tolerance of the subject, the agent and/or agent formulation to be administered (e.g., the potency of the therapeutic agent, the concentration of agent in the formulation, and the like), and a variety of other factors that are appreciated by those of ordinary skill in the art. In some instances, a desired biological or medical response is achieved following administration of multiple dosages of the composition to the subject over a period of days, weeks, or years.

88. The term "isolating" as used herein refers to isolation from a biological sample, i.e., blood, plasma, tissues, exosomes, or cells. As used herein the term "isolated," when used in the context of, e.g., a nucleic acid, refers to a nucleic acid of interest that is at least 60% free, at least 75% free, at least 90% free, at least 95% free, at least 98% free, and even at least 99% free from other components with which the nucleic acid is associated with prior to purification.

89. "Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom, Thus, a gene encodes a protein if transcription and translation of mRNA.

90. The term as used herein "engineered" and other grammatical forms thereof may refer to one or more changes of nucleic acids, such as nucleic acids within the genome of an organism. The term "engineered" may refer to a change, addition and/or deletion of a gene. Engineered cells can also refer to cells that contain added, deleted, and/or changed genes.

91. "Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide.)

92. The "fragments," whether attached to other sequences or not, can include insertions, deletions, substitutions, or other selected modifications of particular regions or specific amino acids residues, provided the activity of the fragment is not significantly altered or impaired compared to the nonmodified peptide or protein. These modifications can provide for some additional property, such as to remove or add amino acids capable of disulfide bonding, to increase its bio-longevity, to alter its secretory characteristics, etc. In any case, the fragment must possess a bioactive property, such as regulating the transcription of the target gene.

93. The term "gene" or "gene sequence" refers to the coding sequence or control sequence, or fragments thereof. A gene may include any combination of coding sequence and control sequence, or fragments thereof. Thus, a "gene" as referred to herein may be all or part of a native gene. A polynucleotide sequence as referred to herein may be used interchangeably with the term "gene", or may include any coding sequence, non-coding sequence or control sequence, fragments thereof, and combinations thereof. The term "gene" or "gene sequence" includes, for example, control sequences upstream of the coding sequence (for example, the ribosome binding site).

94. The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%,94%, 95%, 96%, 97%, 98%, 99% or higher identity over a specified region when compared and aligned for maximum correspondence over a comparison window or designated region) as measured using a BLAST or BLAST 2.0 sequence comparison algorithms with default parameters described below, or by manual alignment and visual inspection (see, e.g., NCBI web site or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 10 amino acids or 20 nucleotides in length, or more preferably over a region that is 10-50 amino acids or 20-50 nucleotides in length. As used herein, percent (%) nucleotide sequence identity is defined as the percentage of amino acids in a candidate sequence that are identical to the nucleotides in a reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN, ALIGN-2 or Megalign (DNASTAR) software. Appropriate parameters for measuring alignment, including any algorithms needed to achieve maximal alignment over the full-length of the sequences being compared can be determined by known methods.

95. For sequence comparisons, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

96. One example of an algorithm that is suitable for determining percent sequence identity and sequence similarity are the BLAST and BLAST 2.0 algorithms, which are described in Altschul et al. (1977) Nuc. Acids Res. 25:3389-3402, and Altschul et al. (1990) J. Mol. Biol. 215:403-410, respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al. (1990) J. Mol. Biol. 215:403-410). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) or 10, M=5, N=-4 and a comparison of both strands. For amino acid sequences, the BLASTP program uses as defaults a word length of 3, and expectation (E) of 10, and the BLOSUM62 scoring matrix (see Henikoff and Henikoff (1989) Proc. Natl. Acad. Sci. USA 89:10915) alignments (B) of 50, expectation (E) of 10, M=5, N=-4, and a comparison of both strands.

97. The BLAST algorithm also performs a statistical analysis of the similarity between two sequences (see, e.g., Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01.

98. The term "nucleic acid" as used herein means a polymer composed of nucleotides, e.g. deoxyribonucleotides (DNA) or ribonucleotides (RNA). The terms "ribonucleic acid" and "RNA" as used herein mean a polymer composed of ribonucleotides. The terms "deoxyribonucleic acid" and "DNA" as used herein mean a polymer composed of deoxyribonucleotides. (Used together with "polynucleotide" and "polypeptide".)

99. Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

100. As used herein, "operatively linked" can indicate that the regulatory sequences useful for expression of the coding sequences of a nucleic acid are placed in the nucleic acid molecule in the appropriate positions relative to the coding sequence so as to effect expression of the coding sequence. This same definition is sometimes applied to the arrangement of coding sequences and/or transcription control elements (e.g. promoters, enhancers, and termination elements), and/or selectable markers in an expression vector. The term "operatively linked" can also refer to the arrangement of polypeptide segments within a single polypeptide chain, where the individual polypeptide segments can be, without limitation, a protein, fragments thereof, linking peptides, and/or signal peptides. The term operatively linked can refer to direct fusion of different individual polypeptides within the single polypeptides or fragments thereof where there are no intervening amino acids between the different segments as well as when the individual polypeptides are connected to one another via one or more intervening amino acids.

101. The term "polynucleotide" refers to a single or double stranded polymer composed of nucleotide monomers.

102. The term "polypeptide" refers to a compound made up of a single chain of D- or L-amino acids or a mixture of D- and L-amino acids joined by peptide bonds.

103. The terms "peptide," "protein," and "polypeptide" are used interchangeably to refer to a natural or synthetic molecule comprising two or more amino acids linked by the carboxyl group of one amino acid to the alpha amino group of another.

104. The term "promoter" as used herein is defined as a DNA sequence recognized by the synthetic machinery of the cell, or introduced synthetic machinery, required to initiate the specific transcription of a polynucleotide sequence.

105. As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

106. The term "variant" as used herein refers to a polypeptide or polynucleotide that differs from a reference polypeptide or polynucleotide, but retains essential properties. A typical variant of a polypeptide differs in amino acid sequence from another, reference, polypeptide. Generally, differences are limited so that the sequences of the reference polypeptide and the variant are closely similar overall (homologous) and, in many regions, identical. A variant and reference polypeptide may differ in amino acid sequence by one or more modifications (e.g., substitutions, additions, and/or deletions).

107. The term "cancer" as used herein is defined as disease characterized by the rapid and uncontrolled growth of aberrant cells. Cancer cells can spread locally or through the bloodstream and lymphatic system to other parts of the body, Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

108. As used herein, the term "metastasis" is meant to refer to the process in which cancer cells originating in one organ or part of the body, with or without transit by a body fluid, and relocate to another part of the body and continue to replicate. Metastasized cells can subsequently form tumors which may further metastasize. Metastasis thus refers to the spread of cancer, from the part of the body where it originally occurred, to other parts of the body.

109. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

### B. Compositions

110. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular VIP-R antagonist is disclosed and discussed and a number of modifications that can be made to a number of molecules including the VIP-R antagonist are discussed, specifically contemplated is each and every combination and permutation of VIP-R antagonist and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

111. The terms "vasoactive intestinal peptide" and "VIP" refer to HSDAVFTDNYTRLRKQMAVKKYLNSILN (SEQ ID NO: 2) unless the context suggests otherwise. VIP is a multifunctional endogenous polypeptide that modulates both innate and adaptive immunity at multiple levels of immune cell differentiation and activation. VIP is typically secreted by a variety of cells such as neurons (in both the central and peripheral nervous systems) B-cells, T-cells, and accessory cells. VIP and the closely related neuropeptide pituitary adenylyl cyclase-activating polypeptide (PACAP) bind to three known receptors-VPAC1, VPAC2, and PAC1. It is believed that T-cells and dendritic cells (DC) express VPAC1 and VPAC2, but not PAC1. PAC1 is mainly expressed on neuron and endocrine cells in the brain and pituitary and adrenal glands, and in most forms selectively binds PACAP.

112. Some cancers are caused by viruses, and traditional vaccines against those viruses, such as HPV vaccine and Hepatitis B vaccine, will prevent those cancers. It is contemplated that peptide disclosed herein can be administered in combination with these vaccines to improve treatment efficacy.

113. It is believed that cancer cells arise and are destroyed by the immune system, and that cancer forms when the immune system fails to destroy them. One approach to cancer vaccination is to separate proteins from cancer cells and immunize cancer patients against those proteins, stimulating an immune reaction that kills the cancer cells. Cancer vaccines are contemplated for the treatment of acute myeloid leukemia, multiple myeloma, lymphoma, breast, lung, colon, skin, kidney, prostate, and other cancers.

114. In certain embodiments, the disclosure relates to treating cancers by administering any vasoactive intestinal peptide receptor (VIP-R) antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) in combination with cancer antigens. Other VIP-R antagonists or VIP antagonists are also reported in U.S. Patent Nos. 6,630,124 and 5,217,953, which are incorporated herein by reference in their entireties.

115. Prevention of the action of microorganisms may be controlled by addition of any of various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

116. It is contemplated that any of the peptides disclose herein may be modified with hydrocarbon or polyethylene glycol groups in order to provide improve properties such as solubility, bioavailability, and/or biological degradation.

117. In certain embodiments, this disclosure relates to methods of coupling any vasoactive intestinal peptide receptor (VIP-R) antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof, as well as, any VIP-R antagonist/VIP antagonist disclosed in U.S. Patent Nos. 6,630,124 and 5,217,953, which are incorporated herein by reference in their entireties) or the nucleic acid encoding the VIP-R antagonist disclosed herein to a nanoparticle. Accordingly, in some embodiments, the VIP-R antagonist disclosed herein or the nucleic acid encoding the VIP-R antagonist disclosed herein is conjugated to and/or encapsulated within a nanoparticle. In certain embodiments, the nanoparticle is comprised of poloxamer-stabilized polypropylene sulfide. The term "nanoparticle" as used herein refers to a particle or structure which typically ranges from about 1 nm to about 1000 nm in size. In certain embodiments, the nanoparticle has a diameter of between about 10 and about 100 nm. In certain embodiments, the nanoparticle has a diameter between about 20 and about 50 nm, preferably about 30 nm. In certain embodiments, the nanoparticle has a diameter from about 50 nm to about 500 nm size, more preferably from about 50 nm to about 350 nm size, more preferably from about 100 nm to about 250 nm size.

118. In certain embodiments, the disclosure contemplates using particles disclosed herein when the peptide sequence couple to the nanoparticle is contains any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, as well as, any VIP-R antagonist disclosed in U.S. Patent Nos. 6,630,124 and 5,217,953, which are incorporated herein by reference in their entireties) plus a C-terminal linker peptide, GGGGSC (SEQ ID NO: 22). In certain embodiments, the chemical linkage between the peptides disclosed herein and the nanoparticles is a disulfide bond.

119. In certain embodiments, the disclosure relates to recombinant peptides comprising sequences disclosed herein or fusions thereof wherein the amino terminal end or the carbon terminal end of the amino acid sequence are optionally attached to a heterologous amino acid sequence, label, or reporter molecule. A "label" refers to a detectable compound or composition that is conjugated directly or indirectly to another molecule, such as an antibody or a protein, to facilitate detection of that molecule. Specific, non-limiting examples of labels include fluorescent tags, enzymatic linkages, and radioactive isotopes. In one example, a "label receptor" refers to incorporation of a heterologous polypeptide in the receptor. A label includes the incorporation of a radiolabeled amino acid or the covalent attachment of biotinyl moieties to a polypeptide that can be detected by marked avidin (for example, streptavidin containing a fluorescent marker or enzymatic activity that can be detected by optical or colorimetric methods). Various methods of labeling polypeptides and glycoproteins are known in the art and may be used. Examples of labels for polypeptides include, but are not limited to, the following: radioisotopes or radionuclides (such as ³⁵S or ¹³¹I) fluorescent labels (such as fluorescein isothiocyanate (FITC), rhodamine, lanthanide phosphors), enzymatic labels (such as horseradish peroxidase, beta-galactosidase, luciferase, alkaline phosphatase), chemiluminescent markers, biotinyl groups, predetermined peptide epitopes recognized by a secondary reporter (such as a leucine zipper pair sequences, binding sites for secondary antibodies, metal binding domains, epitope tags), or magnetic agents, such as gadolinium chelates. In some embodiments, labels are attached by spacer arms of various lengths to reduce potential steric hindrance.

### 1. Peptides

### a) Protein variants

120. As disclosed herein there are numerous variants of the vasoactive intestinal peptide receptor (VIP-R) antagonists that are known and herein contemplated. In addition, to the known functional variants there are derivatives of the VIP-R antagonists which also function in the disclosed methods and compositions. Protein variants and derivatives are well understood to those of skill in the art and in can involve amino acid sequence modifications. For example, amino acid sequence modifications typically fall into one or more of three classes: substitutional, insertional or deletional variants. Insertions include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary protein structure. Substitutional variants are those in which at least one residue has been removed and a different residue inserted in its place. Such substitutions generally are made in accordance with the following Tables 1 and 2 and are referred to as conservative substitutions.

**TABLE 1:Amino Acid Abbreviations**

| **Amino Acid** | **Abbreviations** | |
|---|---|---|
| Alanine | Ala | A |
| allosoleucine | AIle | |
| Arginine | Arg | R |
| asparagine | Asn | N |
| aspartic acid | Asp | D |
| Cysteine | Cys | C |
| glutamic acid | Glu | E |
| Glutamine | Gln | Q |
| Glycine | Gly | G |
| Histidine | His | H |
| Isolelucine | Ile | I |
| Leucine | Leu | L |
| Lysine | Lys | K |
| phenylalanine | Phe | F |
| proline | Pro | P |
| pyroglutamic acid | pGlu | |
| Serine | Ser | S |
| Threonine | Thr | T |
| Tyrosine | Tyr | Y |
| Tryptophan | Trp | W |
| Valine | Val | V |

**TABLE 2:Amino Acid Substitutions**

| Original Residue Exemplary Conservative Substitutions, others are known in the art. | |
|---|---|
| Ala | Ser |
| Arg | Lys; Gln |
| Asn | Gln; His |
| Asp | Glu |
| Cys | Ser |
| Gln | Asn, Lys |
| Glu | Asp |
| Gly | Pro |
| His | Asn;Gln |
| Ile | Leu; Val |
| Leu | Ile; Val |
| Lys | Arg; Gln |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

121. Substantial changes in function or immunological identity are made by selecting substitutions that are less conservative than those in Table 2, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

122. For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.

123. Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

124. Certain post-translational derivatizations are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine, arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

125. It is understood that one way to define the variants and derivatives of the disclosed proteins herein is through defining the variants and derivatives in terms of homology/identity to specific known sequences. For example, SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and SEQ ID NO: 16 set forth a particular sequence of VIP-R antagonist. Specifically disclosed are variants of these and other proteins herein disclosed which have at least, 70% or 75% or 80% or 85% or 90% or 95% homology to the stated sequence. Those of skill in the art readily understand how to determine the homology of two proteins. For example, the homology can be calculated after aligning the two sequences so that the homology is at its highest level.

126. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

127. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989.

128. It is understood that the description of conservative mutations and homology can be combined together in any combination, such as embodiments that have at least 70% homology to a particular sequence wherein the variants are conservative mutations.

129. As this specification discusses various proteins and protein sequences it is understood that the nucleic acids that can encode those protein sequences are also disclosed. This would include all degenerate sequences related to a specific protein sequence, i.e. all nucleic acids having a sequence that encodes one particular protein sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed protein sequence. It is also understood that while no amino acid sequence indicates what particular DNA sequence encodes that peptide or protein within an organism, where particular variants of a disclosed VIP-R antagonist are disclosed herein, the known nucleic acid sequence that encodes peptide are known and herein disclosed and described.

130. It is understood that there are numerous amino acid and peptide analogs which can be incorporated into the disclosed compositions. For example, there are numerous D amino acids or amino acids which have a different functional substituent then the amino acids shown in Table 1 and Table 2. The opposite stereo isomers of naturally occurring peptides are disclosed, as well as the stereo isomers of peptide analogs. These amino acids can readily be incorporated into polypeptide chains by charging tRNA molecules with the amino acid of choice and engineering genetic constructs that utilize, for example, amber codons, to insert the analog amino acid into a peptide chain in a site-specific way.

131. Molecules can be produced that resemble peptides, but which are not connected via a natural peptide linkage. For example, linkages for amino acids or amino acid analogs can include CH₂NH--, --CH₂S--, --CH₂--CH₂ --, --CH=CH-- (cis and trans), --COCH₂ --, --CH(OH)CH₂--, and --CHH₂SO-(These and others can be found in Spatola, A. F. in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci (1980) pp. 463-468; Hudson, D. et al., Int J Pept Prot Res 14:177-185 (1979) (--CH₂NH--, CH₂CH₂--); Spatola et al. Life Sci 38:1243-1249 (1986) (-CH H₂--S); Hann J. Chem. Soc Perkin Trans. I 307-314 (1982) (--CH--CH--, cis and trans); Almquist et al. J. Med. Chem. 23:1392-1398 (1980) (--COCH₂--); Jennings-White et al. Tetrahedron Lett 23:2533 (1982) (--COCH₂--); Szelke et al. European Appln, EP 45665 CA (1982): 97:39405 (1982) (--CH(OH)CH₂--); Holladay et al. Tetrahedron. Lett 24:4401-4404 (1983) (--C(OH)CH₂--); and Hruby Life Sci 31:189-199 (1982) (--CH₂--S--); each of which is incorporated herein by reference. A particularly preferred non-peptide linkage is --CH₂NH--. It is understood that peptide analogs can have more than one atom between the bond atoms, such as b-alanine, g-aminobutyric acid, and the like.

132. Amino acid analogs and analogs and peptide analogs often have enhanced or desirable properties, such as, more economical production, greater chemical stability, enhanced pharmacological properties (half-life, absorption, potency, efficacy, etc.), altered specificity (e.g., a broad-spectrum of biological activities), reduced antigenicity, and others.

133. D-amino acids can be used to generate more stable peptides, because D amino acids are not recognized by peptidases and such. Systematic substitution of one or more amino acids of a consensus sequence with a D-amino acid of the same type (e.g., D-lysine in place of L-lysine) can be used to generate more stable peptides. Cysteine residues can be used to cyclize or attach two or more peptides together. This can be beneficial to constrain peptides into particular conformations.

134. In certain embodiments, this disclosure contemplates derivatives of the VIP-R antagonists disclose herein wherein one or more amino acids are substituted with chemical groups to improve pharmacokinetic properties such as solubility and serum half-life, optionally connected through a linker. In certain embodiments, such a derivative may be a prodrug wherein the substituent or linker is biodegradable, or the substituent or linker is not biodegradable. In certain embodiments, contemplated substituents include a saccharide, polysaccharide, acetyl, fatty acid, lipid, and/or polyethylene glycol. The substituent may be covalently bonded through the formation of amide bonds on the C-terminus or N-terminus of the peptide optionally connected through a linker. In certain embodiments, it is contemplated that the substituent may be covalently bonded through an amino acid within the peptide, e.g. through an amine side chain group such as lysine or an amino acid containing a carboxylic acid side chain group such as aspartic acid or glutamic acid, within the peptide comprising a sequence disclosed herein. In certain embodiments, it is contemplated that the substituent may be covalently bonded through a cysteine in a sequence disclosed herein optionally connected through a linker. In certain embodiments, a substituent is connected through a linker that forms a disulfide with a cysteine amino acid side group.

135. The term "substituted" refers to a molecule wherein at least one hydrogen atom is replaced with a substituent. When substituted, one or more of the groups are "substituents." The molecule may be multiply substituted. In the case of an oxo substituent ("=O"), two hydrogen atoms are replaced. Example substituents within this context may include halogen, hydroxy, alkyl, alkoxy, nitro, cyano, oxo, carbocyclyl, carbocycloalkyl, heterocarbocyclyl, heterocarbocycloalkyl, aryl, arylalkyl, heteroaryl, heteroarylalkyl, -NRaRb, -NRaC(=O)Rb, -NRaC(=O)NRaNRb,-NRaC(=O)ORb, - NRaSO₂Rb, -C(=O)Ra, -C(=O)ORa, -C(=O)NRaRb, -OC(=O)NRaRb, -ORa,-SRa, -SORa, - S(=O)₂Ra, -OS(=O)₂Ra and -S(=O)₂ORa. Ra and Rb in this context may be the same or different and independently hydrogen, halogen hydroxyl, alkyl, alkoxy, alkyl, amino, alkylamino dialkylamino, carbocyclyl, carbocycloalkyl, heterocarbocyclyl, heterocarbocycloalkyl, aryl, arylalkyl, heteroaryl, and heteroarylalkyl. The substituents may further optionally be substituted.

136. As used herein, a "lipid" group refers to a hydrophobic group that is naturally or non-naturally occurring that is highly insoluble in water. As used herein a lipid group is considered highly insoluble in water when the point of connection on the lipid is replaced with a hydrogen and the resulting compound has a solubility of less than 0.63 x 10⁻⁴ % w/w (at 25 °C) in water, which is the percent solubility of octane in water by weight. See Solvent Recovery Handbook, 2nd Ed, Smallwood, 2002 by Blackwell Science, page 195. Examples of naturally occurring lipids include saturated or unsaturated hydrocarbon chains found in fatty acids, glycerolipids, cholesterol, steroids, polyketides, and derivatives. Non-naturally occurring lipids include derivatives of naturally occurring lipids, acrylic polymers, aromatic, and alkylated compounds and derivatives thereof.

137. For example, if a disclosed peptide or a pharmaceutically acceptable form of the peptide contains a carboxylic acid functional group, a prodrug can comprise a pharmaceutically acceptable ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as beta-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

138. If a disclosed peptide or a pharmaceutically acceptable form of the peptide contains an alcohol functional group, a prodrug can be formed by the replacement of the hydrogen atom of the alcohol group with a group such as (C₁-C₆)alkanoyloxymethyl, 1-(( C₁-C₆)alkanoyloxy) ethyl, 1-methyl-1((C₁-C₆)alkanoyloxy)ethyl (C₁-C₆)alkoxycarbonyloxymethyl, -N-(C₁-C₆)alkoxycarbonylaminomethyl, succinoyl, (C₁-C₆)alkanoyl, alpha-amino(C₁-C₄)alkanoyl, arylacyl and alpha-aminoacyl, or alpha-aminoacyl-alpha-aminoacyl, where each alpha-aminoacyl group is independently selected from naturally occurring L-amino acids P(O)(OH)₂, -P(O)(O(C₁-C₆)alkyl)₂, and glycosyl (the radical resulting from the removal of a hydroxyl group of the hemiacetal form of a carbohydrate).

139. If a disclosed peptide or a pharmaceutically acceptable form of the peptide incorporates an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, benzyl, a natural alpha-aminoacyl, -C(OH)C(O)OY₁ wherein Y¹ is H, (C₁-C₆)alkyl or benzyl, -C(OY₂)Y₃ wherein Y₂ is (C₁-C₄) alkyl and Y₃ is (C₁-C₆)alkyl, carboxy(C₁-C₆)alkyl, amino(C₁-C₄)alkyl or mono-Nor di-N,N-(C₁-C₆)alkylaminoalkyl, -C(Y₄)Y₅ wherein Y₄ is H or methyl and Y₅ is mono-N- or di-N,N-( C₁-C₆)alkylamino, morpholino, piperidin-1-yl or pyrrolidin-1-yl.

140. As used herein, "pharmaceutically acceptable esters" include, but are not limited to, alkyl, alkenyl, alkynyl, aryl, arylalkyl, and cycloalkyl esters of acidic groups, including, but not limited to, carboxylic acids, phosphoric acids, phosphinic acids, sulfonic acids, sulfinic acids, and boronic acids.

141. As used herein, "pharmaceutically acceptable enol ethers" include, but are not limited to, derivatives of formula -C=C(OR) where R can be selected from alkyl, alkenyl, alkynyl, aryl, aralkyl, and cycloalkyl. Pharmaceutically acceptable enol esters include, but are not limited to, derivatives of formula -C=C(OC(O)R) where R can be selected from hydrogen, alkyl, alkenyl, alkynyl, aryl, aralkyl, and cycloalkyl.

142. A "linking group" refers to any variety of molecular arrangements that can be used to bridge to molecular moieties together. An example formula may be -Rₘ- wherein R is selected individually and independently at each occurrence as: -CRₘRₘ-, -CHRₘ-, -CH-, -C-, -CH₂-, -C(OH)Rₘ, -C(OH)(OH)-, -C(OH)H, -C(Hal)Rₘ-, -C(Hal)(Hal)-, -C(Hal)H-, -C(N₃)Rₘ-, -C(CN)Rₘ-, -C(CN)(CN)-, -C(CN)H-, -C(N₃)(N₃)-, -C(N₃)H-, -O-, -S-, -N-, -NH-, -NRₘ-, -(C=O)-, -(C=NH)-, -(C=S)-, -(C=CH₂)-, which may contain single, double, or triple bonds individually and independently between the R groups. If an R is branched with an Rₘ it may be terminated with a group such as -CH₃, -H, -CH=CH₂, -CCH, -OH, -SH, -NH₂, -N₃, -CN, or -Hal, or two branched Rs may form a cyclic structure. It is contemplated that in certain instances, the total Rs or "m" may be less than 100, or 50, or 25, or 10. Examples of linking groups include bridging alkyl groups and alkoxyalkyl groups. Linking groups may be substituted with one or more substituents.

### 2. Homology/identity

143. It is understood that one way to define any known variants and derivatives or those that might arise, of the disclosed genes and proteins herein is through defining the variants and derivatives in terms of homology to specific known sequences. For example, any of SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and SEQ ID NO: 16 sets forth a particular sequence of a VIP-R antagonist disclosed herein. Specifically disclosed are variants of these and other genes and proteins herein disclosed which have at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 percent homology to the stated sequence. Those of skill in the art readily understand how to determine the homology of two proteins or nucleic acids, such as genes. For example, the homology can be calculated after aligning the two sequences so that the homology is at its highest level.

144. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

145. The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment.

### 3. Pharmaceutical carriers/Delivery of pharmaceutical products

146. In certain embodiments, the disclosure contemplates pharmaceutical composition comprising peptide disclosed herein, or nanoparticle thereof, or optionally other pharmaceutical agent, or pharmaceutically acceptable salts thereof, and a pharmaceutically acceptable excipient.

147. In certain embodiments, this disclosure relates to compositions such as pharmaceutical compositions and cell growth media comprising peptides disclosed herein. In certain embodiments, this disclosure relates to pharmaceutical compositions comprising any vasoactive intestinal peptide receptor (VIP-R) disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof , as well as, any VIP-R antagonist disclosed in U.S. Patent Nos. 6,630,124 and 5,217,953, which are incorporated herein by reference in their entireties) and pharmaceutically acceptable excipient. In certain embodiments, the pharmaceutical composition is in the form of a capsule, tablets, pill, powder, or granule. In certain embodiments, the pharmaceutical composition is in the form of a sterilized pH buffered aqueous salt solution. In certain embodiments, the pharmaceutical composition is in the form of a container configured to spray a liquid or sealed container with a propellant.

148. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

149. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

150. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.

151. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

152. The compositions, including antibodies, can be used therapeutically in combination with a pharmaceutically acceptable carrier.

153. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

154. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

155. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

156. The pharmaceutical composition may be administered in a number of ways depending on whether local or systemic treatment is desired, and on the area to be treated. Administration may be topical (including ophthalmic, vaginal, rectal, intranasal), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermal.

157. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

158. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

159. Compositions for oral administration include powders or granules, suspensions or solutions in water or non-aqueous media, capsules, sachets, or tablets. Thickeners, flavorings, diluents, emulsifiers, dispersing aids or binders may be desirable.

160. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic

acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

161. In certain embodiment, this disclosure contemplates pharmaceutical compositions comprising peptide disclosed herein or nanoparticle thereof, and agents disclosed herein and pharmaceutically acceptable excipient. In certain embodiments, this disclosure contemplates the production of a medicament comprising peptide disclosed herein, or nanoparticle thereof, or agents disclosed herein and uses for methods disclosed herein.

### b) Therapeutic Uses

162. Effective dosages and schedules for administering the compositions may be determined empirically, and making such determinations is within the skill in the art. The dosage ranges for the administration of the compositions are those large enough to produce the desired effect in which the symptoms of the disorder are affected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above. For peptides disclosed herein or nanoparticle thereof, or other agents, the dosage administered to a patient is typically 0.0001 mg/kg to 100 mg/kg of the patient's body weight. Preferably, the dosage administered to a patient is between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight. Further, the dosage and frequency of administration of peptides disclosed herein or nanoparticle thereof or agent may be reduced by enhancing uptake and tissue penetration by modifications such as, for example, lipidation and the inclusion of natural or artificial pulmonary surfactants.

### 4. Nucleic acids

163. The term "nucleic acid" refers to a polymer of nucleotides, or a polynucleotide. The term is used to designate a single molecule, or a collection of molecules. Nucleic acids may be single stranded or double stranded and may include coding regions and regions of various control elements, as described below.

164. There are a variety of molecules disclosed herein that are nucleic acid based, including for example the nucleic acids that encode, for example, any of the vasoactive intestinal peptide receptor (VIP-R) set forth in SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, fragments thereof, or analogs thereof, as well as various functional nucleic acids or any VIP-R antagonist/VIP antagonist disclosed in U.S. Patent Nos. 6,630,124 and 5,217,953, which are incorporated herein by reference in their entireties. The disclosed nucleic acids are made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if, for example, an antisense molecule is introduced into a cell or cell environment through for example exogenous delivery, it is advantageous that the antisense molecule be made up of nucleotide analogs that reduce the degradation of the antisense molecule in the cellular environment.

### a) Nucleotides and related molecules

165. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. A non-limiting example of a nucleotide would be 3'-AMP (3'-adenosine monophosphate) or 5'-GMP (5'-guanosine monophosphate). There are many varieties of these types of molecules available in the art and available herein.

166. The terms "a nucleic acid sequence encoding" a specified peptide refers to a nucleic acid sequence comprising the coding region of peptide or in other words the nucleic acid sequence that encodes peptide product. The coding region may be present in either a cDNA, genomic DNA or RNA form. When present in a DNA form, the oligonucleotide, polynucleotide, or nucleic acid may be single-stranded (i.e., the sense strand) or double-stranded. Suitable control elements such as enhancers/promoters, splice junctions, polyadenylation signals, etc. may be placed in close proximity to the coding region if needed to permit proper initiation of transcription and/or correct processing of the primary RNA transcript. Alternatively, the coding region utilized in expression vectors may contain endogenous enhancers/promoters, splice junctions, intervening sequences, polyadenylation signals, etc. or a combination of both endogenous and exogenous control elements.

167. A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties. There are many varieties of these types of molecules available in the art and available herein.

168. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid. There are many varieties of these types of molecules available in the art and available herein.

169. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989, 86, 6553-6556). There are many varieties of these types of molecules available in the art and available herein.

170. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

171. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### b) Sequences

172. There are a variety of sequences related to the VIP-R antagonists disclosed herein, all of which are encoded by nucleic acids or are nucleic acids. The sequences for the human analogs of these genes, as well as other analogs, and alleles of these genes, and splice variants and other types of variants, are available in a variety of protein and gene databases, including Genbank. Those of skill in the art understand how to resolve sequence discrepancies and differences and to adjust the compositions and methods relating to a particular sequence to other related sequences. Primers and/or probes can be designed for any given sequence given the information disclosed herein and known in the art.

### 5. Nucleic Acid Delivery

173. In the methods described above which include the administration and uptake of exogenous DNA into the cells of a subject (i.e., gene transduction or transfection), the disclosed nucleic acids can be in the form of naked DNA or RNA, or the nucleic acids can be in a vector for delivering the nucleic acids to the cells, whereby the antibody-encoding DNA fragment is under the transcriptional regulation of a promoter, as would be well understood by one of ordinary skill in the art. The vector can be a commercially available preparation, such as an adenovirus vector (Quantum Biotechnologies, Inc. (Laval, Quebec, Canada). Delivery of the nucleic acid or vector to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the disclosed nucleic acid or vector can be delivered *in vivo* by electroporation, the technology for which is available from Genetronics, Inc. (San Diego, CA) as well as by means of a SONOPORATION machine (ImaRx Pharmaceutical Corp., Tucson, AZ).

174. As one example, vector delivery can be via a viral system, such as a retroviral vector system which can package a recombinant retroviral genome (see e.g., Pastan et al., Proc. Natl. Acad. Sci. U.S.A. 85:4486, 1988; Miller et al., Mol. Cell. Biol. 6:2895, 1986). The recombinant retrovirus can then be used to infect and thereby deliver to the infected cells nucleic acid encoding a broadly neutralizing antibody (or active fragment thereof). The exact method of introducing the altered nucleic acid into mammalian cells is, of course, not limited to the use of retroviral vectors. Other techniques are widely available for this procedure including the use of adenoviral vectors (Mitani et al., Hum. Gene Ther. 5:941-948, 1994), adeno-associated viral (AAV) vectors (Goodman et al., Blood 84:1492-1500, 1994), lentiviral vectors (Naidini et al., Science 272:263-267, 1996), pseudotyped retroviral vectors (Agrawal et al., Exper. Hematol. 24:738-747, 1996). Physical transduction techniques can also be used, such as liposome delivery and receptor-mediated and other endocytosis mechanisms (see, for example, Schwartzenberger et al., Blood 87:472-478, 1996). This disclosed compositions and methods can be used in conjunction with any of these or other commonly used gene transfer methods.

175. As one example, if the antibody-encoding nucleic acid is delivered to the cells of a subject in an adenovirus vector, the dosage for administration of adenovirus to humans can range from about 10⁷ to 10⁹ plaque forming units (pfu) per injection but can be as high as 10¹² pfu per injection (Crystal, Hum. Gene Ther. 8:985-1001, 1997; Alvarez and Curiel, Hum. Gene Ther. 8:597-613, 1997). A subject can receive a single injection, or, if additional injections are necessary, they can be repeated at six month intervals (or other appropriate time intervals, as determined by the skilled practitioner) for an indefinite period and/or until the efficacy of the treatment has been established.

176. Parenteral administration of the nucleic acid or vector, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. For additional discussion of suitable formulations and various routes of administration of therapeutic compounds, see, e.g., Remington: The Science and Practice of Pharmacy (19th ed.) ed. A.R. Gennaro, Mack Publishing Company, Easton, PA 1995.

### 6. Expression systems

177. The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors and may contain upstream elements and response elements.

178. Protein "expression systems" refer to in vivo and in vitro (cell free) systems. Systems for recombinant protein expression typically utilize cells transfecting with a DNA expression vector that contains the template. The cells are cultured under conditions such that they translate the desired protein. Expressed proteins are extracted for subsequent purification. In vivo protein expression systems using prokaryotic and eukaryotic cells are well known. Also, some proteins are recovered using denaturants and protein-refolding procedures. In vitro (cell-free) protein expression systems typically use translation-compatible extracts of whole cells or compositions that contain components sufficient for transcription, translation and optionally post-translational modifications such as RNA polymerase, regulatory protein factors, transcription factors, ribosomes, tRNA cofactors, amino acids and nucleotides. In the presence of an expression vectors, these extracts and components can synthesize proteins of interest. Cell-free systems typically do not contain proteases and enable labeling of the protein with modified amino acids. Some cell free systems incorporated encoded components for translation into the expression vector. See, e.g., Shimizu et al., Cell-free translation reconstituted with purified components, 2001, Nat. Biotechnol., 19, 751-755 and Asahara & Chong, Nucleic Acids Research, 2010, 38(13): e141, both hereby incorporated by reference in their entirety.

### a) Viral Promoters and Enhancers

179. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a *HindIII* E restriction fragment (Greenway, P.J. et al., Gene 18: 355-360 (1982)). Of course, promoters from the host cell or related species also are useful herein.

180. Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

181. The promotor and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

182. In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

183. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

184. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### b) Markers

185. The viral vectors can include nucleic acid sequence encoding a marker product. This marker product is used to determine if the gene has been delivered to the cell and once delivered is being expressed. Preferred marker genes are the *E. Coli* lacZ gene, which encodes β-galactosidase, and green fluorescent protein.

186. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

187. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### C. Kits

188. In some aspects, disclosed herein is a kit comprising the VIP-R antagonist disclosed herein (for example any of the VIP-R antagonists set forth in SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof , as well as, any VIP-R antagonist/VIP antagonist disclosed in U.S. Patent Nos. 6,630,124 and 5,217,953, which are incorporated herein by reference in their entireties). In some embodiments, the kit further comprises an anti-CD3 antibody and/or anti-CD28 antibody. In some embodiments, the kit further comprises a phosphatidylinositol 3-kinase (PI3K) inhibitor (for example, a PI3Kα inhibitor, a PI3Kβ inhibitor, a PI3Kδ inhibitor, or a PI3Kγ inhibitor).

189. Phosphatidylinositol-3-kinase (PI3K)/AKT/mammalian target of rapamycin (mTOR) signaling is one of the most important intracellular pathways, which regulates cell growth, motility, survival, metabolism, and angiogenesis. PI3K is a group of plasma membrane-associated lipid kinases, consisting of three subunits: p85 regulatory subunit, p55 regulatory subunit, and p110 catalytic subunit. PI3K can be divided into 3 classes: classes I, II, and III. Class I PI3Ks comprised of class IA and class IB PI3Ks. Class IA PI3K is a heterodimer of p58 regulatory subunit and p110 catalytic subunit. Class IA PI3K contains p110α, p110β and p110δ catalytic subunits produced from different genes PIK3CA, PIK3CB and PIK3CD, respectively. Subunit p110γ produced by PIK3CG represents the catalytic subunit in class IB PI3K. PI3K inhibitors can inhibit one or more p110 isoforms of the class I PI3Ks. In some embodiments, the PI3K inhibitor described herein is a PI3Kα inhibitor, a PI3Kβ inhibitor, a PI3Kδ inhibitor, or a PI3Kγ inhibitor. In some embodiments, the PI3K inhibitor described herein is a Pan-PI3K inhibitor, an isoform-specific inhibitor, or a dual PI3K inhibitor. Examples of the PI3K inhibitor described herein include, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib). In some embodiments, the PI3K inhibitor described herein is a PI3Kδ inhibitor (e.g., idelalisib).

190. In some embodiments, the kit disclosed herein comprising the VIP-R antagonist disclosed herein (for example, any of the VIP-R antagonists set forth in SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) further comprises an immune checkpoint blockade. In some embodiments, the immune checkpoint blockade is a PD-1 inhibitor, a PD-L1 inhibitor, or a CTLA-4 inhibitor.

191. As used herein, the term "PD-1 inhibitor" refers to a composition that binds to PD-1 and reduces or inhibits the interaction between the bound PD-1 and PD-L1. In some embodiments, the PD-1 inhibitor is a monoclonal antibody that is specific for PD-1 and that reduces or inhibits the interaction between the bound PD-1 and PD-L1. Non-limiting examples of PD-1 inhibitors are pembrolizumab, nivolumab, and cemiplimab. In some embodiments, the pembrolizumab is KEYTRUDA or a bioequivalent. In some embodiments, the pembrolizumab is that described in U.S. Pat. No. 8952136, U.S. Pat. No. 8354509, or U.S. Pat. No. 8900587, all of which are incorporated by reference in their entireties. In some embodiments, the pembrolizumab has the Unique Ingredient Identifier (UNII) of the U.S. Food and Drug Administration of DPT0O3T46P. In some embodiments, the nivolumab is OPDIVO or a bioequivalent. In some embodiments, the nivolumab has the Unique Ingredient Identifier (UNII) of the U.S. Food and Drug Administration of 31YO63LBSN. In some embodiments, the nivolumab is that described in U.S. Pat. No. 7595048, U.S. Pat. No. 8738474, U.S. Pat. No. 9073994, U.S. Pat. No. 9067999, U.S. Pat. No. 8008449, or U.S. Pat. No. 8779105, all of which are incorporated by reference in their entireties. In some embodiments, the cemiplimab is LIBTAYO or a bioequivalent. In some embodiments, the cemiplimab has the Unique Ingredient Identifier (UNII) of the U.S. Food and Drug Administration of 6QVL057INT. In some embodiments, the cemiplimab is that described in U.S. Pat. No. 10844137, which is incorporated by reference in its entirety. In some embodiments, the PD-1 inhibitor described herein is spartalizumab, JTX-4014, camrelizumab, sintilimab, tislelizumab, toripalimab, INCMGA00012 (MGA012), AMP-224, or AMP-514 (MEDI0680).

192. The term "PD-L1 inhibitor" refers to a composition that binds to PDL-1 and reduces or inhibits the interaction between the bound PD-L1 and PD-1. In some embodiments, the PD-L1 inhibitor is a monoclonal antibody that is specific for PD-L1 and that reduces or inhibits the interaction between the bound PD-L1 and PD-1. Non-limiting examples of PD-L1 inhibitors are atezolizumab, avelumab and durvalumab. In some embodiments, the atezolizumab is TECENTRIQ or a bioequivalent. In some embodiments, the atezolizumab has the Unique Ingredient Identifier (UNII) of the U.S. Food and Drug Administration of 52CMI0WC3Y. In some embodiments, the atezolizumab is that described in U.S. Pat. No. 8217149, which is incorporated by reference in its entirety. In some embodiments, the avelumab is BAVENCIO or a bioequivalent. In some embodiments, the avelumab has the Unique Ingredient Identifier (UNII) of the U.S. Food and Drug Administration of KXG2PJ551I. In some embodiments, the avelumab is that described in U.S. Pat. App. Pub. No. 2014321917, which is incorporated by reference in its entirety. In some embodiments, the durvalumab is IMFINZI or a bioequivalent. In some embodiments, the durvalumab has the Unique Ingredient Identifier (UNII) of the U.S. Food and Drug Administration of 28X28X9OKV. In some embodiments, the durvalumab is that described in U.S. Pat. No. 8779108, which is incorporated by reference in its entirety. In some embodiments, the PD-L1 inhibitor described herein is atezolizumab, avelumab, durvalumab, CK-301, or BMS-986189.

193. In some embodiments, the immune checkpoint blockade comprises a PD-1 inhibitor (e.g., pembrolizumab, nivolumab, cemiplimab, dostarlimab, spartalizumab, JTX-4014, camrelizumab, sintilimab, tislelizumab, toripalimab, INCMGA00012 (MGA012), AMP-224, or AMP-514 (MEDI0680)), a PD-L1 inhibitor (e.g., atezolizumab, avelumab, durvalumab, CK-301, or BMS-986189), or a CTLA-4 inhibitor (e.g., ipilimumab or Tremelimumab). In some embodiments, the immune checkpoint blockade comprises pembrolizumab, nivolumab, cemiplimab, dostarlimab, atezolizumab, avelumab, durvalumab, or ipilimumab.

194. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC), pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.

195. In some embodiments, disclosed herein is a kit comprising the VIP-R antagonist disclosed herein (for example any of the VIP-R antagonists set forth in SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof), an immune checkpoint blockade (e.g., pembrolizumab, nivolumab, cemiplimab, dostarlimab, atezolizumab, avelumab, durvalumab, or ipilimumab), and/or a PI3K inhibitor (including, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib)).

### D. Methods of Use

196. In certain embodiments, the disclosure relates to expanding T cells, activating T cells, expanding or reversing senescence in T cells, or reversing exhaustion in T cells with a naturally occurring reactivity to cancer can be found infiltrated in tumors of the subject. The tumor can be harvested, and these tumor-infiltrating lymphocytes (TIL) can be isolated from the tumor and then expanded using methods discloses herein.

197. In certain embodiments, this disclosure relates to compositions and methods of reversing senescence in T cells or reversing exhaustion in T cells by interrupting vasoactive intestinal peptide (VIP) signaling and/or inhibiting phosphatidylinositol-3-kinase (PI3 kinase) inhibitor signaling and uses in managing cancer and chronic viral infections. In certain embodiments, the disclosure contemplates methods of reversing T cell senescence or reversing exhaustion in T cells by mixing T cell *in vitro* with any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, fragment thereof, or an analog thereof) or a nanoparticle comprising any VIP-R antagonist disclosed herein that prevents VIP from interacting with VIP receptors and/or the addition of a PI3 kinase inhibitor. In some embodiments, the method further comprises mixing T cell with an immune checkpoint blockade (for example, a PD-1 inhibitor, a PD-L1 inhibitor, or a CTLA-4 inhibitor). In certain embodiments, the disclosure contemplates the expansion of senescent T cells by mixing with a PI3 kinase inhibitor, a nanoparticle, or a polypeptide disclosed herein, a VIP degrading enzyme, an immune checkpoint blockade, and combinations thereof.

198. In certain embodiments, the disclosure contemplates methods of stimulating isolated T cells or expanding senescent T cells by *in vitro* exposure of T cells to antibodies that bind CD3 and/or CD28 in combination with the PI3 kinase inhibitor (e.g., idelalisib), any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or nanoparticle disclosed herein, a VIP degrading enzyme, and combinations thereof. In certain embodiments, the disclosure contemplates using anti-CD3 and anti-CD28 antibodies or binding agents optionally linked to a solid substrate such as magnetic beads.

199. In certain embodiments, the disclosure contemplates methods of proliferating T cells that are negative for CD28 and/or CD27 using an *in vitro* cell culture as disclosed herein providing replicated T cells that have increased expression of CD28 and/or CD27 compared with levels prior to replication.

200. In certain embodiments, the disclosure contemplates methods of proliferating T cells wherein prior to, during, or after proliferating the T cells, the T cells are mixed with a vector having a nucleic acid sequence encoding a chimeric antigen receptor, wherein the chimeric antigen receptor comprises cancer targeting sequence, a transmembrane domain, a T cell costimulatory molecule domain, and a signal-transduction component of a T-cell antigen receptor domain under conditions such that the cells express a chimeric antigen receptor on the surface of the cells.

201. In certain embodiments, the disclosure relates to *in vitro* cell culture compositions comprising a minimal essential medium and T cells and any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or nanoparticle comprising any of the VIP-R antagonists disclosed herein and a phosphatidylinositol-3-kinase inhibitor, VIP-degrading enzyme, and combinations thereof and optionally further comprising anti-CD3 antibodies and anti-CD28 antibodies optionally immobilized on a solid substrate such as beads. In certain embodiments, the T cells are purified from bone marrow cells or blood cells, peripheral blood.

202. In some embodiments, the PI3K inhibitor described herein is a PI3Kα inhibitor, a PI3Kβ inhibitor, a PI3Kδ inhibitor, or a PI3Kγ inhibitor. In some embodiments, the PI3K inhibitor described herein is a Pan-PI3K inhibitor, an isoform-specific inhibitor, or a dual PI3K inhibitor. Examples of the PI3K inhibitor described herein include, but not limited to, fimepinostat, rigosertib, buparlisib, CH5132799, pilaralisib, ZSTK474, sonolisib, pictilisib, copanlisib, B591, TG-100-115, RIDR-PI-103, dactolisib, apitolisib, gedatolisib, SF1126, omipalisib, samotolisib, bimiralisib, paxalisib, voxtalisib, GSK1059615, MEN1611, ZSTK474, as well as, isoform-specific inhibitiors such as a PI3Kα inhibitor (such as, for example, inavolisib, alpelisib, AZD8835, PWT33597, taselisib, and/or serabelisib), a PI3Kβ inhibitor (such as, for example, AZD8186 and/or GSK2636771), a PI3Kδ inhibitor (such as, for example, AZD8835, AZD8186, nemiralisib, seletalisib, acalisib, CAL263, TG100-115, duvelisib, idelalisib, tenalisib, taselisib, zandelisib, AMG319, linperlisib, parsaclisib, umbralisib, and/or leniolisib), and/or a PI3Kγ inhibitor (such as, for example, eganelisib, tenalisib, taselisib, and/or duvelisib). In certain embodiments, the phosphatidylinositol-3-kinase inhibitor is selected from idelalisib, wortmannin, demethoxyviridin, perifosine, buparlisib, duvelisib, copanlisib, and alpelisib. In certain embodiments, the phosphatidylinositol-3-kinase inhibitor is in a culture at a concentration of greater than about 0.001 nM, 0.1 nM, 1 nM, 10 nM, 100 nM or between about 10 nM and about 10 micromolar or between about 10 nM and about 500 nM, or between about 10nM and about 1 micromolar. In certain embodiments, the phosphatidylinositol-3-kinase inhibitor is selected from idelalisib in a culture at a concentration of greater than about 0.001 nM, 0.1 nM, 1 nM, 10 nM, 100 nM or between about 10 nM and about 10 micromolar or between about 10 nM and about 500 nM, or between about 10 nM and about 1 micromolar.

203. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.

204. In certain embodiments, the culture comprises an enzyme that hydrolyses VIP. In certain embodiments, the culture comprises a VIP degrading enzyme such as a peptidase, serine peptidase, a tryptase, chymase, or human chymase 1 (CMA1). In certain embodiments, the culture has at least at least about 0.001 microgram per mL, about 0.01 microgram per mL, about 0.1 microgram per mL, or about 1 microgram per mL of the VIP degrading enzyme such as a mast cell chymase. In certain embodiments, the disclosure contemplates a T cells culture comprising a minimal essential medium and isolated cells that express CD3 and/or CD4 and/or CD8 and are negative for CD27 and/or CD28 and a PI3 kinase inhibitor, any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or a nanoparticle comprising any of the VIP-R antagonists disclosed herein, and combinations thereof. The cells may be isolated by negative or positive selection using binding agents attached to solid supports such as beads, magnetic beads, or particles of fluorescent binding agents.

205. In certain embodiments, the anti-CD3 antibodies and anti-CD28 antibodies are immobilized on a bead, magnetic bead, or solid surface. In certain embodiments, more than 5.0 % or 10 % or 15 % of the total cells in the culture express CD3 and/or CD4 and/or CD8. In certain embodiments, more than 20 %, 25 % or 50 % of the total cells express CD3 and/or CD4 and/or CD8. In certain embodiments, more than 15 % or 20% or 30% of the T cells in the culture are negative for CD28 and/or CD27. In certain embodiments, more than 20 %, 25 % or 50 % of the T cells are negative for CD28 and/or CD27.

206. In certain embodiments, the purified T cells are obtained from centrifuging blood under conditions such that plasma and red blood cells separate providing purified T cells in a mixture of white blood cells between the plasma and red blood cells. In certain embodiments, the purified T cells are obtained by bone marrow aspirates or a bone marrow biopsy.

207. In certain embodiments, the purified T cells are obtained by mixing cells with a fluorescent marker that binds CD3 and purifying cells by fluorescent activated cell sorting. In certain embodiments, the purified T cells are obtained by mixing cells with a magnetized marker that binds CD3 and purifying cells by magnetic sorting. In certain embodiments, the purified T cells are obtained by mixing cells with a fluorescent marker that binds CD3 and/or CD4 and/or CD8 and purifying cells by fluorescent activated cell sorting. In certain embodiments, the purified T cells are obtained by mixing cells with a magnetized marker that binds CD3 and/or CD4 and/or CD8 and purifying cells by magnetic sorting.

208. In certain embodiments, the disclosure contemplates a solid substrate, such as beads, with anti-CD3 and anti-CD28 antibodies and having a VIP-degrading enzyme coupled to the surface. In certain embodiments, it is contemplated that the beads are arranged in the medium and the T cells are expanded on top of the medium such that the beads are sub-cellular.

209. In certain embodiments, the VIP degrading enzyme comprises human CMA1 Accession number GenBank: AAI03975.1:

MLLKLKEKASLTLAVGTLPFPSQFNFVPPGRMCRVAGWGRTGVLKPGSDTLQEVKLRL
MDPQACSHFRDFDHNLQLCVGNPRKTKSAFKGDSGGPLLCAGVAQGIVSYGRSDAKPP
AVFTRISHYRPWINQILQAN (SEQ ID NO: 19).

210. In certain embodiments, the VIP-degrading enzyme is human recombinant enkephalinase (neutral endopeptidase, EC 3.4.24.11) having the sequence:

DGICKSSDCIKSAARLIQNMDATTEPCTDFFKYACGGWLKRNVIPETSSRYGNFDILRDE
LEVVLKDVLQEPKTEDIVAVQKAKALYRSCINESAIDSRGGEPLLKLLPDIYGWPVATEN

WEQKYGASWTAEKAIAQLNSKYGKKVLINLFVGTDDKNSVNHVIHIDQPRLGLPSRDY
YECTGIYKEACTAYVDFMISVARLIRQEERLPIDENQLALEMNKVMELEKEIANATAKPE
DRNDPMLLYNKMTLAQIQNNFSLEINGKPFSWLNFTNEIMSTVNISITNEEDVVVYAPEY
LTKLKPILTKYSARDLQNLMSWRFIMDLVSSLSRTYKESRNAFRKALYGTTSETATWRR
CANYVNGNMENAVGRLYVEAAFAGESKHVVEDLIAQIREVFIQTLDDLTWMDAETKK
RAEEKALAIKERIGYPDDIVSNDNKLNNEYLELNYKEDEYFENIIQNLKFSQSKQLKKLR
EKVDKDEWISGAAVVNAFYSSGRNQIVFPAGILQPPFFSAQQSNSLNYGGIGMVIGHEIT
HGFDDNGRNFNKDGDLVDWWTQQSASNFKEQSQCMVYQYGNFSWDLAGGQHLNGIN
TLGENIADNGGLGQAYRAYQNYIKKNGEEKLLPGLDLNHKQLFFLNFAQVWCGTYRPE
YAVNSIKTDVESPGNFRIIGTLQNSAEFSEAFHCRKNSYMNPEKKCRVW (SEQ ID NO:
20).

211. In certain embodiments, cell cultures and methods described herein further include IL-12. In certain embodiments, the IL-12 is contemplated to enhance the effect of peptide disclosed herein or nanoparticle thereof on T cell proliferation stimulated *in vitro* with antibodies to CD3 and CD28.

212. In certain embodiments, the disclosure relates to methods of enhancing the immune response to a cell therapy comprising administering any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to a subject in combination with a cell. In certain embodiments, the subject is diagnosed with leukemia or lymphoma. In certain embodiments, the cell is a blood cell, bone marrow cell, leukocyte, T-cell, natural killer cell, a hematopoietic stem cell, a G-CSF mobilized or non-mobilized blood mononuclear cell.

213. In certain embodiments, the cell is selected from the group consisting of autologous T-cells, allogeneic cells from a HLA matched donor, or allogeneic cells from a HLA mis-matched donor. In certain embodiments, the cell is a bone marrow cell. In certain embodiments, the cell is a blood mononuclear cell comprising/expressing granulocyte colony-stimulating factor. The cell therapy may be conducted with non-mobilized blood mononuclear cells.

214. In certain embodiments, it is contemplated that any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) may be administered to subjects before, during, or after a cell-based immunotherapy including the recipient or donor. The immunotherapy may be performed in combinations with chemotherapy and/or a radiation therapy. It is contemplated that peptide may be used in combination with other immune stimulators including, but not limited to, CpG oligonucleotides, granulocyte colony stimulating factor, granulocyte-macrophage colony stimulating factor, interferon alpha, pegylated interferon, interleukin-12, interleukin-2, and pegfilgrastim.

215. In certain embodiments, this disclosure relates to methods of treating or preventing graft versus host disease in a subject comprising administering an effective amount of any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to a subject after a hematopoietic stem cell transplant or a subject that is to receive or received transplanted allogeneic tissue or cells. In certain embodiments, the subject received transplanted allogeneic hematopoietic stem cells. In certain embodiments, the subject received transplanted allogeneic hematopoietic stem cells separated from peripheral blood. In certain embodiments, the subject received chemotherapy to radiation treatments prior to receiving transplanted allogeneic hematopoietic stem cells.

### 1. Methods of Treating Infectious Disease

216. The disclosed VIP-R antagonists can be used in the treatment of infectious disease. In one aspect, disclosed herein are methods of treating, decreasing, inhibiting, reducing, ameliorating, and/or preventing a microbial infection, wherein the microbial infection is a viral infection, and wherein the viral infection is an infection with a virus selected from the group consisting of Herpes Simplex virus- 1, Herpes Simplex virus-2, Varicella-Zoster virus, Epstein-Barr virus, Cytomegalovirus, Human Herpes virus-6, herpes lymphotropic virus, roseolovirus, Kaposi's sarcoma-associated herpesvirus, Variola virus, Vesicular stomatitis virus, Hepatitis A virus, Hepatitis B virus, Hepatitis C virus, Hepatitis D virus, Hepatitis E virus, Rhinovirus, Coronavirus (including, but not limited to avian coronavirus (IBV), porcine coronavirus HKU15 (PorCoV HKU15), Porcine epidemic diarrhea virus (PEDV), HCoV-229E, HCoV-OC43, HCoV-HKU1, HCoV-NL63, SARS-CoV, SARS-CoV-2, or MERS-CoV), Influenza A virus (including, but not limited to H1N1), Influenza B virus, Influenza C virus, Measles virus, Polyomavirus, Human Papillomavirus (HPV) Types 16, 18, 31, 33, 35, 39, 45, 51, 52, 56, 58, 59, parvovirus B19, molluscum contagiosum virus, JC virus (JCV), BK virus, Merkel cell polyomavirus, Respiratory syncytial virus, Adenovirus, Coxsackie virus, Chikungunya virus, Dengue virus, Mumps virus, Poliovirus, Rabies virus, Rous sarcoma virus, Reovirus, Yellow fever virus, human adenovirus types (HAdV-1 to 55), norovirus, rinderpest virus, California encephalitis virus, Friend spleen focus-forming virus (SFFV) or Xenotropic MuLV-Related Virus (XMRV), Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Rotavirus D, Rotavirus E, Sindbis virus, Simian Immunodeficiency virus, Human T-cell Leukemia virus type-1, Hantavirus, Rubella virus, Simian Immunodeficiency virus, Human Immunodeficiency virus type-1, and Human Immunodeficiency virus type-2. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

217. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor. In some embodiments, the immune checkpoint blockade comprises pembrolizumab, nivolumab, cemiplimab, dostarlimab, atezolizumab, avelumab, durvalumab, or ipilimumab.

218. In some embodiments, the disclosure relates to the use of a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) in the production of an anti-viral medicament for the treatment of a viral infection. In some embodiments, the anti-viral medicament further comprises a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the anti-viral medicament further comprises an immune checkpoint blockade (e.g., a PD-1 inhibitor, a PD-L1 inhibitor, or a CTLA-4 inhibitor). In some embodiments, the subject is diagnosed with a chronic viral infection. In certain embodiments, the subject undergoes serological monitoring. In some embodiments, the administration is under conditions such that the viral infection is no longer detected. In some embodiments, the subject is diagnosed with a RNA virus, DNA virus, or retroviruses. In some embodiments, the subject is diagnosed with a virus that is double stranded DNA virus, sense single stranded DNA virus, double stranded RNA virus, sense single stranded RNA virus, antisense single stranded RNA virus, sense single stranded RNA retrovirus or a double stranded DNA retrovirus. In some embodiments, the subject is diagnosed to have a rotavirus, an influenza virus, a herpes virus, a hepatitis virus, or a lentivirus. In some embodiments, titer of the virus in the subject is reduced after the treatment as compared to pretreatment.

219. In certain embodiments, the disclosure relates to methods of treating, decreasing, inhibiting, reducing, ameliorating, and/or preventing a viral infection comprising administering any of the VIP-R antagonists disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16) to a subject at risk of, exhibiting symptoms of, or diagnosed with a viral infection. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor). In certain embodiments, the subject is immune compromised or the subject is an allogeneic bone marrow transplant donor or recipient. In typical embodiments, the subject is an organ transplant recipient, undergoing hemodialysis, diagnosed with cancer, receiving an immunosuppressive drug, and/or diagnosed with an HIV-infection. In certain embodiments, the disclosure relates to preventing a viral infection in an immunocompromised subject at risk of infection by administering any of the VIP-R antagonists disclosed herein and optionally one or more antiviral agents.

220. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor. In some embodiments, the immune checkpoint blockade comprises pembrolizumab, nivolumab, cemiplimab, dostarlimab, atezolizumab, avelumab, durvalumab, or ipilimumab.

221. The disclosed VIP-R antagonists (such as, for example, SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16) are not limited to the treatment of viral infections, but are also useful in the treatment of other microbial infections, including, but not limited to bacterial, fungal, and parasitic infections. Accordingly, also disclosed herein are methods of treating, decreasing, inhibiting, reducing, ameliorating, and/or preventing a microbial infection, wherein the microbial infection is a bacterial infection, and wherein the bacterial infection is an infection with a bacteria selected from the group consisting of Mycobacterium tuberculosis, Mycobacterium bovis, Mycobacterium bovis strain BCG, BCG substrains, Mycobacterium avium, Mycobacterium intracellular, Mycobacterium africanum, Mycobacterium kansasii, Mycobacterium marinum, Mycobacterium ulcerans, Mycobacterium avium subspecies paratuberculosis, Nocardia asteroides, other Nocardia species, Legionella pneumophila, other Legionella species, Acetinobacter baumanii, Salmonella typhi, Salmonella enterica, other Salmonella species, Shigella boydii, Shigella dysenteriae, Shigella sonnei, Shigella flexneri, other Shigella species, Yersinia pestis, Pasteurella haemolytica, Pasteurella multocida, other Pasteurella species, Actinobacillus pleuropneumoniae, Listeria monocytogenes, Listeria ivanovii, Brucella abortus, other Brucella species, Cowdria ruminantium, Borrelia burgdorferi, Bordetella avium, Bordetella pertussis, Bordetella bronchiseptica, Bordetella trematum, Bordetella hinzii, Bordetella pteri, Bordetella parapertussis, Bordetella ansorpii other Bordetella species, Burkholderia mallei, Burkholderia psuedomallei, Burkholderia cepacian, Chlamydia pneumoniae, Chlamydia trachomatis, Chlamydia psittaci, Coxiella burnetii, Rickettsial species, Ehrlichia species, Staphylococcus aureus, Staphylococcus epidermidis, Streptococcus pneumoniae, Streptococcus pyogenes, Streptococcus agalactiae, Escherichia coli, Vibrio cholerae, Campylobacter species, Neiserria meningitidis, Neiserria gonorrhea, Pseudomonas aeruginosa, other Pseudomonas species, Haemophilus influenzae, Haemophilus ducreyi, other Hemophilus species, Clostridium tetani, other Clostridium species, Yersinia enterolitica, and other Yersinia species.

222. In one aspect, disclosed herein are methods of treating, decreasing, inhibiting, reducing, ameliorating, and/or preventing a microbial infection, wherein the microbial infection is a fungal infection, and wherein the fungal infection is an infection with a fungus selected from the group consisting of Candida albicans, Cryptococcus neoformans, Histoplasma capsulatum, Aspergillus fumigatus, Coccidiodes immitis, Paracoccidiodes brasiliensis, Blastomyces dermitidis, Pneumocystis carnii, Penicillium marneffi, and Alternaria alternata.

217. Also disclosed herein are methods of treating, decreasing, inhibiting, reducing, ameliorating, and/or preventing a microbial infection, wherein the microbial infection is a parasitic infection, and wherein the parasitic infection is an infection with a parasite selected from the group consisting of Toxoplasma gondii, Plasmodium falciparum, Plasmodium vivax, Plasmodium malariae, other Plasmodium species, Entamoeba histolytica, Naegleria fowleri, Rhinosporidium seeberi, Giardia lamblia, Enterobius vermicularis, Enterobius gregorii, Ascaris lumbricoides, Ancylostoma duodenale, Necator americanus, Cryptosporidium spp., Trypanosoma brucei, Trypanosoma cruzi, Leishmania major, other Leishmania species, Diphyllobothrium latum, Hymenolepis nana, Hymenolepis diminuta, Echinococcus granulosus, Echinococcus multilocularis, Echinococcus vogeli, Echinococcus oligarthrus, Diphyllobothrium latum, Clonorchis sinensis; Clonorchis viverrini, Fasciola hepatica, Fasciola gigantica, Dicrocoelium dendriticum, Fasciolopsis buski, Metagonimus yokogawai, Opisthorchis viverrini, Opisthorchis felineus, Clonorchis sinensis, Trichomonas vaginalis, Acanthamoeba species, Schistosoma intercalatum, Schistosoma haematobium, Schistosoma japonicum, Schistosoma mansoni, other Schistosoma species, Trichobilharzia regenti, Trichinella spiralis, Trichinella britovi, Trichinella nelsoni, Trichinella nativa, and Entamoeba histolytica.It is understood and herein contemplated that despite the ability of the disclosed VIP-R antagonists (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to inhibit microbial virulence and effectuate microbial clearance in tissue without the addition of an anti-microbial agent, there can be instances where the addition (either in the composition itself or as a separate administration) of an anti-microbial is desired. Accordingly, disclosed herein are methods of treating, decreasing, inhibiting, reducing, ameliorating, and/or preventing a microbial infection, autoimmune disease, auto inflammatory disease, or cancer, further comprising administering to the subject an anti-microbial agent. Anti-microbial agents can comprise any antibiotics, antibodies, small molecules, and functional nucleic acids (siRNA, RNAi, anti-sense oligonucleotides), that directly attack the infecting microbe or alter host conditions rendering the host system inhospitable to the microbe. Such agents include, but are not limited to Abacavir, Acyclovir, Adefovir, Amantadine, Amprenavir, Ampligen, Arbidol, Atazanavir, Atripla, Balavir, Beta-D-N4-hydroxycitidine (NHC, EIDD-1931), Cidofovir, Combivir, Dolutegravir, Darunavir, Delavirdine, Didanosine, Docosanol, Edoxudine, Efavirenz, Emtricitabine, Enfuvirtide, Entecavir, Ecoliever, Famciclovir, Fomivirsen, Fosamprenavir, Foscarnet, Fosfonet, Ganciclovir, Hydroxy-chloroquine, Ibacitabine, Imunovir, Idoxuridine, Imiquimod, Indinavir, Inosine, Lamivudine, Lopinavir, Loviride, Maraviroc, Moroxydine, Methisazone, Nelfinavir, Nevirapine, Nexavir, Nitazoxanide, Norvir, Oseltamivir, Peginterferon alfa-2a, Penciclovir, Peramivir, Pleconaril, Podophyllotoxin, Raltegravir, Remdecivir, Ribavirin, Rimantadine, Ritonavir, Pyramidine, Saquinavir, Sofosbuvir, Stavudine, Telaprevir, Tenofovir, Tenofovir disoproxil, Tipranavir, Trifluridine, Trizivir, Tromantadine, Truvada, Valaciclovir, Valganciclovir, Vicriviroc, Vidarabine, Viramidine, Zalcitabine, Zanamivir, Zidovudine, Clofazimine; Dapsone; Capreomycin; Cycloserine; Ethambutol(Bs); Ethionamide; Isoniazid; Pyrazinamide; Rifampicin; Rifabutin; Rifapentine; Streptomycin; Arsphenamine; Chloramphenicol(Bs); Fosfomycin; Fusidic acid; Metronidazole; Mupirocin; Platensimycin; Quinupristin/Dalfopristin; Thiamphenicol; Tigecycline(Bs); Tinidazole; Trimethoprim(Bs); aminoglycosides such as, for example, Amikacin, Gentamicin, Kanamycin, Meropenem, Neomycin, Netilmicin, Tobramycin, Paromomycin, Streptomycin, Spectinomycin, Nitazoxanide, Melarsoprol Eflornithine, Metronidazole, Tinidazole, Miltefosine, Mebendazole, Pyrantel pamoate , Thiabendazole, Diethylcarbamazine, Ivermectin, Niclosamide, Praziquantel, Albendazole, Praziquantel, Rifampin, Amphotericin B, Fumagillin, Amphotericin B, Candicidin, Filipin, Hamycin, Natamycin, Nystatin, Rimocidin, Bifonazole, Butoconazole, Clotrimazole, Econazole, Fenticonazole, Isoconazole, Ketoconazole, Luliconazole, Miconazole, Omoconazole, Oxiconazole, Sertaconazole, Sulconazole, Tioconazole, Albaconazole, Efinaconazole, Epoxiconazole, Fluconazole, Isavuconazole, Itraconazole, Posaconazole, Propiconazole, Ravuconazole, Terconazole, Voriconazole, Abafungin, Anidulafungin, Caspofungin, Micafungin, Aurones, Benzoic acid, Ciclopirox, Flucytosine, Griseofulvin, Haloprogin, Tolnaftate, Undecylenic acid, Crystal violet, Balsam of Peru, Orotomide, Miltefosine, ; ansamycins, such as, for example, geldanamycin, rifaximin, herbimycin; Carbapenems, such as, for example, Ertapenem, Doripenem, Imipenem/Cilastatin, and Meropenem; Cephalosporins, such as, for example, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefixime, Cefdinir, Cefditoren, Cefoperazone, Cefotaxime, Cefpodoxime, Ceftazidime, Ceftibuten, Ceftizoxime, Moxalactam, Ceftriaxone, Cefepime, Ceftaroline fosamil, and Ceftobiprole; Glycopeptides, such as, for example Teicoplanin, Vancomycin, Telavancin, Dalbavancin, and Oritavancin; Lincosamides(Bs), such as, for example, Clindamycin and Lincomycin; Lipopeptides, such as, for example, Daptomycin; Macrolides(Bs), such as, for example, Azithromycin, Clarithromycin, Erythromycin, Roxithromycin, Telithromycin, and Spiramycin; Monobactams, such as, for example, Aztreonam; Nitrofurans, such as, for example, Furazolidone and Nitrofurantoin(Bs); Oxazolidinones(Bs), such as, for example, Linezolid, Posizolid, Radezolid, and Torezolid; Penicillins, such as, for example, Amoxicillin, Ampicillin, Azlocillin, Dicloxacillin, Flucloxacillin, Mezlocillin, Methicillin, Nafcillin, Oxacillin, Penicillin G, Penicillin V, Piperacillin, Penicillin G, Temocillin, and Ticarcillin; Polypeptides, such as, for example, Bacitracin, Colistin, and Polymyxin B; Quinolones/Fluoroquinolones, such as, for example, Ciprofloxacin, Enoxacin, Gatifloxacin, Gemifloxacin, Levofloxacin, Lomefloxacin, Moxifloxacin, Nadifloxacin, Nalidixic acid, Norfloxacin, Ofloxacin, Trovafloxacin, Grepafloxacin, Sparfloxacin, and Temafloxacin; Sulfonamides(Bs), such as, for example, Mafenide, Sulfacetamide, Sulfadiazine, Silver sulfadiazine, Sulfadimethoxine, Sulfamethizole, Sulfamethoxazole, Sulfanilamide (archaic), Sulfasalazine, Sulfisoxazole, Trimethoprim-Sulfamethoxazole (Co-trimoxazole) (TMP-SMX), and Sulfonamidochrysoidine (archaic); Tetracyclines(Bs), such as, for example, Demeclocycline, Doxycycline, Metacycline, Minocycline, Oxytetracycline, and Tetracycline; monoclonal antibodies such as, for example, Actoxumab, Atidortoxumab, Berlimatoxumab,' Bezlotoxumab, Cosfroviximab, Edobacomab, Felvizumab, Firivumab, Foravirumab, Larcaviximab, Motavizumab, Navivumab, Panobacumab, Palivizumab, Porgaviximab, CR6261, Rafivirumab, Pagibaximab, Obiltoxaximab, Ibalizumab, Regavirumab, Rmab, Sevirumab, Rivabazumab pegol, Tefibazumab, Suvratoxumab, and Tuvirumab; and checkpoint inhibitors; Pembrolizumab, Nivolumab, Atezolizumab, Avelumab, Durvalumab, pidilizumab, AMP-224, AMP-514, PDR001, cemiplimab, and Ipilimumab. In certain embodiments, the subject is administered a pharmaceutical composition comprising a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) and a second antiviral agent.

218. In certain embodiments, the disclosure relates to treating a subject with a viral infection after infection by administering a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) and an immunoglobulin.

219. In certain embodiments, the disclosure relates to treating or preventing a viral infection by administering a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) and a viral vaccine or in the absence of a viral vaccine.

220. In certain embodiments, the disclosure relates to enhancing the immune response to a vaccine comprising administering a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to a subject in need thereof. Typically, the vaccine is selected from the group of vaccines consisting of herpes zoster vaccine, smallpox vaccine, polio vaccine, pertussis vaccine, influenza vaccine, diphtheria vaccine, tetanus vaccine, meningococcal vaccine, influenza A vaccine including subtype H1N1 vaccine, influenza B vaccine, influenza C vaccine, rotavirus A vaccine, rotavirus B vaccine, rotavirus C vaccine, rotavirus D vaccine, rotavirus E vaccine, SARS coronavirus vaccine, human adenovirus types (HAdV-1 to 55) vaccine, human papillomavirus (HPV) vaccine, parvovirus B19 vaccine, molluscum contagiosum vaccine, JC vaccine, BK vaccine, Merkel cell polyomavirus vaccine, coxsackie A vaccine, norovirus vaccine, Rubella vaccine, lymphocytic choriomeningitis vaccine, yellow fever vaccine, measles vaccine, mumps vaccine, respiratory syncytial vaccine, rinderpest vaccine, California encephalitis vaccine, hantavirus vaccine, rabies vaccine, Ebola vaccine, marburg vaccine, herpes simplex virus-1 (HSV-1) vaccine, herpes simplex virus-2 (HSV-2) vaccine, varicella zoster vaccine, Epstein-Barr virus (EBV) vaccine, cytomegalovirus (CMV) vaccine, herpes lymphotropic vaccine, roseolovirus vaccine, Kaposi's sarcoma-associated herpesvirus vaccine, hepatitis A (HAV) vaccine, hepatitis B (HBV) vaccine, hepatitis C (HCV) vaccine, hepatitis D (HDV) vaccine, hepatitis E (HEV) vaccine, human immunodeficiency virus (HIV) vaccine, The Human T-lymphotropic virus Type I (HTLV-1) vaccine, Friend spleen focus-forming virus (SFFV) vaccine, and Xenotropic MuLV-Related Virus (XMRV) vaccine. In certain embodiments, the vaccine for a subject diagnosed with a chronic viral infection.

221. In certain embodiments, the vaccine comprises a protein or peptide, carbohydrate, sugar, polysaccharide, or nucleic acid. Typically, the vaccine is an attenuated replication competent virus or an inactivated virus. In certain embodiments, the vaccine comprises a live or a killed or inactivated prokaryotic or eukaryotic cell.

222. In certain embodiments, the human T cells are activated *in vitro* by co-incubation with viral antigens. In certain embodiments, the viral antigens are presented on microvesicles. In certain embodiments, the viral antigens are presented on dendritic cells.

223. Nucleic acid vaccines, typically a DNA plasmid or RNA vaccine, are genetically engineered to encode and/or produce one or more antigens from a pathogen. The nucleic acid transfects or infects host cells, where the inner machinery of the cells expresses the proteins. Because these proteins are recognized as foreign, when they are processed by the host cells and displayed on their surface immune response is triggered. Cytotoxic T lymphocytes responses can also be enhanced by co-inoculation with co-stimulatory molecules such as GM-CSF, B7-1, or B7-2. In certain embodiments, a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) may be administered in combination with nucleic acid vaccines or other co-stimulatory molecules.

224. In certain embodiments, the disclosure relates to vaccine compositions comprising a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) and methods of administering a VIP-R antagonist disclosed herein in combination with a vaccine. In certain embodiments, the vaccine contains an antigen from a pathogen and is presented to the immune system from weakened or killed forms of the microbe or its toxins. The antigen stimulates the immune system. Vaccines may be prophylactic (e.g. to prevent or ameliorate the effects of a future infection by any pathogen), or therapeutic by being administered after infection or diagnosis of the disease.

225. Some vaccines contain killed, but previously virulent, microorganisms that have been destroyed with chemicals or heat. The influenza vaccine, cholera vaccine, bubonic plague vaccine, polio vaccine, hepatitis A vaccine, and rabies vaccine are examples of a killed vaccine that are contemplated by this disclosure.

226. Some vaccines contain live, attenuated microorganisms. Typically, these are live viruses that have been cultivated under conditions that disable certain virulent properties, or which use closely related but less dangerous organisms to produce a broad immune response; however, some are bacterial in nature.

227. In certain embodiments, the vaccine is a protein subunit. Rather than introducing an inactivated or attenuated microorganism to an immune system, a fragment of it can be used to create an immune response. Examples include the subunit vaccine against Hepatitis B virus that is composed of only the surface proteins of the virus, the virus-like particle (VLP) vaccine against human papillomavirus (HPV) that is composed of the viral major capsid protein, and the hemagglutinin and neuraminidase subunits of the influenza virus.

228. In certain embodiments, the vaccine comprises a polysaccharide. Certain bacteria have polysaccharide outer coats that are typically immunogenic. By linking these polysaccharides to proteins (e.g. toxins), the immune system can be led to recognize the polysaccharide as if it were a protein antigen.

229. Toxoid vaccines are made from inactivated toxic compounds. Examples of toxoid-based vaccines include diphtheria and tetanus toxoid. In certain embodiments, a VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) is administered in combination with DPT. DPT (also DTP and DTwP) refers to a class of combination vaccines against three infectious diseases in humans: diphtheria, pertussis (whooping cough) and tetanus. The vaccine components include diphtheria, tetanus toxoids, and killed whole cells of the organism that causes pertussis (wP). DTaP (also known as Tdap, DTPa, and TDaP) refers to similar combination vaccines in which the pertussis component is acellular. Also contemplated is the DT or TD vaccine, which lacks the pertussis component.

230. Other specific vaccines contemplated by the disclosure include the anthrax vaccine, e.g., culture filtrates of an avirulent, nonencapsulated strain known as V770-NP1-R, Bacille Calmette-Guérin (BCG), e.g., a strain of the attenuated live bovine tuberculosis bacillus, haemophilus influenzae type B vaccine, e.g., Hib polysaccharide-protein conjugate vaccine, hepatitis A vaccine, e.g., inactivated Hepatitis A virus, hepatitis B vaccine, e.g., hepatitis B surface antigen, human papillomavirus (HPV) vaccine, e.g., non-infectious virus-like particles assembled from the L1 proteins of HPV types 6, 11, 16 and 18, meningococcal vaccine, e.g., capsular polysaccharide antigens of Neisseria meningitides serogroups A, C, Y, and W-135 strains individually conjugated to diphtheria toxoid protein.

231. In certain embodiments, this disclosure relates to methods of treating an active cytomegalovirus infection comprising administering an effective amount of a vasoactive intestinal peptide antagonist disclosed herein to a subject diagnosed with and exhibiting signs or symptoms of an active cytomegalovirus infection, wherein the vasoactive intestinal peptide antagonist comprises a peptide having a C-terminal amide and is optionally modified with hydrocarbon or polyethylene glycol groups. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

232. In certain embodiments, this disclosure relates to methods of reducing an active cytomegalovirus infection comprising administering an effective amount of a vasoactive intestinal peptide antagonist disclosed herein to a subject suffering from an active cytomegalovirus infection, wherein the vasoactive intestinal peptide antagonist comprises a peptide having a C-terminal amide and is optionally modified with hydrocarbon or polyethylene glycol groups. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor). In certain embodiments, a titer of cytomegalovirus in the subject is reduced after administering the vasoactive intestinal peptide antagonist as compared to pretreatment.

233. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor. In some embodiments, the immune checkpoint blockade comprises pembrolizumab, nivolumab, cemiplimab, dostarlimab, atezolizumab, avelumab, durvalumab, or ipilimumab.

### 2. Method of treating cancer

234. In certain embodiments, it is contemplated that any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) is used in certain cellular immunotherapies that are effective for treating cancer such as lymphocyte infusions or allogeneic bone marrow transplantations. Donor immune cells, particularly NK cells and T-cells, cells have anti-cancer cytotoxic activity. VIP antagonism of the peptide enhances cellular immune responses in vivo. VIP antagonism increases the cytotoxic activity of antigen-specific T-cells and NK cells. VIP antagonism is predicted to increase the anti-cancer activity of NK cells or antigen-specific T-cells. VIP antagonism in conjunction with cellular immunotherapy is predicted to increase the efficacy of said therapy. It is believed that the absence of VIP does not increase the "off-target" graft versus host disease activity of donor lymphocytes in recipients of allogeneic bone marrow transplantation. Thus, administration of VIP-R antagonists to subjects with cancer receiving cellular therapies, e.g., donor lymphocyte infusions or allogeneic bone marrow transplantation, will increase the anti-cancer activity of said therapy. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

235. In some embodiments, the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor. In some embodiments, the immune checkpoint blockade comprises pembrolizumab, nivolumab, cemiplimab, dostarlimab, atezolizumab, avelumab, durvalumab, or ipilimumab.

236. "Cancer" refers any of various cellular diseases with malignant neoplasms characterized by the proliferation of cells. It is not intended that the diseased cells must actually invade surrounding tissue and metastasize to new body sites. Cancer can involve any tissue of the body and have many different forms in each body area. Within the context of certain embodiments, whether "cancer is reduced" may be identified by a variety of diagnostic manners known to one skill in the art including, but not limited to, observation the reduction in size or number of tumor masses or if an increase of apoptosis of cancer cells observed, e.g., if more than a 5 % increase in apoptosis of cancer cells is observed for a sample compound compared to a control without the compound. It may also be identified by a change in relevant biomarker or gene expression profile, such as PSA for prostate cancer, HER2 for breast cancer, serum levels of VIP in patients with pancreatic cancer, or others.

237. The disclosed compositions can be used to treat, inhibit, decrease, reduce, ameliorate, and/or prevent any disease where uncontrolled cellular proliferation occurs such as cancers. A representative but non-limiting list of cancers that the disclosed compositions can be used to treat is the following: malignancies located in the colon, abdomen, bone, breast, digestive system, liver, pancreas, peritoneum, endocrine glands (adrenal, parathyroid, hypophysis, testicles, ovaries, thymus, thyroid), eye, head and neck, nervous system (central and peripheral), lymphatic system, pelvis, skin, soft tissue, spleen, thorax and genitourinary apparatus and, more particularly, childhood acute lymphoblastic leukemia, acute lymphoblastic leukemia, acute lymphocytic leukemia, acute myeloid leukemia, adrenocortical carcinoma, adult (primary) hepatocellular cancer, adult (primary) liver cancer, adult acute lymphocytic leukemia, adult acute myeloid leukemia, adult Hodgkin's disease, adult Hodgkin's lymphoma, adult lymphocytic leukemia, adult non-Hodgkin's lymphoma, adult primary liver cancer, adult soft tissue sarcoma, AIDS-related lymphoma, AIDS-related malignant tumors, anal cancer, astrocytoma, cancer of the biliary tract, cancer of the bladder, bone cancer, brain stem glioma, brain tumors, breast cancer, cancer of the renal pelvis and ureter, primary central nervous system lymphoma, central nervous system lymphoma, cerebellar astrocytoma, brain astrocytoma, cancer of the cervix, childhood (primary) hepatocellular cancer, childhood (primary) liver cancer, childhood acute lymphoblastic leukemia, childhood acute myeloid leukemia, childhood brain stem glioma, childhood cerebellar astrocytoma, childhood brain astrocytoma, childhood extracranial germ cell tumors, childhood Hodgkin's disease, childhood Hodgkin's lymphoma, childhood visual pathway and hypothalamic glioma, childhood lymphoblastic leukemia, childhood medulloblastoma, childhood non-Hodgkin's lymphoma, childhood supratentorial primitive neuroectodermal and pineal tumors, childhood primary liver cancer, childhood rhabdomyosarcoma, childhood soft tissue sarcoma, childhood visual pathway and hypothalamic glioma, chronic lymphocytic leukemia, chronic myeloid leukemia, cancer of the colon, cutaneous T-cell lymphoma, endocrine pancreatic islet cells carcinoma, endometrial cancer, ependymoma, epithelial cancer, cancer of the esophagus, Ewing's sarcoma and related tumors, cancer of the exocrine pancreas, extracranial germ cell tumor, extragonadal germ cell tumor, extrahepatic biliary tract cancer, cancer of the eye, breast cancer in women, Gaucher's disease, cancer of the gallbladder, gastric cancer, gastrointestinal carcinoid tumor, gastrointestinal tumors, germ cell tumors, gestational trophoblastic tumor, head and neck cancer, hepatocellular cancer, Hodgkin's disease, Hodgkin's lymphoma, hypergammaglobulinemia, hypopharyngeal cancer, intestinal cancers, intraocular melanoma, islet cell carcinoma, islet cell pancreatic cancer, Kaposi's sarcoma, cancer of kidney, cancer of the larynx, cancer of the lip and mouth, cancer of the liver, cancer of the lung, lymphoproliferative disorders, macroglobulinemia, breast cancer in men, malignant mesothelioma, malignant thymoma, medulloblastoma, melanoma, mesothelioma, Merkel cell carcinoma, occult primary metastatic squamous neck cancer, primary metastatic squamous neck cancer, metastatic squamous neck cancer, multiple myeloma, multiple myeloma/plasmatic cell neoplasia, myelodysplastic syndrome, myelogenous leukemia, myeloid leukemia, myeloproliferative disorders, paranasal sinus and nasal cavity cancer, nasopharyngeal cancer, neuroblastoma, non-Hodgkin's lymphoma during pregnancy, non-melanoma skin cancer, non-small cell lung cancer, metastatic squamous neck cancer with occult primary, buccopharyngeal cancer, malignant fibrous histiocytoma, malignant fibrous osteosarcoma/histiocytoma of the bone, epithelial ovarian cancer, ovarian germ cell tumor, ovarian low malignant potential tumor, pancreatic cancer, paraproteinemias, purpura, parathyroid cancer, cancer of the penis, pheochromocytoma, hypophysis tumor, neoplasia of plasmatic cells/multiple myeloma, primary central nervous system lymphoma, primary liver cancer, prostate cancer, rectal cancer, renal cell cancer, cancer of the renal pelvis and ureter, retinoblastoma, rhabdomyosarcoma, cancer of the salivary glands, sarcoidosis, sarcomas, skin cancer, small cell lung cancer, small intestine cancer, soft tissue sarcoma, squamous neck cancer, stomach cancer, pineal and supratentorial primitive neuroectodermal tumors, T-cell lymphoma, testicular cancer, thymoma, thyroid cancer, transitional cell cancer of the renal pelvis and ureter, transitional renal pelvis and ureter cancer, trophoblastic tumors, cell cancer of the renal pelvis and ureter, cancer of the urethra, cancer of the uterus, uterine sarcoma, vaginal cancer, optic pathway and hypothalamic glioma, cancer of the vulva, Waldenstrom's macroglobulinemia, Wilms' tumor and any other hyperproliferative disease, as well as neoplasia, located in the system of a previously mentioned organ.

238. In one aspect, it is understood the treatment of cancer does not need to be limited to the administration of VIP-R antagonists but can include the further administration of anti-cancer agents to treat, inhibit, reduce, decrease, ameliorate, and/or prevent a cancer or metastasis. Anti-cancer therapeutic agents (such as checkpoint inhibitors, chemotherapeutics, immunotoxins, peptides, and antibodies) that can be used in the methods of treating, inhibiting, reducing, decreasing, ameliorating, and/or preventing a cancer and/or metastasis and in combination with any of the disclosed VIP-R antagonists can comprise any anti-cancer therapeutic agent known in the art, the including, but not limited to Abemaciclib, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Afatinib Dimaleate, Afinitor (Everolimus), Akynzeo (Netupitant and Palonosetron Hydrochloride), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Aloxi (Palonosetron Hydrochloride), Alunbrig (Brigatinib), Ambochlorin (Chlorambucil), Amboclorin Chlorambucil), Amifostine, Aminolevulinic Acid, Anastrozole, Aprepitant, Aredia (Pamidronate Disodium), Arimidex (Anastrozole), Aromasin (Exemestane),Arranon (Nelarabine), Arsenic Trioxide, Arzerra (Ofatumumab), Asparaginase Erwinia chrysanthemi, Atezolizumab, Avastin (Bevacizumab), Avelumab, Axitinib, Azacitidine, Bavencio (Avelumab), BEACOPP, Becenum (Carmustine), Beleodaq (Belinostat), Belinostat, Bendamustine Hydrochloride, BEP, Besponsa (Inotuzumab Ozogamicin), Bevacizumab, Bexarotene, Bexxar (Tositumomab and Iodine I 131 Tositumomab), Bicalutamide, BiCNU (Carmustine), Bleomycin, Blinatumomab, Blincyto (Blinatumomab), Bortezomib, Bosulif (Bosutinib), Bosutinib, Brentuximab Vedotin, Brigatinib, BuMel, Busulfan, Busulfex (Busulfan), Cabazitaxel, Cabometyx (Cabozantinib-S-Malate), Cabozantinib-S-Malate, CAF, Campath (Alemtuzumab), Camptosar , (Irinotecan Hydrochloride), Capecitabine, CAPOX, Carac (Fluorouracil--Topical), Carboplatin, CARBOPLATIN-TAXOL, Carfilzomib, Carmubris (Carmustine), Carmustine, Carmustine Implant, Casodex (Bicalutamide), CEM, Ceritinib, Cerubidine (Daunorubicin Hydrochloride), Cervarix (Recombinant HPV Bivalent Vaccine), Cetuximab, CEV, Chlorambucil, CHLORAMBUCIL-PREDNISONE, CHOP, Cisplatin, Cladribine, Clafen (Cyclophosphamide), Clofarabine, Clofarex (Clofarabine), Clolar (Clofarabine), CMF, Cobimetinib, Cometriq (Cabozantinib-S-Malate), Copanlisib Hydrochloride, COPDAC, COPP, COPP-ABV, Cosmegen (Dactinomycin), Cotellic (Cobimetinib), Crizotinib, CVP, Cyclophosphamide, Cyfos (Ifosfamide), Cyramza (Ramucirumab), Cytarabine, Cytarabine Liposome, Cytosar-U (Cytarabine), Cytoxan (Cyclophosphamide), Dabrafenib, Dacarbazine, Dacogen (Decitabine), Dactinomycin, Daratumumab, Darzalex (Daratumumab), Dasatinib, Daunorubicin Hydrochloride, Daunorubicin Hydrochloride and Cytarabine Liposome, Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Durvalumab, Duvelisib, Efudex (Fluorouracil--Topical), Elitek (Rasburicase), Ellence (Epirubicin Hydrochloride), Elotuzumab, Eloxatin (Oxaliplatin), Eltrombopag Olamine, Emend (Aprepitant), Empliciti (Elotuzumab), Enasidenib Mesylate, Enzalutamide, Epirubicin Hydrochloride, EPOCH, Erbitux (Cetuximab), Eribulin Mesylate, Erivedge (Vismodegib), Erlotinib Hydrochloride, Erwinaze (Asparaginase Erwinia chrysanthemi), Ethyol (Amifostine), Etopophos (Etoposide Phosphate), Etoposide, Etoposide Phosphate, Evacet (Doxorubicin Hydrochloride Liposome), Everolimus, Evista, (Raloxifene Hydrochloride), Evomela (Melphalan Hydrochloride), Exemestane, 5-FU (Fluorouracil Injection), 5-FU (Fluorouracil--Topical), Fareston (Toremifene), Farydak (Panobinostat), Faslodex (Fulvestrant), FEC, Femara (Letrozole), Filgrastim, Fludara (Fludarabine Phosphate), Fludarabine Phosphate, Fluoroplex (Fluorouracil--Topical), Fluorouracil Injection, Fluorouracil--Topical, Flutamide, Folex (Methotrexate), Folex PFS (Methotrexate), FOLFIRI, FOLFIRI-BEVACIZUMAB, FOLFIRI-CETUXIMAB, FOLFIRINOX, FOLFOX, Folotyn (Pralatrexate), FU-LV, Fulvestrant, Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, Gemcitabine-cisplatin, Gemcitabine-oxaliplatin, Gemtuzumab Ozogamicin, Gemzar (Gemcitabine Hydrochloride), Gilotrif (Afatinib Dimaleate), Gleevec (Imatinib Mesylate), Gliadel (Carmustine Implant), Gliadel wafer (Carmustine Implant), Glucarpidase, Goserelin Acetate, Halaven (Eribulin Mesylate), Hemangeol (Propranolol Hydrochloride), Herceptin (Trastuzumab), HPV Bivalent Vaccine, Recombinant, HPV Nonavalent Vaccine, Recombinant, HPV Quadrivalent Vaccine, Recombinant, Hycamtin (Topotecan Hydrochloride), Hydrea (Hydroxyurea), Hydroxyurea, Hyper-CVAD, Ibrance (Palbociclib), Ibritumomab Tiuxetan, Ibrutinib, ICE, Iclusig (Ponatinib Hydrochloride), Idamycin (Idarubicin Hydrochloride), Idarubicin Hydrochloride, Idelalisib, Idhifa (Enasidenib Mesylate), Ifex (Ifosfamide), Ifosfamide, Ifosfamidum (Ifosfamide), IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Recombinant, Interleukin-2 (Aldesleukin), Intron A (Recombinant Interferon Alfa-2b), Iodine I 131 Tositumomab and Tositumomab, Ipilimumab, Iressa (Gefitinib), Irinotecan Hydrochloride, Irinotecan Hydrochloride Liposome, Istodax (Romidepsin), Ixabepilone, Ixazomib Citrate, Ixempra (Ixabepilone), Jakafi (Ruxolitinib Phosphate), JEB, Jevtana (Cabazitaxel), Kadcyla (Ado-Trastuzumab Emtansine), Keoxifene (Raloxifene Hydrochloride), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Leustatin (Cladribine), Levulan (Aminolevulinic Acid), Linfolizin (Chlorambucil), LipoDox (Doxorubicin Hydrochloride Liposome), Lomustine, Lonsurf (Trifluridine and Tipiracil Hydrochloride), Lupron (Leuprolide Acetate), Lupron Depot (Leuprolide Acetate), Lupron Depot-Ped (Leuprolide Acetate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Melphalan, Melphalan Hydrochloride, Mercaptopurine, Mesna, Mesnex (Mesna), Methazolastone (Temozolomide), Methotrexate, Methotrexate LPF (Methotrexate), Methylnaltrexone Bromide, Mexate (Methotrexate), Mexate-AQ (Methotrexate), Midostaurin, Mitomycin C, Mitoxantrone Hydrochloride, Mitozytrex (Mitomycin C), MOPP, Mozobil (Plerixafor), Mustargen (Mechlorethamine Hydrochloride) , Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neosar (Cyclophosphamide), Neratinib Maleate, Nerlynx (Neratinib Maleate), Netupitant and Palonosetron Hydrochloride, Neulasta (Pegfilgrastim), Neupogen (Filgrastim), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nolvadex (Tamoxifen Citrate), Nplate (Romiplostim), Obinutuzumab, Odomzo (Sonidegib), OEPA, Ofatumumab, OFF, Olaparib, Olaratumab, Omacetaxine Mepesuccinate, Oncaspar (Pegaspargase), Ondansetron Hydrochloride, Onivyde (Irinotecan Hydrochloride Liposome), Ontak (Denileukin Diftitox), Opdivo (Nivolumab), OPPA, Osimertinib, Oxaliplatin, Paclitaxel, Paclitaxel Albumin-stabilized Nanoparticle Formulation, PAD, Palbociclib, Palifermin, Palonosetron Hydrochloride, Palonosetron Hydrochloride and Netupitant, Pamidronate Disodium, Panitumumab, Panobinostat, Paraplat (Carboplatin), Paraplatin (Carboplatin), Pazopanib Hydrochloride, PCV, PEB, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Portrazza (Necitumumab), Pralatrexate, Prednisone, Procarbazine Hydrochloride , Proleukin (Aldesleukin), Prolia (Denosumab), Promacta (Eltrombopag Olamine), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, R-CHOP, R-CVP, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), R-EPOCH, Revlimid (Lenalidomide), Rheumatrex (Methotrexate), Ribociclib, R-ICE, Rituxan (Rituximab), Rituxan Hycela (Rituximab and Hyaluronidase Human), Rituximab, Rituximab and , Hyaluronidase Human, ,Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), STANFORD V, Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), TAC, Tafinlar (Dabrafenib), Tagrisso (Osimertinib), Talc, Talimogene Laherparepvec, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Tecentriq , (Atezolizumab), Temodar (Temozolomide), Temozolomide, Temsirolimus, Thalidomide, Thalomid (Thalidomide), Thioguanine, Thiotepa, Tisagenlecleucel, Tolak (Fluorouracil-Topical), Topotecan Hydrochloride, Toremifene, Torisel (Temsirolimus), Tositumomab and Iodine I 131 Tositumomab, Totect (Dexrazoxane Hydrochloride), TPF, Trabectedin, Trametinib, Trastuzumab, Treanda (Bendamustine Hydrochloride), Trifluridine and Tipiracil Hydrochloride, Trisenox (Arsenic Trioxide), Tykerb (Lapatinib Ditosylate), Unituxin (Dinutuximab), Uridine Triacetate, VAC, Vandetanib, VAMP, Varubi (Rolapitant Hydrochloride), Vectibix (Panitumumab), VeIP, Velban (Vinblastine Sulfate), Velcade (Bortezomib), Velsar (Vinblastine Sulfate), Vemurafenib, Venclexta (Venetoclax), Venetoclax, Verzenio (Abemaciclib), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, VIP, Vismodegib, Vistogard (Uridine Triacetate), Voraxaze (Glucarpidase), Vorinostat, Votrient (Pazopanib Hydrochloride), Vyxeos (Daunorubicin Hydrochloride and Cytarabine Liposome), Wellcovorin (Leucovorin Calcium), Xalkori (Crizotinib), Xeloda (Capecitabine), XELIRI, XELOX, Xgeva (Denosumab), Xofigo (Radium 223 Dichloride), Xtandi (Enzalutamide), Yervoy (Ipilimumab), Yondelis (Trabectedin), Zaltrap (Ziv-Aflibercept), Zarxio (Filgrastim), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zofran (Ondansetron Hydrochloride), Zoladex (Goserelin Acetate), Zoledronic Acid, Zolinza (Vorinostat), Zometa (Zoledronic Acid), Zydelig (Idelalisib), Zykadia (Ceritinib), and/or Zytiga (Abiraterone Acetate). Anti-cancer agents and immune regulators can also include checkpoint inhibitors. Checkpoint inhibitors include, but are not limited to, antibodies that block PD-1 (JTX-4014, cemiplimab, Camrelizumab, dostarlimab, Toripalimab, Tislelizumab, Spartalizumab, Sintilimab, Nivolumab (BMS-936558 or MDX1106), CT-011, pembrolizumab (MK-3475)), PD-L1 (atezolizumab, avelumab, durvalumab, CK-301, MDX-1105 (BMS-936559), MPDL3280A, MSB0010718C), PD-L2 (rHIgM12B7), CTLA-4 (Ipilimumab (MDX-010) and Tremelimumab (CP-675,206)), IDO, B7-H3 (MGA271), B7-H4, TIM3, LAG-3 (relatimab, BMS-986016).

239. In certain embodiments, the method of administration is in a subject with a lymphodepleted environment. In certain embodiments, lymphodepleting agents are cyclophosphamide and fludarabine.

240. As used herein the term "idelalisib" refers to the compound (S)-2-(1-(9H-purin-6-ylamino)propyl)-5-fluoro-3-phenylquinazolin-4(3H)-one or alternative salts thereof.

241. The disclosed VIP-R antagonists can further be combined with radiotherapy and/or adoptive cell transfer therapies including but not limited to the administration of expanded, modified, or cultured tumor infiltrating lymphocytes (TILs); expanded, modified, or cultured marrow infiltrating lymphocytes (MILs); expanded, modified, or cultured Tumor infiltrating Natural killer cells (TINKs); chimeric antigen receptor (CAR) T cells; TCR Modified T Cells, and/or CAR NK cells. In certain embodiments, this disclosure relates to methods of treating a subject diagnosed with cancer comprising administering a cell in combination with any of the VIP-R antagonists disclosed herein to a subject in need thereof. In certain embodiments, the subject is diagnosed with leukemia. In certain embodiments, the subject is diagnosed with lymphoma. In certain embodiments, the cell is a blood mononuclear cell. In certain embodiments, the cell is a bone marrow cell. In certain embodiments, the cell is a leukocyte. In certain embodiments, the cell is a T-cell. In certain embodiments, the cell is a natural killer cell. In certain embodiments, the cell is a hematopoietic stem cell. In certain embodiments, the cell is a G-CSF mobilized blood mononuclear cell. In certain embodiments, the cell is an HLA matched or mis-matched allogeneic cell. In certain embodiments, the cell is syngeneic cell. In certain embodiments, the cell is an autologous cell. In certain embodiments, the peptide has a C-terminal amide and/or is optionally modified with hydrocarbon or polyethylene glycol groups. In one aspect, disclosed herein are methods of treating, inhibiting, reducing, decreasing, ameliorating, and/or preventing leukemia comprising administering any of the VIP-R antagonists disclosed herein (such as, for example, SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to a subject in combination with transplanting hematopoietic stem cells. In certain embodiments, this disclosure relates to methods comprising expanding lymphocytes in vitro providing expanded cells and exposing the expanded cells with any of the VIP-R antagonists disclosed herein.

242. In certain embodiments, the disclosure relates to methods of treating cancer by performing a stem cell transplantation comprising administering any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) to the subject in combination with transplanting a multipotent hematopoietic stem cell derived from the subject (self) or a donor. The stem cells may be collected from peripheral blood such as cord blood or placenta-derived stem cells or from the bone marrow. To limit the risks of transplanted stem cell rejection or of severe graft-versus-host disease, the donor will typically have the substantially the same human leukocyte antigens (HLA) as the recipient; however, the donor may have mis-matches for certain antigens. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

243. In certain embodiments, the disclosure relates to methods of providing lymphocyte infusions after a hematopoietic progenitor cell transplant to treat a hematologic malignancy (e.g., cancer of the blood or bone marrow, such as leukemia or lymphoma). A transplant recipient is typically infused with lymphocytes obtained in a leukapheresis procedure from the original allogeneic stem cell (hematopoietic progenitor cell) donor.

244. In certain embodiments, the disclosure relates to extraction of lymphocytes from the blood and expanding in vitro against tumor antigen(s) and optionally exposing the cells with an appropriate stimulatory cytokine and/or any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof).

245. In certain embodiments, the disclosure relates to methods of enhancing topical immunotherapies comprising administering any VIP-R antagonist disclosed herein (such as, for example, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) in combination with providing an immune enhancement cream, such as imiquimod, comprising an interferon-producing drug that causes the activation of T-cells. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

246. In certain embodiments, it is contemplated that peptides disclosed herein can be used in combination with adoptive cell therapies. For example, T cells with a naturally occurring reactivity to cancer can be found infiltrated in tumors of the subject. The tumor can be harvested, and these tumor-infiltrating lymphocytes (TIL) can be expanded, or made more effective, in vitro using interleukin-2 (IL-2), anti-CD3 and allo-reactive feeders. These T cells can then be transferred back into the subject along with administration of a VIP-R antagonist. Before reinfusion, lymphodepletion of the recipient is typically done to eliminate regulatory T cells as well as normal endogenous lymphocytes that compete with the transferred cells. It is also contemplated that the adoptive cell transfer of lymphocytes may be transduced with a vector encoding T cell receptors (TCRs) that recognize a cancer antigen. In some embodiments, the method comprises further administering to the subject a therapeutically effective amount of a phosphatidylinositol 3-kinase (PI3K) inhibitor (for example, a PI3Kα inhibitor, a PI3Kβ inhibitor, a PI3Kδ inhibitor, or a PI3Kγ inhibitor). In some embodiments, the method comprises further administering to the subject a therapeutically effective amount of an immune checkpoint blockade (e.g., a PD-1 inhibitor, a PD-L1 inhibitor, or a CTLA-4 inhibitor).

247. In certain embodiments, this disclosure relates to methods of augmenting T-cell activation and ex vivo expansion by co-incubation of human T cells with a nanoparticle containing a small molecule antagonist of VIP signaling. In certain embodiments, the human T cells are activated with anti-CD3 antibody bound to a plate. In certain embodiments, the human T cells are activated in a mixed lymphocyte reaction. In certain embodiments, the human T cells are activated in vitro by co-incubation with tumor-associated antigens. In certain embodiments, the tumor associate antigens are presented on tumor microvesicles. In certain embodiments, the activated human T cells are infused into a human patient with cancer.

248. In certain embodiments, the activated human T cells are infused into a human patient with cancer. In certain embodiments, the human patient with cancer has leukemia. In certain embodiments, the human patient with cancer has lymphoma. In certain embodiments, the human patient with cancer has multiple myeloma. In certain embodiments, the human patient with cancer has an epithelial cancer. In certain embodiments, the human patient has lung cancer. In certain embodiments, the human patient has breast cancer. In certain embodiments, the human patient has colon cancer. In certain embodiments, the human patient has prostate cancer. In certain embodiments, the human patient has malignant melanoma. In certain embodiments, the human patient has brain cancer.

249. In certain embodiments, this disclosure relates to methods of augmenting anti-cancer immune responses by infusion of any of the VIP-R antagonists disclosed herein (such as, for example, SEQ ID NO: 6, SEQ ID NO: 7; SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, and/or SEQ ID NO: 16, a fragment thereof, or an analog thereof) or nanoparticle expressing a VIP-R antagonist disclosed herein. In certain embodiments, the activated T-cells are infused into a patient with chronic CMV infection. In certain embodiments, the activated T-cells are infused into a patient with chronic EBV infection. In certain embodiments, the activated T-cells are infused into a patient with chronic BK virus infection. In certain embodiments, the activated T-cells are infused into a patient with chronic adenovirus infection.

250. Particular embodiments of the invention include:
1. A vasoactive intestinal peptide receptor (VIP-R) antagonist comprising KPRRPYX¹X²X³X⁴TX⁵LRKQX⁶AVX⁷X⁸KYLX⁹X¹⁰ILN (SEQ ID NO: 3) or a fragment thereof, wherein
   X¹ is T or A;
   X² is D, V, or S;
   X³ is N or D;
   X⁴ is Y or C;
   X⁵ is R or S;
   X⁶ is M or I;
   X⁷ is K or N;
   X⁸ is K or absent;
   X⁹ is N or M;
   X¹⁰ is S, or L; and
   provided that the peptide is not KPRRPYTDNYTRLRKQMAVKKYLNSILN (SEQ ID NO: 1) or the combination wherein X¹ is T, X² is D, X³ is N; X⁴ is Y, X⁵ is R, X⁶ is M, X⁷ is K, X⁹ is N, and X¹⁰ is S.
2. A vasoactive intestinal peptide receptor (VIP-R) antagonist comprising KPRRPYX¹X²X³X⁴TX⁵LRKQX⁶AVX⁷KYLX⁸X⁹ILN (SEQ ID NO: 21) or a fragment thereof, wherein
   X¹ is T or A;
   X² is D, V, or S;
   X³ is N or D;
   X⁴ is Y or C;
   X⁵ is R or S;
   X⁶ is M or I;
   X⁷ is K or N;
   X⁸ is N or M;
   X⁹ is S, or L; and
   provided that the peptide is not KPRRPYTDNYTRLRKQMAVKKYLNSILN (SEQ ID NO: 1) or the combination wherein X¹ is T, X² is D, X² is N; X⁴ is Y, X⁵ is R, X⁶ is M, X⁷ is K, X⁸ is N, and X⁹ is S.
3. The VIP-R antagonist of claim 1 or 2, wherein the VIP-R antagonist comprises the amino acid sequence:
   KPRRPYADNYTRLRKQMAVNKYLNLILN (SEQ ID NO: 6),
   KPRRPYAVNYTRLRKQIAVKKYLMSILN (SEQ ID NO: 7),
   KPRRPYAVNYTRLRKQMAVNKYLMSILN (SEQ ID NO: 8),
   KPRRPYADNCTRLRKQIAVNKKYLNSILN (SEQ ID NO: 9),
   KPRRPYTVNYTSLRKQIAVKKYLMLILN (SEQ ID NO: 10),
   KPRRPYTDNCTSLRKQIAVNKYLNLILN (SEQ ID NO: 11),
   KPRRPYAVNCTSLRKQIAVNKYLNSILN (SEQ ID NO: 12),
   KPRRPYAVNCTSLRKQIAVKKYLMSILN (SEQ ID NO: 13),
   KPRRPYTVNCTSLRKQIAVKKYLMLILN (SEQ ID NO: 14),
   KPRRPYTSDYTRLRKQMAVKKYLNSILN (SEQ ID NO: 15),
   KPRRPYTSDYTRLRKQMAVKKYLNLILN (SEQ ID NO: 16), or a fragment thereof.
4. The VIP-R antagonist of any of Embodiments 1-3, wherein an amino, carboxyl, hydroxyl, or thiol group in the peptide is substituted.
5. The VIP-R antagonist of any of Embodiments 1-4, wherein the peptide is conjugated to and/or encapsulated within a nanoparticle.
6. A pharmaceutical composition comprising the vasoactive intestinal peptide receptor (VIP-R) antagonist of any of Embodiments 1-5 and a pharmaceutically acceptable carrier.
7. The pharmaceutical composition of Embodiment 6 in the form of a capsule, tablets, pill, powder, or granule.
8. The pharmaceutical composition of Embodiment 6 in the form of a sterilized pH buffered aqueous salt solution.
9. A nucleic acid encoding the amino acid sequence of a vasoactive intestinal peptide receptor (VIP-R) antagonist of any of Embodiments 1-5.
10. The nucleic acid encoding the amino acid sequence of embodiment 9, wherein the nucleic acid is in operable combination with a promoter.
11. An expression vector comprising the nucleic acid of Embodiment 9 or 10.
12. A cell comprising the expression vector of Embodiment 11.
13. A method of *ex vivo* augmenting T cell activation and/or expansion, comprising mixing one or more T cells with the vasoactive intestinal peptide receptor (VIP-R) antagonist of any of Embodiments 1-5 or the pharmaceutical composition of any of Embodiments 6-8.
14. The method of Embodiment 13, wherein mixing the one or more T cells is in combination with an anti-CD3 antibody and/or an anti-CD28 antibody.
15. The method of Embodiment 13 or 14, wherein mixing the one or more T cells is in combination with a phosphatidylinositol 3-kinase (PI3K) inhibitor.
16. The method of Embodiment 15, wherein the PI3K inhibitor is a PI3Kα inhibitor, a PI3Kβ inhibitor, a PI3Kδ inhibitor, or a PI3Kγ inhibitor.
17. The method of Embodiment 15 or 16, wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
18. The method of any of Embodiments 13-17, wherein mixing the one or more T cells is in combination with an immune checkpoint blockade.
19. The method of Embodiment 18, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
20. The method of Embodiment 19, wherein the immune checkpoint blockade comprises a PD-1 inhibitor, a PD-L1 inhibitor, or a CTLA-4 inhibitor.
21. The method of Embodiment 20, the immune checkpoint blockade comprises pembrolizumab, nivolumab, cemiplimab, dostarlimab, atezolizumab, avelumab, durvalumab, or ipilimumab.
22. A kit comprising the vasoactive intestinal peptide receptor (VIP-R) antagonist of any of Embodiments 1-5 or the pharmaceutical composition of any of Embodiments 6-8.
23. The kit of Embodiment 22, further comprising an anti-CD3 antibody and/or anti-CD28 antibody
24. The kit of Embodiment 22 or 23, further comprising a phosphatidylinositol 3-kinase (PI3K) inhibitor.
25. The kit of Embodiment 24, wherein the PI3K inhibitor is a PI3Kα inhibitor, a PI3Kβ inhibitor, a PI3Kδ inhibitor, or a PI3Kγ inhibitor.
26. The kit of Embodiment 24 or 25, wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
27. The kit of any one of Embodiments 20-26, further comprising an immune checkpoint blockade.
28. The kit of Embodiment 27, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
29. The kit of Embodiment 28, wherein the immune checkpoint blockade comprises a PD-1 inhibitor, a PD-L1 inhibitor, or a CTLA-4 inhibitor.
30. The kit of Embodiment 29, wherein the immune checkpoint blockade comprises pembrolizumab, nivolumab, cemiplimab, dostarlimab, atezolizumab, avelumab, durvalumab, or ipilimumab.
31. An *in vitro* cell culture composition, comprising
   one or more T cells, and
   the vasoactive intestinal peptide receptor (VIP-R) antagonist of any of Embodiments 1-5 or the pharmaceutical composition of any of Embodiments 6-8.
32. The *in vitro* cell culture composition of Embodiment 31, further comprising an anti-CD3 antibody and/or anti-CD28 antibody.
33. The *in vitro* cell culture composition of Embodiment 31 or 32, further comprising a phosphatidylinositol 3-kinase (PI3K) inhibitor.
34. The *in vitro* cell culture composition of Embodiment 33, wherein the PI3K inhibitor is a PI3Kα inhibitor, a PI3Kβ inhibitor, a PI3Kδ inhibitor, or a PI3Kγ inhibitor.
35. The *in vitro* cell culture composition of Embodiment 33 or 34, wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
36. The *in vitro* cell culture composition of any one of Embodiments 31-35, further comprising an immune checkpoint blockade.
37. The *in vitro* cell culture composition of Embodiment 36, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
38. The *in vitro* cell culture composition of Embodiment 36 or 37, wherein the immune checkpoint blockade comprises a PD-1 inhibitor, a PD-L1 inhibitor, or a CTLA-4 inhibitor.
39. The *in vitro* cell culture composition of Embodiment 38, wherein the immune checkpoint blockade comprises pembrolizumab, nivolumab, cemiplimab, dostarlimab, atezolizumab, avelumab, durvalumab, or ipilimumab.
40. A method of treating a microbial infection in a subject in need, comprising administering to the subject infected with a microbe or at risk for a microbial infection a therapeutically effective amount of the vasoactive intestinal peptide receptor (VIP-R) antagonist of any of Embodiments 1-5 or the pharmaceutical composition of any of Embodiments 6-8.
41. The method of embodiment 40, wherein the microbial infection is a viral infection, a bacterial infection, a fungal infection, and/or a parasitic infection.
42. The method of Embodiment 40 or 41, further comprising administering to the subject a therapeutically effective amount of a phosphatidylinositol 3-kinase (PI3K) inhibitor.
43. The method of embodiment 42, wherein the PI3K inhibitor is a PI3Kα inhibitor, a PI3Kβ inhibitor, a PI3Kδ inhibitor, or a PI3Kγ inhibitor.
44. The method of embodiment 42 or 43, wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
45. The method of any one of Embodiments 40-44, further comprising administering to the subject a therapeutically effective amount of an immune checkpoint blockade.
46. The method of Embodiment 45, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
47. The method of Embodiment 45 or 46, wherein the immune checkpoint blockade comprises a PD-1 inhibitor, a PD-L1 inhibitor, or a CTLA-4 inhibitor.
48. The method of embodiment 47, wherein the immune checkpoint blockade comprises pembrolizumab, nivolumab, cemiplimab, dostarlimab, atezolizumab, avelumab, durvalumab, or ipilimumab.
49. A method of treating a cancer and/or metastasis in a subject in need, comprising administering to the subject a therapeutically effective amount of the vasoactive intestinal peptide receptor (VIP-R) antagonist of any of Embodiments 1-5 or the pharmaceutical composition of any of Embodiments 6-8.
50. The method of Embodiment 49, wherein the cancer comprises pancreatic cancer, colon cancer, leukemia, liver cancer, lung cancer, or melanoma.
51. The method of Embodiment 49 or 50, further comprising administering to the subject a therapeutically effective amount of a phosphatidylinositol 3-kinase (PI3K) inhibitor.
52. The method of Embodiment 51, wherein the PI3K inhibitor is a PI3Kα inhibitor, a PI3Kβ inhibitor, a PI3Kδ inhibitor, or a PI3Kγ inhibitor.
53. The method of Embodiment 51 or 52, wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
54. The method of any one of Embodiments 49-53, further comprising administering to the subject a therapeutically effective amount of an immune checkpoint blockade.
55. The method of Embodiment 54, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
56. The method of Embodiment 54 or 55, wherein the immune checkpoint blockade comprises a PD-1 inhibitor, a PD-L1 inhibitor, or a CTLA-4 inhibitor.
57. The method of embodiment 56, wherein the immune checkpoint blockade comprises pembrolizumab, nivolumab, cemiplimab, dostarlimab, atezolizumab, avelumab, durvalumab, or ipilimumab.
58. A method of enhancing an immune response to a cancer and/or metastasis in a subject, comprising administering to the subject a therapeutically effective amount of the vasoactive intestinal peptide receptor (VIP-R) antagonist of any of Embodiments 1-5 or the pharmaceutical composition of any of Embodiments 6-8.
59. The method of Embodiment 58, wherein the cancer comprises pancreatic cancer, colon cancer, leukemia, liver cancer, lung cancer, or melanoma.
60. The method of Embodiment 58 or 59, further comprising administering to the subject a therapeutically effective amount of a phosphatidylinositol 3-kinase (PI3K) inhibitor.
61. The method of embodiment 60, wherein the PI3K inhibitor is a PI3Kα inhibitor, a PI3Kβ inhibitor, a PI3Kδ inhibitor, or a PI3Kγ inhibitor.
62. The method of embodiment 60 or 61, wherein the the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.
63. The method of any one of Embodiments 58-62, further comprising administering to the subject a therapeutically effective amount of an immune checkpoint blockade.
64. The method of Embodiment 63, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-l-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.
65. The method of Embodiment 63 or 64, wherein the immune checkpoint blockade comprises a PD-1 inhibitor, a PD-L1 inhibitor, or a CTLA-4 inhibitor.
66. The method of embodiment 65, wherein the immune checkpoint blockade comprises pembrolizumab, nivolumab, cemiplimab, dostarlimab, atezolizumab, avelumab, durvalumab, or ipilimumab.
67. A method of treating a cancer or a chronic infection in a subject in need, comprising
   providing one or more T cells;
   mixing the one or more T cells with the vasoactive intestinal peptide receptor (VIP-R) antagonist of any of Embodiments 1-5 or the pharmaceutical composition of any of Embodiments 6-8 thereby expanding the one or more T cells; and
   administering a therapeutically effective amount of the expanded T cells to the subject.
68. The method of embodiment 67, wherein mixing the one or more T cells is in combination with an anti-CD3 antibody and/or an anti-CD28 antibody.
69. The method of embodiment 67 or 68, wherein mixing the one or more T cells is in combination with a phosphatidylinositol 3-kinase (PI3K) inhibitor.
70. The method of embodiment 69, wherein the PI3K inhibitor is a PI3Kα inhibitor, a PI3Kβ inhibitor, a PI3Kδ inhibitor, or a PI3Kγ inhibitor.
71. The method of any one of embodiments 67-70, wherein mixing the one or more T cells is in combination with an immune checkpoint blockade.
72. The method of any one of embodiments 67-71, wherein the one or more T cells are derived from the subject.
73. The method of any one of embodiments 67-72, wherein the one or more T cells comprises a chimeric antigen receptor.
74. The method of any one of embodiments 67-73, wherein the expanded T cells have increased levels of CD28 and/or CD27 compared with levels prior to expansion.
75. The method of any one of embodiments 67-74, wherein expanded T cells have decreased levels of PD-1, TIM-3, and/or Lag3 compared with levels prior to expansion.
76. The method of any one of embodiments 67-75, further comprising administering to the subject a PI3 kinase inhibitor or a VIP receptor antagonist, or an immune checkpoint blockade, or a combination thereof before, during, or after administering the expanded T cells.

### VI. EXAMPLES

251. The following examples are put forth so as to provide those of ordinary skill in the art with a complete disclosure and description of how the compounds, compositions, articles, devices and/or methods claimed herein are made, evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

252. Whether tumor specific expression of vasoactive intestinal polypeptide represents a mechanism of tumor-mediated immune escape was evaluated. There is a spectrum of VIP expression across tumors with highest expression being seen in pancreatic exocrine cancer and lowest expression seen in melanoma. In general, levels of VIP expression by tumors are inversely proportional to the expression of other co-inhibitory pathway molecules such as PDL1. Some tumors expressing and secreting VIP may have mutations in VIP coding sequence, such that the resultant peptide molecule secreted by the cancer has improved pharmacokinetics or pharmacodynamics in the tumor microenvironment. The pharmacokinetic advantage to the cancer cell secreting VIP might be that the mutation renders the mutant VIP less susceptible to proteases, improving its half-life. A pharmacodynamic advantage to the cancer in more potently inhibiting anti-cancer immunity might be the consequence of a mutation in VIP that enhances binding affinity to the receptor, thus enhancing inhibitory signaling on the T cell that expresses VAPC1 and/or VPAC2.

253. Experiments were performed to determine whether mutated VIP produced by tumors would lead to more sustained suppression of anticancer T-cells in the tumor microenvironment. Analyzing mutations in specific genes curated from deposited tumor sequences, there are multiple cancers with mutations in the coding sequence of VIP. In particular, there were 140 missense mutations and 17 truncating mutations within the VIP gene cluster listed in the Cancer Genome Atlas. Within the coding sequence of the 28 amino acid VIP peptide, mutations were identified in VIP coding sequence present in breast cancer, prostate adenocarcinoma, esophageal adenocarcinoma, cutaneous melanoma, small-cell lung cancer, stomach adenocarcinoma, uterine endometrial carcinoma, cutaneous melanoma, esophageal adenocarcinoma, colorectal adenocarcinoma, uterine carcinosarcoma, hepatocellular adenoma, lung adenocarcinoma, stomach adenocarcinoma. Eight specific mutations that were present in the C-terminal amino acids including the alpha helix of VIP that binds the receptor.

254. VIP-related peptides tested in silico for predicted binding affinities to human VPAC1 and VPAC2 (Creative Biolabs). Docking scores show predicted free energy change associated with peptide binding. Note that a greater negative score is predicted to be associated with higher binding affinity. Based upon an initial screen of 300 peptides for binding affinity to VPAC1 and screening a subset of 100 peptides for binding affinity to VPAC2, a select group of peptides were synthesized and tested for their ability to stimulate proliferation of luciferase+ mouse T cells in vitro. The lowest concentration of peptide that led to maximal luminescence is shown. Proliferation data represents the relative level of luminescence from quadruplicate wells containing 1 x 10E5 T cells/well in a 96 well tissue culture plate stimulated for 96 hours with plate-bound anti-CD3 antibodies. Confirmatory *in vivo* testing of select peptides was then performed based upon their ability to induce autologous anti-leukemia responses in C57Bl/6 mice that had been previously injected with 1 x 10E6 C1498 acute myeloid leukemia cells. Leukemia cells were injected on day 0, 10 ug peptide was injected s.c. daily on days 6-12. The predicted binding affinity to VPAC1 and/or VPAC2 was associated with enhanced activity in stimulating mouse T cell proliferation and anti-leukemia activity in mice. Sequence differences between VIP and tested peptides are shown in red font (Table 3).

255. To test the ability of the disclosed VIP-R antagonists to effect survival to acute myeloid leukemia survival, B6 (CD45.2, H-2K^{b}) mice were administered C1498 1 x 10⁶ /mouse through tail vein. VIP novel peptides were subcutaneously injected 10 microgram per mouse daily from day 6 following leukemia administration, totally 7 doses. Survival of the mice was observed daily. As shown if Figures 1 and 2, in each case, survival was increased relative to negative controls and VIP (SEQ ID NO: 1).

256. Next, we looked at the correlation of increasing survival with the affinity and potency of competitive binding VPAC by each of the VIP-R antagonists. As shown in Figure 3 there is direct correlation between the affinity and potency of competitive binding for both human VPAC1 (Panel A) and VPAC2 (Panel B), and the sum of binding affinity to VPAC1 and VPAC2 (Panel C).

257. Figure 4 shows treatment with VIP derived novel peptides reduced levels of leukemic cells in blood of the leukemic mice up to 20 days following administrations C1498 1 x 10⁶ /mouse through tail vein. VIP novel peptides were subcutaneously injected 10 microgram per mouse daily from day6 following leukemia administration, totally 7 doses.

258. In a rechallenge model of leukemia, mice previously inoculated with C1498 leukemia cells that achieved complete clearance of leukemia after treatment with the VIP-derived novel peptide(s) and had prolonged survival without detectable leukemia were re-challenged with Luc-1498 1 x 10⁶ /mouse through tail vein. 16 days following leukemia re-challenge, the mice were luminescence-imaged by weekly (Figure 5). Additionally, as shown in Figure 6, mice previously treated with the VIP-derived novel peptides had a prolonged survival when re-challenged with acute myeloid leukemia, indicating that prior treatment with the VIP-derived novel peptide(s) had developed immunological memory that allowed the mice to reject the newly injected leukemia cells.

259. Additionally, we have tested the ANT308 peptide in two models of pancreatic cancer as a single agent and in combination with anti-PD1 MoAb and found that ANT308 has single agent activity in controlling tumor growth as well as synergy when it is combined with anti-PD1 therapy. As shown in Figure 7, ANT308 treatment reduced tumor volume in of C57BL/6 mice with subcutaneously implanted KPC tumors. This decease in tumor volume was more pronounced in combination with anti-PD1 treatment. We also examined the effect of ANT308 in combination with Iso IgG or anti-PD1 antibody had on tumor volume as measured as a percent change in volume (Figure 8A) and in measured volume (Figure 8B). Interestingly, treatment with the combination of VIP-R antagonist and anti-PD1 had a synergistic effect on decreasing tumor volume measurements and controlling the growth of subcutaneous pancreatic tumors in mice (Figure 8C). Investigating this effect more completely, we observed that combination treatment with both anti-PD1 and VIP-R antagonist was able to slow tumor growth relative to control mice treated with scrambled peptide and iso-type matched IgG, with the combination showing a statistically significant improvement in the rate of tumor growth (Figure 8D). Examining viability and regression of each of the various treatment groups further, we observed that the 20% of mice treated with ANT308 and anti-PD1 were tumor free with an additional 50% of tumors showing regression. In comparison, only 10% of anti-PD1 treated mice were tumor free, with 20% showing tumor regression, with 30% of the remainder having progressing tumors, and 40% of mice euthanized due to the growth of large tumors. The growth of tumors in mice treated with ANT308 alone was not statistically different from the growth of tumors in control mice that received scrambled peptide and isotype-matched IgG (Figure 9).

### 2. Example 2: Treatment of human T cells with VIP-R antagonists (Ant08, Ant308, Ant195)

260. This study adopted an *ex-vivo* system using human T cells isolated from healthy donors to screen for novel VIP-R antagonist peptides. Human T cells were cultured over 6 or 24 hours with activation in the presence or absence of peptides and were assessed for percent CD69 surface expression by flow cytometry to measure the state of T cell activation. The activity of the peptides was determined by percent increase in CD69 expression relative to no peptide control. VIP scrambled 1 (VS1) was used as a peptide control. Two novel peptides, Ant308 and Ant195 were tested for activity when compared to Ant08, which has demonstrated high activity in our previous screens. Thus far, all screenings were performed using murine T cells, however, this study reports an alternative screening method using human T cells, which allows robust results within 24 hours that informs the activity of peptides on different human T cell subtypes. Using this method, this study shows that treatment of human T cells activated in vitro in the presence of VIP-R antagonists, Ant08, Ant308, and Ant195, demonstrate increase activation as early as 6 hours after treatment with approximately 10 to 15% increase in the mean CD69 expression relative to T cells in no-peptide control cultures (---) (Figure 10A). All donors, represented with dots of same color, show an increase in CD69 expression at 6 hours in the antagonist-treated group despite the donor-to-donor variability. Of the three antagonists, Ant195 shows highest activity across multiple donors. The activity of the antagonists to stimulate T cells is further shown to be maintained at 24 hour, an effect which is more prominent in the CD4+ T cells compared to CD8+ T cells (Figure 10B). However, the results also indicate that VS1 peptide stimulates T cells compared to no-peptide control by ~3%, although lower than the three antagonists on question. We also observed that amongst the donors, most were classified as average responders, with measured value for CD69 expression within the error bars while two others had measured values outside the error bars, one sample reflecting a super-responder (purple dots) and one sample reflecting a slow-responder (green dots) (Figures 10A and 10B). A representative flow plot from one average responder (blue dots) showing higher percentage of CD4+CD69+ in the presence of VIP-R antagonists at 24hrs compared to control, as shown by increasing intensity of red signal in the gated population (Figure 10C). Overall, this study shows that all VIP-R antagonists can augment activation of human T cells, with data supporting that Ant195 had the highest activity when compared to Ant08 and Ant308.

261. Using the same T cells, the effect of treatment on T cell activation was assessed as indicated by TIM3 expression (Figure 11). As with CD69, the percentage of CD4+TIM3+ and CD8+TIM3+ cells increased at 24 hours in treated groups. By contrast CXCR4 expression decreased at 24 hours in treated mice (Figure 12).

262. This study also investigated the expression VIP receptors in the pancreatic cancer model using various pancreatic cancer cells (KPC, Panc02, PANC01, Capan02, BxPC3, and MT5). As measured by western blot, VPAC1, VPAC2, and PAC1 were all expressed in the cancer cells (Figures 13A-13D). Additionally, viability and growth of the cells was unaffected by treatment with VIP-R antagonists.

263. The next experiment assessed the ability of ANT308 and anti-PD-1 treatment to effect adoptively transferred T cell homing and infiltration into established pancreatic tumors. Mice were injected with 5x10⁵ KPC cells subcutaneously and 15 days post inoculation were treated with an infusion of immunologically naive GFP+ T cells along with ANT308 and anti-PD-1 antibody. While mock-treated control mice showed minimal infiltration of GFP+ T cells, mice treated with ANT308 and anti-PD-1 showed significant infiltration of GFP+ T cells (Figures 14A-14C). Lastly, this study measured the effect of the combination of ANT308 and anti-PD-1 on survival. While mock-treated animals inoculated with pancreatic cancer cells all died by 30 days, the treated group had 90% survival at least to 35 days post inoculation (Figure 15). Unlike many solid tumor malignancies, pancreatic ductal adenocarcinoma (PDAC) is generally unresponsive to immune checkpoint blockade (ICB) therapies that target molecules such as programmed death-1 (PD-1), programmed death-ligand 1 (PD-L1) or cytotoxic T lymphocyte antigen-4 (CTLA-4). Therapeutic resistance of PDAC to ICB is thought to be partly due to low tumor mutational burden, with the exception of the very small fraction of PDCA patients with tumors that have high microsatellite instability. In addition, the tumor microenvironment (TME) in PDAC is characterized by cancer-associated fibroblasts (CAF) that secrete immunosuppressive proteins and metabolites, abundant regulatory T cells (Tregs), immunosuppressive tumor-associated macrophages (TAMs) and dendritic cells, and limited numbers of functional T cells. While several clinical strategies have targeted immunosuppressive cells in some cancers, there has been limited improvement in the clinical management of PDAC over the past few years. Recently, pre-clinical and translational clinical studies have shown that the combination of treatment with agnostic CD40 antibody and gemcitabine/nab-paclitaxel can induce potent immune-mediated control of PDAC but these promising pre-clinical results were not recapitulated in an early phase clinical trial. This study identified overexpression of VIP, an immunosuppressive neuropeptide, as a novel target for immune checkpoint therapy in PDAC.

264. VIP is a 28-amino acid long neuropeptide present in the brain, pancreas, colon and lung. Overexpression of VIP and its receptors has been previously reported in breast, prostate, and lung cancers, where it was noted to promote growth and metastasis of tumors. Immune cells including T cells have VIP receptors that are upregulated upon T cell activation and respond to VIP receptor signaling by inhibiting activation and proliferation as well as promoting the generation of Tregs and Th2 cells. Inhibition of VIP-R signaling by treating mice with a VIP-R antagonist improved T cell-dependent antitumor response in preclinical models of acute leukemia, and augmented adaptive anti-viral immunity. This study explores VIP-R antagonists in solid tumor cancer models.

265. This study shows that VIP is robustly expressed in murine and human PDAC in comparison to most other solid tumor malignancies. Paracrine production of VIP production by tumor cells within the PDAC TME constitutes an immune checkpoint pathway that limits the antitumor activity of VIP-receptor-expressing T cells. Inhibition of VIP receptor signaling can improve T cell-dependent responses to checkpoint therapy and improve survival in preclinical models of PDAC. This study investigated these concepts by treating tumor-bearing mice with more potent VIP-R antagonists designed to have higher binding affinities to VIP receptors compared to VIPhyb. Shown herein is that the combination of VIP-R antagonist and anti-PD-1 significantly enhances activation, decreases exhaustion and recruits tumor-infiltrating T cells in murine PDAC (TIL). Furthermore, combination drug therapy decreases tumor growth rates, resulting in regression of tumors.

266. **VIP is overexpressed in human and murine pancreatic cancer.** Expression levels of VIP mRNA across different human tumors were compared using the Cancer Genome Atlas (TCGA). Pancreatic and gastrointestinal cancers had the highest VIP mRNA expression when compared to other solid tumor malignancies (Fig. 16a). Immunofluorescence (IF) staining of human PDAC tumors showed increased expression of VIP in pancreatic ductal carcinoma cells with co-expression of cytokeratin- 19 (CK19) when compared to adjacent normal tissues (Figs. 16b and 23a). Additionally, analysis of culture supernatants obtained from human and murine PDAC cell lines showed that most PDAC cell lines secrete VIP (Fig. 16c). On the other hand, supernatants from murine melanoma cell lines B16F10 and D4M had low to undetectable VIP (Fig. 16c). The potential for tumor-secreted VIP to have systemic effects on the immune system is supported by the observation that immunocompetent C57BL/6 mice implanted with murine PDAC tumors had significantly elevated levels of plasma VIP when compared to mice with comparable tumor volumes of B16F10 melanoma (Fig. 16d). These findings are in accordance with the human VIP mRNA expression data in which melanoma had low expression of VIP, while PDAC had high levels of VIP mRNA. Similarly, human PDAC patients with pancreatic cancer had significantly higher plasma VIP levels than healthy volunteers (Fig. 16e), indicating that plasma VIP is a biomarker for PDAC. Corroborating this notion, plasma VIP increased linearly with increased volume of KPC-Luc tumors in mice (Fig. 16f).

267. Orthotopic implantation of KPC-Luc cells resulted in higher plasma levels of VIP than both culture supernatants of KPC-Luc and plasma from mice with subcutaneous KPC-Luc tumors, suggesting that the desmoplastic TME created in the orthotopic model contributes to higher blood VIP levels (Fig. 16d). In support of this, supernatants from primary CAFs from PDAC patients and that from h-iPSC-PDAC-1, a human pancreatic CAF cell line, secreted high levels of VIP (Fig. 16g). These data confirm expression of high levels of VIP by tumor cells and stromal cells within the TME of human and murine PDAC.

268. **Inhibiting VIP-R signaling promoted T cell activation while decreasing exhaustion *in vitro.*** Expression of VIP by PDAC and the TME showed that VIP may function as a paracrine and/or autocrine factor for cancer cell survival. Human PDAC tissues express both VPAC1 and VPAC2, the two VIP receptors that appear most relevant to VIP-R signaling on immune cells. Additionally, VPAC1 and VPAC2 expression is confirmed in both murine and human PDAC cell lines via western blot (Figures 23b-23d). Thus, to test the autocrine effect of VIP made by tumor cells on VIP-receptor expressing tumor cells, we first evaluated whether inhibiting VIP-R signaling affects the growth of PDAC cells *in vitro.* These and subsequent studies used peptide VIP-R antagonists predicted to have greater receptor affinity to human VPAC1 and VPAC2 than VIPhyb based upon *in silico* modeling. Treatment of PDAC cells with increasing concentrations of ANT008 did not affect viability of PDAC cell lines *in vitro,* except a transient effect in MT5 (Figure 24a). Notably, growth of KPC-Luc, Panc02, Capan02 and BxPC3 was not affected by addition of VIP-R antagonists (Figure 24a). To test whether VIP produced by PDAC can have an autocrine effect on PDAC growth through VIP-R, VPAC2 was knocked out from the PDAC cell line Panc02 (Figures 24b-24d). VPAC2-knockout Panc02 cells had similar growth rates *in vitro* compared to the wild-type parenteral cell line (Figure 24e). Treatment of VPAC2 knockout or wild-type Panc02 cells with the VIP-R antagonist ANT308 did not inhibit *in vitro* growth (Figure 24f). VPAC2 KO cells had a slight *in vivo* growth delay as compared to wild-type cells (Figure 24g) and improved survival (Figure 24h), suggesting a modest direct effect of VIP-R signaling on tumor growth mediated by the VPAC2 receptor in the Panc02 cell line.

269. It has been previously reported that inhibiting VIP-R signaling with VIPhyb decreases phosphorylation of CREB (phospho-CREB) and enhances T cell proliferation. This study evaluated the effect of ANT008 or ANT308 on downstream phospho-CREB signaling leading to higher T cell activation and proliferation. Human T cells activated with anti-CD3 antibody up-regulated VPAC1 and VPAC2 expression within 48 hours of activation (Figures 17a and 17b), with kinetics that are slightly delayed relative to that of PD-1 and CTLA-4, the two targetable immune checkpoint molecules (Figures 17a and 17c). To determine the effect of VIP-R antagonists on T cell activation, this study measured CD69 expression after treatment with scrambled-VIP-sequence control peptide (Scram), ANT008, or ANT308 using the gating strategy shown in Figure 25. Inhibiting VIP-R signaling with the addition of VIP-R antagonists significantly increased CD69 expression (Figure 17d). Notably, ANT308, which has higher predicted binding affinity to VPAC1 and VPAC2 compared to ANT008, significantly increased levels of CD69 in both CD4 and CD8 human T cells (Figure 17d). Similarly, addition of ANT308 to the cultures more potently inhibited activation-induced phosphorylation of CREB and increased T cell activation, when compared to treatment with a control scrambled peptide sequence (Figure 17e, Figure 26).

270. This study next investigated the effect of VIP-R antagonist on *ex vivo* expansion of human T cells isolated from PDAC patient peripheral blood. *In vitro* treatment with ANT008 significantly decreased the proportion of human Tregs (CD4+ CD25+ FoxP3+) assessed after 9 days of T cell expansion (Figures 17f and 17g). Additionally, ANT008 also significantly decreased T cells with an "exhausted" phenotype, as measured by the proportion of T cells co-expressing PD1 and Tim-3 or PD1 and Lag-3 or PD1, Tim-3 and Lag-3 in both CD4+ and CD8+ T cell subsets (Figures 17h and 17i, Figure 27).

271. **Antitumor effects of combined blockade of VIP receptor with anti-PD-1 inhibition is T cell-dependent in murine PDAC.** To evaluate the effect of VIP-R-mediated signaling inhibition on the growth of PDAC tumors *in vivo,* three different murine PDAC tumors (KPC-Luc, MT5 and Panc02) were implanted in syngeneic immunocompetent C57BL/6 mice. After tumors were palpable, mice were randomly allocated to treatment with VIP-R antagonists and/or anti-PD-1 monoclonal antibody, or control groups that received scrambled peptide or isotype-matched IgG. Single agent VIP-R antagonist treatment in MT5 cells produced a modest decrease in growth *in vitro* (Figure 28a), and mice bearing MT5 tumors had significantly improved survival and decreased tumor burden following monotherapy with VIP-R antagonist (Figure 18a and Figure 28a), indicating direct inhibition of autocrine signaling of VIP through VIP-R on MT5. In all mice bearing either MT5, KPC- Luc, or Panc02 tumors, the combination of VIP-R antagonist with anti-PD-1 significantly decreased tumor burden (Figures 28a-28c) and improved survival when compared to mice treated with scrambled peptide and isotype matched IgG (Figures 18a and 18b). Notably in all three tumor models, combination therapy resulted in tumor eradication in a significant proportion of mice (40% in KPC and MT5 tumor-bearing mice and 30% in mice with Panc02 tumors) with no difference in outcome by sex of the mice (Figures 28d and 28e). In addition to the antitumor efficacy, the safety of VIP- R antagonists in immunologically naïve mice was confirmed (Figures 29a-29g). Specifically, daily subcutaneous administration of ANT008 or ANT308 at the same dose and frequency as used in the anti-cancer treatment protocol did not affect the overall survival, activity levels or weight of mice (Figure 29a). Analysis of blood samples showed a modest decrease in total leukocytes with treatment (Figure 29b), while analysis of splenocytes did not show any significant differences in frequencies of T, B, NK, dendritic cells (DCs), or myeloid derived suppressor cells (MDSCs) (Figure 29b). Furthermore, H&E staining of sections of colon and lung tissue in ANT008- and ANT308-treated mice did not show lymphocytic infiltrates or histopathology to suggest auto-immunity (Figures 29c-29d).

272. The study next tested whether the enhanced survival in KPC-Luc tumors treated with VIP-R antagonist and anti-PD-1 is T cell-dependent. The enhanced survival seen with VIP-R antagonist and anti-PD-1 combination therapy was abrogated by depletion of either CD4+ T cells, or CD8+ T cells (Figure 18c). Combination therapy failed to improve survival in both CD4-/- and CD8-/- tumor-bearing mice (Figures 18d and 18e, respectively) indicating the enhanced antitumor response seen with the combination therapy is both CD4+ and CD8+ T cell-dependent.

273. **Increased T cell activation in tumors of mice treated with a combination of VIP-R antagonist and anti-PD-1.** To investigate whether inhibiting VIP-R signaling promotes T cell activation *in vivo,* the study analyzed tumor infiltrating T cells in subcutaneous KPC-Luc tumors for differences in activation markers. While there were no differences in the proportions of Ki67-, IFN-γ- or IL-4-expessing CD4+ or CD8+ T cells, monotherapy with ANT008 or anti-PD-1 significantly altered the levels of PD-1- and/or Tim-3-expressing T cells. ANT008 monotherapy increased levels of PD-1+ Tim-3- T cells in CD4+ and CD8+ T cell subsets, indicating enhanced T cell activation (Figures 19a and 19b). On the other hand, anti-PD-1 monotherapy increased the frequency of PD-1+ Tim-3+ CD4+ T cells, consistent with T cell exhaustion (Figures 19a and 19b). Furthermore, ANT008 or anti-PD-1 monotherapy, as well as combination therapy, significantly decreased the frequency of Tregs within the PDAC tumors (Figures 19c and 19d). These findings are in accordance with the effects of VIP-R antagonist on T regs *in vitro* (Figure 17f). This experiment further confirmed the effect of ANT008 and anti-PD-1 on T cell activation by analyzing mRNA expression in TIL with Nanostring. While there were no genes significantly upregulated in TIL from mice treated with single-agent ANT008 (Figure 19e) or anti-PD-1 (Figure 19f), combination therapy significantly upregulated expression of genes associated with TCR activation and co-stimulation (>4-fold change, FDR<0.1) (Figure 19g). Notably, several markers of T cell activation and co-stimulation such as CD27, CD28, CD247, ICOS, TIGIT and CTLA4, were upregulated in the combination group. Cytokines such as IFN-γ, TNF-α and IL2, that are expressed by activated T cells were also expressed at significantly higher levels in TIL from the combination group (Figure 19h). Overall, the TCR activation and co-stimulatory pathway score was significantly higher in T cells in the tumors of mice treated with combination therapy when compared to control mice treated with scrambled peptide + isotype IgG (Figure 19i).

274. **Combination therapy with VIP-R antagonist and anti-PD-1 induces a tumor specific T cell response and confers protective immunity to tumor re-challenge.** The next experiment evaluated if combination therapy increased the frequencies of specific T cell clones and/or antigen-specific T cells within the tumor. DNA was extracted from tumors followed by amplification of TCRβ genes. Deep sequencing of the TCRβ genes showed increased TCR diversity in tumors from the ANT008 and anti-PD-1 treatment groups (n=4) when compared to control-treated mice (scrambled peptide and isotype IgG; n=4) as shown by Shannon's entropy (Figure 20a), showing more unique TCR responses in the combination group. Upon analyzing the top 50 highest frequency clones in each group, the combination treatment group had the largest numbers of clones shared by at least 2 samples when compared to all other treatment groups (ANT008 and anti-PD-1: 12, control peptide and anti-PD-1: 8, ANT008 and isotype IgG: 6, control peptide and isotype IgG: 6) (Figure 20b). No significant differences were seen in the frequencies of the shared clones in each treatment group (Figure 20c).

275. Next investigated was whether combination therapy with VIP-R antagonist and anti- PD-1 promotes tumor-specific T cell responses. MuLV p15E is an antigen expressed on KPC-Luc, Panc02 and MC38 tumors that is considered a tumor-specific antigen due to lack of expression of MuLVpl5E in C57BL/6 mice. Antigen-specific CD8+ TILs were enumerated with flow cytometry using a MuLV p15E-H2Kb tetramer reagent. It was found that when the tumors were analyzed after 10 days of treatment, tumors from ANT308 and anti-PD-1 treated mice had significantly increased frequencies of tumor-antigen-specific tetramer+ CD8+ T cells compared to control treatment tumors (2.85% versus 0.72%, p<0.01; Figure 20d and Figure 20e). Together, these data show that combination therapy with VIP-R antagonist promotes tumor-antigen-specific T cell responses in KPC-Luc tumors.

276. The KPC-Luc model has been shown to be partially responsive to single-agent anti-PD1 antibody and some mice were observed without evident KPC-Luc tumors after single agent anti-PD1 treatment (Figure 18a). To test whether treatment with the combination of VIP-R antagonists with anti-PD1 antibodies enhanced anti-cancer immunological memory more than treatment with anti-PD1 antibodies alone, tumor-free mice that had no detectable tumor by palpation or BLI following treatment with anti-PD1 monotherapy or the combination of anti-PD1 and either ANT008 or Ant308 were rechallenged with a second inoculation of KPC-Luc. These mice were 80-100 days after initial treatment with either anti-PD1 alone (n=6) or the combination of anti-PD1 antibody with either ANT008 (n=5) or ANT308 (n=3) (Figure 20f). Mice previously treated with the combination of VIP-R antagonist and anti-PD1 had 100% survival after tumor rechallenge versus 0% long-term survival among mice that had been treated initially with single agent anti-PD1 antibody (Figure 20g). These results substantiate the generation of long-term protective anti-cancer immunological memory following treatment with only the combination of VIP-R antagonist peptides and anti-PD-1 and not with anti-PD1 monotherapy. Synergism between ANT008/ANT308 and anti-PD-1 decreased tumor burden and increased intra-tumoral T cell frequency in orthotopic murine PDAC

277. The next experiment tested the efficacy of combination ANT008 and anti-PD-1 therapy in a more clinically-relevant orthotopic KPC-Luc model, in which PDAC cells are implanted directly into the pancreas, recapitulating some elements of the TME in clinical PDAC. Bioluminescent imaging (BLI) confirmed successful engraftment of KPC-Luc cells into the tail of the pancreas in wild type mice 6 to 7 days after implantation. On Day 7 post orthotopic implantation, tumor-bearing mice were randomly assigned to treatment with combinations of control peptide, isotype control antibody, ANT008, or anti-PD-1 MoAb (Figure 21a). Antitumor responses were the greatest in the combination ANT008 plus anti- PD-1 group, such that tumors regressed in 7 of 11 mice (63%) versus 5/9 mice in the group receiving anti-PD-1 monotherapy (55%) and 4/10 mice in the ANT008 monotherapy group (40%) (Figure 21b). In addition, combination of ANT008 and anti-PD-1 had a synergistic effect (Table 4) and led to slower growth of tumors and the lowest tumor burden (measured by the weight of the pancreas on day 25) when compared with control mice (Figure 21c and 21d). Also, one out of 10 mice each in the anti- PD-1 monotherapy and ANT008/anti-PD-1 combination treatment groups were tumor- free as judged by histological analysis of serial H&E-stained tissue sections. The accuracy of the bioluminescent signal (tumor flux) from the tumors was validated by comparing IVIS and MRI images with cross sectional images of H&E-stained paraffin embedded pancreatic tissue, obtained at the time of necropsy (Figure 30a) and by correlating tumor BLI flux with weight of the pancreas (Figure 30b).

278. Finally, immunohistochemical analysis of pancreas at Day 25 of therapy evaluated collagen and infiltrating T cells across tumors from mice in the different treatment groups (Figure 21e). Bands of collagen were visualized in all tumors, consistent with evidence of desmoplastic TME characteristic of human PDAC (Figure 21e, Figure 30c). Tumors from mice that received the combination therapy had significantly higher intra-tumoral levels of CD4+ and CD8+ T cells (Figures 21f and 21g), as well as a higher proportion of Ki67+ CD4+ and CD8+ T cells (Figure 21h and 21i). Furthermore, there was an inverse correlation between T cell density and pancreas weight, such that the smallest tumors in the combination group had higher numbers of proliferating T cells (Figures 30d-30g). These findings indicated that combination therapy with ANT008 and anti-PD-1 not only leads to enhanced T cell activation, but also promotes T cell infiltration in the collagen-rich TME of orthotopic murine PDAC tumors.

279. **Combination therapy with VIP-R antagonist and anti-PD-1 promotes T cell homing into tumors and decreases CXCR4 expression on T cells in tumor draining lymph nodes.** To test whether the enhanced anti-tumor response with combination therapy was due to increased infiltration of T cells into the TME, immunologically naive GFP+ T cells were adoptively transferred from C57Bl/6 EGFP-transgenic mice to wild-type C57BL/6 mice bearing subcutaneously-implanted KPC-Luc tumors, and measured infiltration of GFP+ T cells into the tumor (Figure 22a). Following three days of treatment with ANT308 and/or anti-PD1 MoAb, mice treated with combination therapy had increased numbers of GFP+ T cells within the tumor when compared to all other treatment groups (Figures 31a-31c). Fluorescent microscopy of DAPI stained frozen tumor sections further confirmed significantly increased GFP+ T cell infiltration with combination therapy, that infiltrated the entire tumor (Figure 22b).

280. Preclinical and clinical studies have previously shown that downregulation of CXCR4 promotes mobilization and increases intra-tumoral T cell infiltration in human and murine PDAC tumors. The next experiment tested whether combination therapy with VIP-R antagonist and anti-PD1 modulates expression levels of CXCR4 on T cells. While anti-PD-1 monotherapy increased proportions of Ki67 or CD69 expressing CD4+ and CD8+ T cells, a significant proportion of activated or proliferating cells also expressed CXCR4 (Figure 22c and 22d). On the other hand, treatment with the combination of anti-PD-1 plus VIP-R antagonist increased the proportion of activated CD69+ CD4+ or CD8+ cells with decreased CXCR4 expression (Figure 22c and 22d). As AMD3100, a CXCR4 antagonist has been clinically evaluated as a strategy to mobilize CD8+ T cells into PDAC tumors, the next experiment tested treatment with AMD3100 combined with anti-PD-1 and/or VIP-R antagonists. Combination therapy with VIP-R antagonist and anti-PD-1 was superior to the combination of AMD3100 and anti-PD-1 (Figure 22e), resulting in complete regression of tumors in 20% and 10% of the mice, respectively (p=NS) (Figure 22f). When all three drugs (VIP-R antagonist, anti-PD-1, and AMD3100) were used in combination, survival was not significantly better than in control mice receiving scrambled peptide, isotype matched IgG and PBS (Figure 22e). These findings show that down-regulation of CXCR4, but not complete CXCR4 blockade, can be a superior therapeutic strategy to promote T cell trafficking and cytotoxicity within the PDAC TME.

281. The clinical efficacy of immune checkpoint blockade in pancreatic cancer targeting PD-1 and CTLA-4 has been modest, despite the remarkable success of ICB in treatment of patients with other solid tumor malignancies. Clinical and preclinical studies have shown that the poor responsiveness of PDAC to ICB is largely due to an immunologically cold TME characterized by limited numbers of T cells in the tumor parenchyma, and multiple mechanisms that restrict intra-tumoral T cell activation. Therefore, strategies that 'boost' T cell priming or activation can promote enhanced T cell-mediated anti-tumor responses and improve responsiveness to anti-PD-1 or anti- CTLA-4 ICB. Using preclinical models of murine PDAC the study tested whether treatment with VIP-R antagonists promote activation and proliferation of antitumor T cells and whether these drugs synergize with anti-PD-1.

282. The results herein with selective depletion of CD4+ or CD8+ T cells (Figures 18c-18e) and the generation of cancer-antigen specific immunological memory (Figures 19e and 19g) show that the dominant effect of VIP-R antagonists is via inhibition of paracrine signaling of VIP produced by tumor cells on T cells that express the VIP-R. While knock-out of VPAC2 in Panc02 cells indicated modest direct effects of VIP-signaling on the growth of cancer cells *in vivo,* with mice given injections of VPAC2 knock-out Panc02 tumors having a median survival benefit of 7 days compared to mice given VPAC2 wild type tumors (Figure 24h). In contrast, the combination of VIP-R antagonist and anti-PD-1 synergistically improved T cell-dependent antitumor responses in mice with PDAC, resulting in tumor elimination in up to 40% of treated tumor-bearing mice. Furthermore, since rechallenging the tumor free mice resulted in complete tumor rejection in mice that received the combination of VIP-R antagonist and anti-PD-1, it further emphasized the role of the enhanced adaptive and long-lasting anti-tumor immunity generated in response to inhibiting VIP-R signaling. Recipients of VIP-R antagonist peptide/anti-PD-1 combination therapy had increased homing, activation and proliferation of intra-tumoral CD4+ and CD8+ T cells and marked increases in tumor-antigen-specific T cells within the TME. Induction of a robust T-cell response via CD40-signaling overcomes refractoriness of PDAC tumors to ICB.

283. Exclusion of T cells from the TME is an important characteristic of PDAC tumors that is considered a consequence of a dense desmoplastic stroma containing robust immunosuppressive cells and soluble factors that limit T cell activation. In multiple studies components of the stroma such as cancer associated fibroblasts (and the cytokines and chemokines that they secrete), suppressing effector functions of T cells results in an immunologically "cold" tumor. The role of the CXCR4/CXCL12 axis in T cell infiltration in PDAC tumors is complex. CXCR4 expression facilitates homing of naive T cells to lymph nodes with high CXCL12 levels where priming to tumor antigens can occur. However, CXCR4+ T cells can be "trapped" in the peri-tumoral extracellular matrix by binding to CXCL12 expressed by CAF and tethered onto KRT19. Thus, high expression of CXCR4 is a predictive marker for poor survival in PDAC patients, and treatment with CXCR4 antagonists increase CD8+ T cell infiltration in the TME. Consistent with those data, the current findings of synergy between VIP-R antagonists and anti-PD1 align with modulation of CXCR4 levels. Treatment with anti-PD-1 increases expression of CXCR4 on intra-tumoral T cells while the addition of VIP-R antagonist to anti-PD-1 significantly decreased CXCR4 expression on T cells potentially preventing T cells from being "trapped" in the extracellular matrix. Treatment with a triple combination of VIP-R antagonist, anti-PD-1 and a CXCR4 antagonist resulted in similar tumor growth rates and survival to control mice receiving no drugs (Figure 22e) indicating that down-regulation of CXCR4, but not complete blockade, can be a superior therapeutic strategy to promote T cell trafficking and cytotoxicity within the TME.

284. TIL in mice receiving combination drug therapy expressed significantly higher levels of transcripts associated with TCR signaling and activation, and increased mRNA levels of Th1 cytokines TNF-α, IFN-γ, and IL-2. Furthermore, upregulation of mRNA levels for chemokines CCL2, CCL4 and CCL5 in T cells and the chemokine receptor CXCR6 indicate that intra-tumoral T cells activated by combination VIP-R antagonist/anti-PD1 therapy can promote recruitment of additional T cells from blood into the TME. This is supported by increased accumulation of immunologically naive GFP+ T cells into tumors of mice treated with combination therapy.

285. These data show VIP-R antagonists act by blocking an inhibitory signaling pathway that limits the activation, proliferation, and survival of immune cells. Decreased CREB phosphorylation following in vitro treatment of human T cells with VIP-R antagonists suggests that enhanced NF-κB signaling is responsible for the enhanced T cell activation seen in the murine PDAC models. One of the safety concerns in the use of any ICB is induction of autoimmunity. Notably, treatment of wild type mice with 10 days of daily subcutaneous injections of the ANT008 or ANT308 VIP-R antagonists was not associated with histopathological evidence of autoimmunity, consistent with the absence of auto-immune disease in VIP knockout mice and no evident effect on behavior. Thus, the salubrious effect of VIP-R antagonist treatment on anti-cancer immunity in the PDAC models is likely due to local effects of VIP in the TME where VIP is pathologically overexpressed.

286. This study shows that mouse T cells penetrated deep into orthotopically-implanted tumors treated with the combination of VIP-R antagonists and anti-PD1, apparently crossing dense stromal bands of collagen. While the current study focused on the treatment of PDAC, several other cancers can also be potential targets for VIP-R antagonists. Published studies indicate antitumor activity of VIP-R antagonists in murine models of myeloid leukemia and lymphoma, and other cancers over-express VIP. The focus and conclusions of the current study are orthogonal to previous studies that examined the suppression of autoimmunity by native VIP, or studies that examined the activity of VIP-R antagonists as tumor cytostatic drugs.

287. Finally, why hasn't VIP signaling been previously identified as a targetable ICB pathway? Pharmacologic antagonism of VIP receptors was explored as a strategy to block autocrine signaling of VIP expressed by tumor cells that stimulates tumor growth. The human pancreatic cancer cell line CAPAN-2 expresses VIP receptors and growth is stimulated by VIP. VIP-R antagonists inhibit c-fos mRNA induction by VIP and retard the growth of CAPAN-2 cells in nude mice indicating that VIP receptor antagonists have a tumor-intrinsic cytostatic effect. Preclinical studies explored VIP-R antagonists as cytostatic anti-cancer drugs but did not lead to clinical trials of VIP-R antagonists in humans, or to studies testing the potential of VIP-R antagonists to augment adaptive immunity. While VIP was identified more than four decades ago, the effects of VIP on immunity were studied. While the focus of the current study has been on the effect of anti-PD1 antibody and VIP-R antagonists on anti-tumor T cells, this therapy also has the potential to affect immune-suppressive myeloid cells in the TME and block the generation of tolerogenic dendritic cells and affect antigen presentation by tumor-infiltrating macrophages and dendritic cells in tertiary lymphoid structures within the tumor.

288. The current study shows that VIP-R antagonists represent a novel and tractable approach in the treatment of PDAC. Of note, VIP amino acid sequence is identical between humans and mice, and VIP-R sequences are highly conserved, showing the VIP-R antagonists with immunological activity in tumor-bearing mice can have comparable properties in human patients. In support of this notion, *ex vivo* treatment of human T cells isolated from the blood of PDAC patients with VIP-R antagonists promoted T cell activation, down-regulation of PD-1, Tim-3 and Lag-3 immune checkpoint molecules associated with immunological senescence and decreased the frequencies of regulatory T cells (Fig. 17f). Furthermore, over-expression of VIP may represent a biomarker useful to identify patients with PDAC and other cancers sensitive to the ICB activity of VIP-R antagonists (Fig. 16b and 16e), analogous to the use of PD-L1 staining of cancer as a predictive biomarker for response to anti-PD1 ICB.

### 3. Example 3: Materials and Methods

289. *Cell lines and reagents.* MT5 and KPC-Luc cells were generous gifts from Dr. Tuveson (Cold Spring Harbor Laboratory, Cold Spring Harbor, NY) and Dr. Logsdon (MD Anderson Cancer Center, Houston, Texas), respectively and Panc02 cells were provided by Dr. Pilon-Thomas (H. Lee Moffitt Cancer Center, Tampa, FL). BXPC3, Panc1 and B15F10 were from ATCC (Manassass, VA) and SM1 were from Dr. Antoni Ribas (UCLA, Los Angeles, CA). MT5 and BXPC3 cells were cultured in Roswell Park Memorial Institute (RPMI) medium supplemented with 5% and 10% Fetal Bovine Serum (FBS), respectively, in addition to 10mM L-glutamine and antibiotics. KPC-Luc, Panc02 and Panc1 cells were cultured in Dulbecco's Modified Eagle Medium (DMEM) supplemented with 10% FBS, 10mM L-glutamine and antibiotics. Synthego (Redwood City, CA) provided CRISPR/Cas9 VPAC2 knockout pools of Panc02 cell line. Single clones of VPAC2 KO Panc02 cells were selected by limit-dilution and expanded in same media as wild type Panc02 cells. B16F10 cells were cultured in DMEM with L-glutamine and sodium pyruvate, supplemented with 10% FBS, 100ug/mL streptomycin, and 1500mg/L sodium bicarbonate. The human pancreatic cancer associate stellate (PSC) cell line h-iPSC-PDAC-1 was generated and maintained as previously described.

290. Pharmaceutical grade murine antibodies to PD-1 (Clone RMP1-14) or isotype control (Clone 2A3) were purchased from BioXcell (West Lebanon, NH). Pharmaceutical grade AMD3100 was purchased from Selleck Chemicals LLC (Houston, Texas). Scrambled peptide, ANT008 and ANT308 were purchased from RS synthesis (Louisville, KY) at a purity of >95%. 200µM stock solutions were prepared in DEPC-Treated pyrogen free water from IBI Scientific (Dubuque, IA) and stored at -80°C until use. Creative Biolabs (Shirley, NY) performed in-silico modeling to predict the binding affinity of ANT008 and ANT308 to VPAC1 and VPAC2 receptors.

291. *Patients and samples.* Primary human PSC/CAF were isolated from resected pancreatic tumors in accordance with an Institutional Review Board (IRB)-approved protocol at the Winship Cancer Institute of Emory University on de-identified tumor tissues. Briefly, freshly resected pancreatic tissue was dissected into 1mm3 pieces, plated in a culture dish and incubated with DMEM + 10% FBS+ antibiotics for 2-3 weeks until a coalesce was observed. Cell free supernatant was then collected to quantify levels of VIP via VIP specific enzyme immunoassay as described below. Blood samples from consented pancreatic cancer patients and healthy volunteers were collected in EDTA coated vacutainer tubes from Becton Dickinson and Company (Franklin Lakes, NJ). Plasma was isolated as previously described, where in the EDTA vacutainer tubes were centrifuges at 2000xg for 15 minutes and stored at -80°C until used for analysis of levels of VIP. Patient demographics is described Table 3. Peripheral blood mononuclear cells (PBMCs) were isolated from consented patients with PDAC or healthy volunteers (IRB 00087397 and IRB 00046063, respectively) by ficoll-hypaque density-gradient centrifugation as previously described and cryopreserved in CryoStor cell cryopreservation media CS10 (STEMCELL Technologies, Vancouver, Canada), until required for T cell isolation and *ex-vivo* expansion.

292. *Antibodies.* For western blot analysis of VPAC1, VPAC2, PD1 and CTLA4 expression, anti-VPAC1 (1:500), anti-VPAC2 (1:500) monoclonal antibodies from Sigma Aldrich (St. Louis, MO) and anti-PD1 (1:1000) and anti-CTLA-4 (1:500) monoclonal antibodies from Cell Signaling Technology (Danvers, MA) were used. For IF, anti-VIP monoclonal antibody at 1:50 from OriGene (Clone OT15B5) and anti-CK18 monoclonal antibody at 1:400 from Abcam (Clone EP1580Y) was used. Details of fluorochrome conjugated antibodies for flow cytometric analysis are provided Table 4. Fixable Aqua live/dead stain from Thermo Fisher Scientific (Waltham, MA) was used to detect and gate for live cells in all samples analyzed via flow cytometric analysis. To identify tumor specific T cells, APC conjugated MHC Tetramer H-2kb MuLV p15E from MBL International Corporation (Woburn, MA) was used.

293. *Mice.* All experimental procedures were approved by the Institutional Animal Care and Use Committee (IACUC) at Emory University. Female or male C57BL/6, CD4KO (B6.129S2-Cd4^{tm1Mak}/J) and CD8KO (B6.129S2-Cd8a^{tm1Mak}/J) were obtained from the Jackson Laboratory (Bar Harbor, ME) at 6-8 weeks of age and housed in micro-isolator cages. Transgenic mice expressing enhanced green fluorescence protein (EGFP) from on a C57BL/6 background (strain designation: C57BL/6-Tg(Act-EGFP)C14-Y01-FM131 Osb) were a gift from Dr. Masaru Okabe (Osaka University, Osaka, Japan) and were bred and maintained at the Emory University Animal Care Facility (Atlanta, GA) Experiments were performed when mice were 8-10 weeks old and animal care and maintenance was provided following The Guide for Care and Use of Laboratory Animals (National Research Council). For CD4+ and/or CD8+ T cell depletion studies, antibody to deplete CD4+ T cells (Clone GK1.5) or CD8+ T cells (Clone 2.43) from BioXcell (West Lebanon, NH) were injected intraperitoneally at 200µg per mouse on days -3, -1, +1, +3, +7 with respect to tumor implantation and twice a week until the completion of the experiment.

294. *Preparation of single cell suspensions.* Harvested tumor tissues or tumor draining lymph nodes (TDLNs) from murine KPC-Luc or Panc02 bearing mice were cut into small pieces using a scalpel and treated triple enzyme digestion cocktail containing 10mg/ml collagenase, 1 mg/ml hyaluronidase and 200 mg/ml DNase in HBSS at 37°C for 20 minutes (TDLNs) or 1 hour (tumors) and vortexed every 15 minutes. The tissue pieces were then mechanically dissociated, washed, centrifuged, and passed through a 70µm nylon mesh filter to obtain a single cell suspension for staining and analysis via flow cytometry. For spleen samples, the single cell suspension obtained by mechanical dissociation, was passed through 70µm nylon mesh filter and depleted of red blood cells using ammonium chloride lysis buffer and washed twice. Blood samples were collected in tubes with 0.1ml diluted heparin (500 USP units/ml) followed by red blood cell depletion using ammonium chloride lysis buffer and washed twice.

295. *VIP specific enzyme immunoassay.* 3 x 10⁵ B16F10, KPC-Luc, MT5, Panc02, BXPC3 and Panc1 cells were cultured in 6 well plates with 3 ml of respective media. Cell free media was collected 24 hours after culture and stored at -80°C until tested for VIP levels. Peripheral blood collected from consented PDAC patients and healthy volunteers in EDTA tubes were centrifuged at 2000g for 10 minutes to isolate plasma that was also stored in -80°C until analysis. VIP levels in cell free supernatant and plasma were quantified via VIP specific enzyme immunoassay (EIA) kit following manufacturer's protocol (RayBiotech, Peachtree Corners, Georgia). Absorbance was measured at 450nm using synergy plate reader (BioTek, Winooski, Vermont), a standard curve generated and used to determine the concentration of VIP in the samples.

296. *Cell viability assay.* To determine the effect of Ant-08 on the growth of PDAC cell lines *in-vitro,* MT5, KPC- Luc, Panc02, BXPC3, Capan-02 and cells were plated on a 96 well plate and treated with varying concentrations of Ant-08 (0-5µM) in respective media, for 24-72 hours. Cell viability was assessed using cell proliferation kit I from Roche (Basel, Switzerland), following the manufacturer's instructions. Briefly, at the end of the incubation time, 10ul of 0.5mg/ml MTT labeling reagent was added and the plate was incubated in a humidified C02 incubator for 4 h. This was followed by incubation with solubilization buffer overnight and reading the absorbance at 570nm. Percentage of cell viability with respect to control (0 µM ANT008) was plotted. Similar assay was performed to for wild type and VPAC2 KO Panc02 cells following treatment with Ant-08 or Ant-308 at 3µm for 72 hours.

297. *In vivo efficacy studies.* For the subcutaneous model, 5x10⁵ KPC-Luc cells were injected subcutaneously near the right flank of female or male C57BL/6 mice. For the MT5 or Panc02 models, 5x10⁵ were injected subcutaneously near the right flank of female C57BL/6 mice. For the orthotopic KPC-Luc model, mice were anesthetized and the KPC-Luc cells were suspended in Matrigel and injected in the tail of the pancreas following laparotomy. 6-7 days after tumor implantation mice were randomized into 4 treatment groups and treated with VIP-R antagonist and/or anti-PD-1. While scram+IgG control mice received scrambled peptide and isotype IgG, the VIP-R antagonist, anti-PD-1 and VIP-R antagonist and anti-PD-1 groups received VIP-R antagonist and IgG; scrambled peptide and anti-PD-1; and VIP-R antagonist and anti-PD-1, respectively. The treatment regimen involved administering 10µg of scrambled or VIP-R antagonist: ANT008 or ANT308, subcutaneously every day and 200µg of IgG or anti-PD-1 intraperitoneally once every three days, for a total of 10 days. In experiments involving male mice, 20µg of VIP-R antagonist was used due to the higher body weight when compared to female mice. In experiments were mice received AMD3100, 5mg/kg of AMD3100 in PBS was administered subcutaneously every day for 10 days. In the KPC-Luc models, the tumor growth rate was plotted using the tumor flux measurements quantified using IVIS bioluminescent imaging. In the orthotopic KPC-Luc model, one mouse per group with biggest non-ulcerated tumor was sacrificed on day 28 and placed supine within a custom- built cradle and imaged with 9.4 Tesla MRI scanner. Rapid acquisition with relaxation enhancement (RARE) imaging sequence was used with a slice thickness of 0.4mm and a total of 40 slices per mouse (RARE factor = 8, Average = 25 and Field of view = 30.7X 30.7 mm²). In experiments with subcutaneous tumors, Vernier calipers were used to measure the tumor dimensions and the tumor volume was calculated using the formula: tumor volume = 1/2(length x width x height).

298. *Nanostring analysis of Tumor infiltrating T cells.* Singlet cell suspensions of tumors were subjected to magnetic T cell isolation using EasySep^{™} Mouse CD90.2 Positive Selection kit II from STEMCELL technologies (Vancouver, Canada). Qiagen RNeasy Micro kit was used to extract RNA, and quantity and quality was assessed using the Nanodrop and Agilent 2100. RNA analyzed using the nCounter Metabolic Pathways Panel (Nanostring Technologies, Seattle, WA).

299. *TCR deep sequencing* Subcutaneously implanted KPC-Luc tumors treated with ANT008 and/or anti-PD-1 were harvested on day 21 after tumor implantation. Qiagen RNeasy Micro kit was used to extract RNA, and quantity and quality was assessed using the Nanodrop and Agilent 2100. Sequencing of TCR-β CDR3 V and J sequences performed by Adaptive Biotechnologies (Seattle, WA).

300. *Human T cell activation and expansion.* One day before in vitro T cell expansion, cryopreserved PBMCs were thawed and rested by culturing at 37°C in a 5% CO₂ humidified incubator overnight in complete RPMI media supplemented with 10% FBS, 100U/mL penicillin and 100ug/mL streptomycin, MEM nonessential amino acids, 20mM N-2-hydroxyethylpiperazine-N-2-ethane sulfonic acid (HEPES), and 50uM 2-mercaptoethanol. T cells were isolated via negative magnetic isolation using EasySep Human T cell isolation kit from STEMCELL Technologies (Vancouver, Canada). 50,000 to 1 million T cells were seeded on each well of a 96 well plate that was coated with 10ug/ml of Ultra-LEAF Purified anti-human CD3 antibody (clone: UCHT1) from Biolegend (San Diego, CA) and cultured with 30U/ml recombinant human IL-2 (Peprotech, Inc., Cranbury, NJ) with or without 3µM scrambled peptide, ANT008 or ANT308. Cells were counted using Trypan blue dye and phenotyped via flow cytometry on day 9. Every three or four days, the cells were split to 6 or 48 well or plates coated with anti-human CD3.

301. *Peptides.* ANT008 has the peptide sequence of VIPhyb modified by replacing serine at amino acid position 25 with leucine. ANT308 is a further modification of ANT008 sequence in which the aspartic acid and asparagine residues at positions 8 and 9 are replaced with serine and aspartic acid, respectively. (ANT008: -60.17 kcal/mol free binding energy for VPAC1 and -51.07 for VPAC2, ANT308: -71.56 for VPAC1 and -56.27 for VPAC2 as per *in silico* analysis).

302. *Adoptive transfer of GFP+ T cells* One million KPC-Luc cells were subcutaneously implanted into C57BL/6 mice. On day 15 after tumor implantation, spleen from EGFP transgenic mice were harvested and processed as above and T cells were magnetically isolated using Pan T cell isolation kit II from Miltenyi Biotech (Auburn, CA). 10 million GFP+ T cells were then injected intravenously to the KPC-Luc tumor bearing C57BL/6 mice. Mice were randomized into 4 treatment groups: scram+IgG, ANT308+IgG, scram+aPD-1, ANT308+aPD-1 and treated subcutaneously with 10ug of scrambled peptide/ANT308 on day 1, 2 and 3 and/or 200ug IgG or anti-PD-1 intraperitoneally on day 1 after T cell transfer. Mice were sacrificed on day 18 after tumor implantation and harvested tumors were flash frozen in OCT compound (Sakura Finetek, Torrance, CA) for embedding and cryosectioning. Tissue slides were then stained with 2ug/ml Hoescht 33342 (Abcam, Cambridge, MA) and imaged on a BZ-X810 epifluorescence microscope (Keyence Corp, Itasca, IL) using DAPI (359nm/461nm) and GFP (488nm/510nm) filter sets (Chroma Technology Corp, Bellows Falls, VT). Plan Fluor 40X 1.3 NA oil immersion objective from Nikon Inc. (Melville, NY) were used with 100% of the light from excitation source reaching the sample, 1/1.2 second camera exposure and image aspect ratio of 1920 x 1440. Multiple images were acquired spanning the entire tissue section, which were then stitched using Keyence' image stitcher function and merged into a composite image using Fiji image analysis software.

303. *Immunofluorescence.* Paraffin-embedded PDAC tissues and adjacent normal tissues were deparaffinized, hydrated and antigen retrieved by boiling with 1XTrilogy for 15 minutes (Cell Marque- Trilogy Buffer) and subsequent washing with distilled water. Permeabilization was performed using 0.3% Triton-X-100, followed by blocking step with eBioscience^{™} low protein blocking buffer for 1 hour at room temperature. Mouse anti-VIP (OriGene Technologies, Inc. Rockville, MD) diluted at 1:50 and rabbit anti-cytokeratin-19 (Abcam, Cambridge, MA) diluted at 1:400 was applied and incubated overnight at 4 °C. Secondary antibodies for anti-mouse IgG (H+L) conjugated with Alexa Fluor 647 and anti-rabbit IgG (H+L) conjugated with TRITC was applied and incubated for 1 hour at room temperature. Tissue slides were then stained with 2ug/ml Hoescht 33342 (Abcam, Cambridge, MA) and imaged on a BZ-X810 epifluorescence microscope (Keyence Corp, Itasca, IL) using DAPI (359nm/461nm) and Alexa Fluor 647 (594nm/633nm) and TRITC (579nm/599nm) filter sets (Chroma Technology Corp, Bellows Falls, VT). Plan Fluor 40X 1.3 NA oil immersion objective from Nikon Inc. (Melville, NY) were used with 100% of the light from excitation source reaching the sample, 1/1.7 second camera exposure and image aspect ratio of 1920 x 1440. Multiple images were acquired spanning the entire tissue section, which were then stitched using Keyence' image stitcher function and merged into a composite image using Fiji image analysis software.

304. *Histology.* All tissues for histology were fixed at 4°C for 2-3 days in 4% paraformaldehyde in PBS, embedded in paraffin and cut into 5-µm thick sections. Slides were deparaffinized with EZ-Prep (# 05279771001, Ventana, Tucson, AZ) and then were antigen retrieved for 64 minutes with CC1 reagent (#950-500, Ventana, Tucson, AZ). Mouse anti-VPAC1 and anti-VPAC2 from Sigma Aldrich (St. Louis, MO) diluted at 1:500 or rabbit anti-cytokeratin-19 (Abcam, Cambridge, MA) diluted at 1:500 were applied and incubated for 40 minutes. DISCOVERY OmniMap anti-mouse or anti-rabbit HRP was applied and incubated for 12 minutes. The detection was completed in combination with DISCOVERY ChromoMap DAB kit as per manufacturer's recommendations. Pancreas harvested from mice with orthotopically implanted KPC-Luc tumors or colon, liver and inflated lungs harvested from naive C57BL/6 mice receiving ANT008 or ANT308 were formalin-fixed and paraffin embedded before being stained with H&E (Leica 560 MX, Wetzlar, Germany) or Masson's Trichrome (Polyscientific Inc., Bay Shore, NY). All slides were dehydrated, cover-slipped and scanned on Hamamatsu Nanozoomer 2.0 HT at 40x and reviewed by a pathologist.

305. *Multiplex Immunohistochemistry* Multiplex IHC staining was performed on the Roche Ventana DISCOVERY automated immunostainer from Ventana Medical Systems (Tucson, AZ). The Ventana DISCOVERY uses a sequential staining procedure with a denaturation step between each staining sequence. Slides were deparaffinized with EZ-Prep (# 05279771001, Ventana) and then were antigen retrieved for 64 minutes with CC1 reagent (#950-500, Ventana). Cell Conditioning 2 buffer (CC2, #950-123, Ventana) was used for deactivation of the bound primary antibody and secondary anti-horse radish peroxide between each staining sequence. Four pre-diluted primary antibodies were sequentially applied in the following order using the indicated chromogenic detection: rabbit anti-Ki67 (#ab833 from Abcam, Cambridge, MA) with Opal 570, rabbit anti-Foxp3 (#NB100-39002 from Novus Biologicals, Littleton, CO) with Opal 480, rabbit monoclonal CD4 (#ab133616 from Abcam) with Opal 620 and rabbit anti-CD8 (#ab4055 from Abcam) with Opal 690 at dilutions of 1:300, 1:500, 1:500 and 1:250, respectively. Slides were cover slipped with VECTASHIELD Antifade mounting medium (Vector Laboratories) and stained slides were stored at 4°C. Numbers of CD4+, CD8+, Ki67+CD4+ and Ki67+CD8+ T cells were quantified using QuPath, an open source software for digital pathology image analysis.

306. *Statistics.* For survival data, Kaplan-Meier method with log-rank tests was performed to determine statistical differences between treatment groups. For comparison of tumor volume and differences in immune cell subsets where 4 treatment groups were compared, one-way ANOVA followed by Dunnett's multiple comparison post-hoc test was used. For data from *ex-vivo* expansion of T cells from healthy volunteers, repeated measures ANOVA was used followed by Dunnett's post-hoc test. For data from expansion of T cells from PDAC patients were T cell phenotype and characteristics between scrambled or ANT008 treated were compared, pair wise student t-test was utilized. In Figures 30d-30g, R-squared values were generated from a linear regression model. P values less than 0.05 were considered significant. All statistical analyses were conducted using GraphPad Prism software, version 8.2 (GraphPad Software, Inc., San Diego, CA, USA).

**Table 3. Table depicting demographics of PDAC patients tested for serum VIP levels.**

| Patient # | Age | Gender | Race | Ethnicity |
|---|---|---|---|---|
| 1 | 71 | Female | Caucasian | Non-Hispanic |
| 2 | 66 | Male | Caucasian | Non-Hispanic |
| 3 | 61 | Female | African American | Non-Hispanic |
| 4 | 75 | Male | Caucasian | Hispanic |
| 5 | 73 | Male | Caucasian | Non-Hispanic |
| 6 | 76 | Male | Caucasian | Non-Hispanic |
| 7 | 59 | Female | Caucasian | Non-Hispanic |
| 8 | 47 | Female | African American | Non-Hispanic |
| 9 | 69 | Male | Caucasian | Non-Hispanic |
| 10 | 69 | Male | Caucasian | Non-Hispanic |
| 11 | 64 | Male | African American | Non-Hispanic |
| 12 | 71 | Female | Caucasian | Non-Hispanic |
| 13 | 59 | Female | African American | Non-Hispanic |
| 14 | 69 | Female | Caucasian | Non-Hispanic |
| 15 | 71 | Female | Caucasian | Non-Hispanic |
| 16 | 67 | Male | Caucasian | Non-Hispanic |
| 17 | 69 | Male | Caucasian | Non-Hispanic |
| 18 | 52 | Male | African American | Non-Hispanic |
| 19 | 63 | Male | Caucasian | Non-Hispanic |

**Table 4. Details of fluorescent conjugated antibodies used in this study.**

| **Human/mouse** | **Fluorochrome** | **Target** | **Clone** | **Vendor** |
|---|---|---|---|---|
| **Human** | PE-CF594 | CD3 | UCHT1 | BD |
| | APC-Cy7 | CD4 | RPA-T4 | BD |
| | Alexa Fluor 700 | CD8 | RPA-T8 | BD |
| | BV650 | CD69 | FN50 | Biolegend |
| | PE-eFluor 610 | CXCR4 | 12G5 | eBioscience |
| | FITC | PD-1 | EH12.2H7 | Biolegend |
| | PE-Cy7 | Tim-3 | 7D3 | BD |
| | APC | Lag-3 | 7H2C65 | Biolegend |
| | BV605 | CD25 | 2A3 | BD |
| | PerCPcy 5.5 | FoxP3 | 236A/E7 | BD |
| **Mouse** | BV480 | CD45 | 30-F11 | BD |
| | FITC | CD3 | 17A2 | BD |
| | Alexa Fluor 700 | CD4 | RM4-5 | BD |
| | PerCP cy5.5 | CD8 | 53-6.7 | BD |
| | PE-cy7 | Ki67 | 16A8 | Biolegend |
| | APC-Cy7 | CD25 | 3C7 | Biolegend |
| | PE | FoxP3 | FJK-16s | eBioscience |
| | BV785 | PD-1 | 29F.1A12 | Biolegend |
| | PE | Tim-3 | B8.2C12 | Biolegend |
| **Human and Mouse** | Alexa(R) 647 | P-CREB (S133) | 87G3 | Cell Signaling |

**Table 5: VPAC Binding Affinity**

| Peptide Name | amino acid sequence | SEQ ID NO | Docking scores from in silico screening (Creative Biolabs) | | In vitro daily addition of 3 uM peptide | Leukemia-bearing mice with 1 x 10E6 C1498 i.v. on day 0 10 ug peptide administered s.c. daily on days 6-12 | | |
|---|---|---|---|---|---|---|---|---|
| | | | VPAC1-R | VPAC2-R | luc+ T cell proliferation | Percentage alive day 60 | Median survival time (days) | number of animals p value c/w SRAM1 |
| VIP | HSDAVFTDNYTRLRKQ MAVKKYLNSILN | SEQ ID NO: 2 | -65.8 | -52.61 | 77% + 13% (0.1 uM) | 0% | 30 days | n=10, p=0.1001 |
| VIPhyb | KPRRPYTDNYTRLRKQ MAVKKYLNSILN | SEQ ID NO: 1 | -60.62 | -51.007 | 197% + 38% (1 uM) | 5% | 34 days | n=20, p<0.0001 |
| ANT005 | KPRRPYTDNCTRLRKQ MAVKKYLNSILN | SEQ ID NO: 4 | -64.27 | -64.45 | 187% + 1% (3 uM) | | | |
| ANT008 | KPRRPYTDNYTRLRKQ MAVKKYLNLILN | SEQ ID NO: 5 | -60.17 | -53.978 | 185% + 24% (1 uM) | 16% | 35 days | n=25, p<0.0001 |
| ANT058 | KPRRPYADNYTRLRKQ MAVNKYLNLILN | SEQ ID NO: 6 | -76.3 | -60.602 | | 30% | 35 days | n=20, p<0.0001 |
| ANT105 | KPRRPYAVNYTRLRKQI AVKKYLMSILN | SEQ ID NO: 7 | -68.19 | -55.355 | 121% + 6% (3 uM) | | | |
| ANT107 | KPRRPYAVNYTRLRKQ MAVNKYLMSILN | SEQ ID NO: 8 | -73.12 | -52.346 | | 30% | 38 days | n=10, p=0.0025 |
| ANT114 | KPRRPYADNCTRLRKQI AVNKKYLNSILN | SEQ ID NO: 9 | -73.57 | -54.216 | | 20% | 34 days | n=5, p=0.0018 |
| ANT195 | KPRRPYTVNYTSLRKQI AVKKYLMLILN | SEQ ID NO: 10 | -70.44 | -71.439 | 161% + 23% (3 uM) | 40% | 38 days | n=25, p<0.0001 |
| ANT197 | KPRRPYTDNCTSLRKQI AVNKYLNLILN | SEQ ID NO: 11 | -63.6 | -69.074 | 161% + 13% (3 uM) | 35% | 37 days | n=20, p<0.0001 |
| ANT202 | KPRRPYAVNCTSLRKQI AVNKYLNSILN | SEQ ID NO: 12 | -56.35 | -67.02 | 171% + 8% (3 uM) | | | |
| ANT203 | KPRRPYAVNCTSLRKQI AVKKYLMSILN | SEQ ID NO: 13 | -75.34 | -50.942 | 185% + 8% (3 uM) | 25% | 34 days | n=20, p<0.0001 |
| ANT219 | KKPRRPYTVNCTSLRK QIAVKKYLMLILN | SEQ ID NO: 14 | -69.37 | -69.116 | 189% + 8% (3 uM) | 20% | 35 days | n=10, p=0.0002 |
| ANT300 | KPRRPYTSDYTRLRKQ MAVKKYLNSILN | SEQ ID NO: 15 | -72.36 | -61.60 | 216% + 20% (0.3 uM) | 33.30% | 47 days | n=15, p<0.0001 |
| ANT308 | KPRRPYTSDYTRLRKQ MAVKKYLNLILN | SEQ ID NO: 16 | -71.56 | -56.27 | 109% + 8% (3 uM) | 40% | 34 days | n=15, p=0.0009 |
| SCRAM1 | HSDAVFINTKLDKLNTR LVSAQNYMKYR | SEQ ID NO: 17 | -42.84 | -37.102 | | 0% | 28 days | n=30, n=NS |
| SCRAM2 | KPRRPYINTKLDKLNTR LVSAQNYMKYR | SEQ ID NO: 18 | -43.69 | -37.423 | 108.74% | 0% | | |
| | | | | | | | | |
| CONTROL S- NO PEPTIDE | | | | | | | | |
| PBS | Wild-type mice | | n/a | | n/a | 0% | 27 days | n=30, n=NS |
| PBS | VIP KO-type mice | | n/a | | n/a | 10% | | |

| | | | | | | | | |
|---|---|---|---|---|---|---|---|---|
| Note:- ANT 300 and ANT 308 have SD at aa positions 9,9 according to the sequence of PHI. ANT 308 has the resides S,D at aa positions 8,9 from PHI plus L at aa position 25 from ANT 008. | | | | | | | | |

### References:

1. Kabacaoglu, D., et al., Immune Checkpoint Inhibition for Pancreatic Ductal Adenocarcinoma: Current Limitations and Future Options. Front Immunol, 2018. 9: p. 1878.
2. Saung, M.T. and L. Zheng, Current Standards of Chemotherapy for Pancreatic Cancer. Clin Ther, 2017. 39(11): p. 2125-2134.
3. Le, D.T., et al., Mismatch repair deficiency predicts response of solid tumors to PD-1 blockade. Science, 2017. 357(6349): p. 409-413.
4. Young, K., et al., Immunotherapy and pancreatic cancer: unique challenges and potential opportunities. Ther Adv Med Oncol, 2018. 10: p. 1758835918816281.
5. Hosein, A.N., R.A. Brekken, and A. Maitra, Pancreatic cancer stroma: an update on therapeutic targeting strategies. Nat Rev Gastroenterol Hepatol, 2020. 17(8): p. 487-505.
6. Lin, J.H. and R.H. Vonderheide, Dendritic cell dysfunction precedes tumor formation during pancreatic oncogenesis. Cancer Research, 2020. 80(16).
7. Johnson, B.A., 3rd, et al., Strategies for Increasing Pancreatic Tumor Immunogenicity. Clin Cancer Res, 2017. 23(7): p. 1656-1669.
8. Vonderheide, R.H., The Immune Revolution: A Case for Priming, Not Checkpoint. Cancer Cell, 2018. 33(4): p. 563-569.
9. Khalil, M. and R.H. Vonderheide, Anti-CD40 agonist antibodies: preclinical and clinical experience. Update Cancer Ther, 2007. 2(2): p. 61-65.
10. Vonderheide, R.H., CD40 Agonist Antibodies in Cancer Immunotherapy. Annu Rev Med, 2020. 71: p. 47-58.
11. O'Hara, M.H., O'Reilly, E.M., Wolff, R.A., Wainberg, Z.A., Ko,A.H., Rahma, O.E., Fisher, G.A., Lyman, J.P, Cabanski,C.R., Karakunnel, J.J., Gherardini, P.F., Kitch, L.J., Bucktrout, S., Christopher, E., Mick,R., Chen,R., Trifan, O.C., Salvador, L., O'Donnell-Tormey,J. and Vonderheide, R.H., Gemcitabine (Gem) and nab-paclitaxel (NP) ± nivolumab (nivo) ± CD40 agonistic monoclonal antibody APX005M (sotigalimab), in patients (Pts) with untreated metastatic pancreatic adenocarcinoma (mPDAC): Phase (Ph) 2 final results. Journal of Clinical Oncology 2021.
12. Delgado, M. and D. Ganea, Vasoactive intestinal peptide: a neuropeptide with pleiotropic immune functions. Amino Acids, 2013. 45(1): p. 25-39.
13. Fernandez-Martinez, A.B., et al., Multifunctional role of VIP in prostate cancer progression in a xenograft model: suppression by curcumin and COX-2 inhibitor NS-398. Peptides, 2009. 30(12): p. 2357-64.
14. Moody, T.W. and 1. Gozes, Vasoactive intestinal peptide receptors: a molecular target in breast and lung cancer. Curr Pharm Des, 2007. 13(11): p. 1099-104.
15. Moody, T.W., et al., VIP receptor antagonists and chemotherapeutic drugs inhibit the growth of breast cancer cells. Breast Cancer Res Treat, 2001. 68(1): p. 55-64.
16. Moody, T.W., B. Nuche-Berenguer, and R.T. Jensen, Vasoactive intestinal peptide/pituitary adenylate cyclase activating polypeptide, and their receptors and cancer. Curr Opin Endocrinol Diabetes Obes, 2016. 23(1): p. 38-47.
17. Moody, T.W., et al., A Vasoactive-Intestinal-Peptide Antagonist Inhibits Nonsmall Cell Lung-Cancer Growth. Proceedings of the National Academy of Sciences of the United States of America, 1993. 90(10): p. 4345-4349.
18. Anderson, P. and E. Gonzalez-Rey, Vasoactive intestinal peptide induces cell cycle arrest and regulatory functions in human T cells at multiple levels. Mol Cell Biol, 2010. 30(10): p. 2537-51.
19. Gonzalez-Rey, E. and M. Delgado, Vasoactive intestinal peptide and regulatory T-cell induction: a new mechanism and therapeutic potential for immune homeostasis. Trends Mol Med, 2007. 13(6): p. 241-51.
20. Delgado, M. and D. Ganea, Cutting edge: is vasoactive intestinal peptide a type 2 cytokine? J Immunol, 2001. 166(5): p. 2907-12.
21. Petersen, C.T., J.M. Li, and E.K. Waller, Inhibition of CREB signaling through antagonism of vasoactive intestinal peptide enhances CD8 T cell function in a murine model of AML. Journal of Immunology, 2016. 196**.**
22. Petersen, C.T., J.M. Li, and E.K. Waller, Administration of a vasoactive intestinal peptide antagonist enhances the autologous anti-leukemia T cell response in murine models of acute leukemia. Oncoimmunology, 2017. 6(5): p. e1304336.
23. Li, J.M., et al., VIPhyb, an antagonist of vasoactive intestinal peptide receptor, enhances cellular antiviral immunity in murine cytomegalovirus infected mice. PLoS One, 2013. 8(5): p. e63381.
24. Li, J.M., et al., Pharmacological inhibition of VIP signaling enhances antiviral immunity and improves survival in murine cytomegalovirus-infected allogeneic bone marrow transplant recipients. Blood, 2013. 121(12): p. 2347-51.
25. Zia, H., et al., Breast cancer growth is inhibited by vasoactive intestinal peptide (VIP) hybrid, a synthetic VIP receptor antagonist. Cancer Res, 1996. 56(15): p. 3486-9.
26. Reubi, J.C., et al., Vasoactive intestinal peptide/pituitary adenylate cyclase-activating peptide receptor subtypes in human tumors and their tissues of origin. Cancer Res, 2000. 60(11): p. 3105-12.
27. Schulz, S., et al., Immunocytochemical identification of VPAC1, VPAC2, and PAC1 receptors in normal and neoplastic human tissues with subtype-specific antibodies. Clin Cancer Res, 2004. 10(24): p. 8235-42.
28. Schulz, S., et al., VPAC2 receptor expression in human normal and neoplastic tissues: evaluation of the novel MAB SP235. Endocr Connect, 2015. 4(1): p. 18-26.
29. Buchbinder, E.I. and A. Desai, CTLA-4 and PD-1 Pathways: Similarities, Differences, and Implications of Their Inhibition. Am J Clin Oncol, 2016. 39(1): p. 98-106.
30. Jin, H.T., et al., Cooperation of Tim-3 and PD-1 in CD8 T-cell exhaustion during chronic viral infection. Proc Natl Acad Sci U S A, 2010. 107(33): p. 14733-8.
31. Dong, Y., et al., CD4(+) T cell exhaustion revealed by high PD-1 and LAG-3 expression and the loss of helper T cell function in chronic hepatitis B. BMC Immunol, 2019. 20(1): p. 27.
32. Zarour, H.M., Reversing T-cell Dysfunction and Exhaustion in Cancer. Clin Cancer Res, 2016. 22(8): p. 1856-64.
33. Bronte, V., et al., Effective genetic vaccination with a widely shared endogenous retroviral tumor antigen requires CD40 stimulation during tumor rejection phase. J Immunol, 2003. 171(12): p. 6396-405.
34. Burrack, A.L., et al., Combination PD-1 and PD-L1 Blockade Promotes Durable Neoantigen-Specific T Cell-Mediated Immunity in Pancreatic Ductal Adenocarcinoma. Cell Rep, 2019. 28(8): p. 2140-2155 e6.
35. Biasci, D., et al., CXCR4 inhibition in human pancreatic and colorectal cancers induces an integrated immune response. Proc Natl Acad Sci U S A, 2020. 117(46): p. 28960-28970.
36. Seo, Y.D., et al., Mobilization of CD8(+) T Cells via CXCR4 Blockade Facilitates PD-1 Checkpoint Therapy in Human Pancreatic Cancer. Clin Cancer Res, 2019. 25(13): p. 3934-3945.
37. Fearon DT, J.T., AMD3100/Plerixafor overcomes immune inhibition by the CXCL12-KRT19 coating on pancreatic and colorectal cancer cells. . Br J Cancer, 2021.
38. Taube, J.M., et al., Association of PD-1, PD-1 ligands, and other features of the tumor immune microenvironment with response to anti-PD-1 therapy. Clin Cancer Res, 2014. 20(19): p. 5064-74.
39. Royal, R.E., et al., Phase 2 trial of single agent Ipilimumab (anti-CTLA-4) for locally advanced or metastatic pancreatic adenocarcinoma. J Immunother, 2010. 33(8): p. 828-33.
40. Saka, D., et al., Mechanisms of T-Cell Exhaustion in Pancreatic Cancer. Cancers (Basel), 2020. 12(8).
41. Balachandran, V.P., G.L. Beatty, and S.K. Dougan, Broadening the Impact of Immunotherapy to Pancreatic Cancer: Challenges and Opportunities. Gastroenterology, 2019. 156(7): p. 2056-2072.
42. Martinez-Bosch, N., J. Vinaixa, and P. Navarro, Immune Evasion in Pancreatic Cancer: From Mechanisms to Therapy. Cancers (Basel), 2018. 10(1).
43. Watt, J. and H.M. Kocher, The desmoplastic stroma of pancreatic cancer is a barrier to immune cell infiltration. Oncoimmunology, 2013. 2(12).
44. Gorchs, L., et al., Human Pancreatic Carcinoma-Associated Fibroblasts Promote Expression of Co-inhibitory Markers on CD4(+) and CD8(+) T-Cells. Front Immunol, 2019. 10: p. 847.
45. Barrett, R.L. and E. Pure, Cancer-associated fibroblasts an their influence on tumor immunity and immunotherapy. Elife, 2020. 9**.**
46. Ware, M.B., B.F. El-Rayes, and G.B. Lesinski, Mirage or long-awaited oasis: reinvigorating T-cell responses in pancreatic cancer. J Immunother Cancer, 2020. 8(2).
47. Sackstein, R., T. Schatton, and S.R. Barthel, T-lymphocyte homing: an underappreciated yet critical hurdle for successful cancer immunotherapy. Lab Invest, 2017. 97(6): p. 669-697.
48. Fearon, D.T. and T. Janowitz, AMD3100/Plerixafor overcomes immune inhibition by the CXCL12-KRT19 coating on pancreatic and colorectal cancer cells. Br J Cancer, 2021.
49. Marechal, R., et al., High expression of CXCR4 may predict poor survival in resected pancreatic adenocarcinoma. British Journal of Cancer, 2009. 100(9): p. 1444-1451.
50. Zhang, J.B., et al., Mechanisms by which CXCR4/CXCL12 cause metastatic behavior in pancreatic cancer. Oncology Letters, 2018. 15(2): p. 1771-1776.
51. Wei, S.C., C.R. Duffy, and J.P. Allison, Fundamental Mechanisms of Immune Checkpoint Blockade Therapy. Cancer Discov, 2018. 8(9): p. 1069-1086.
52. He, X. and C. Xu, Immune checkpoint signaling and cancer immunotherapy. Cell Res, 2020. 30(8): p. 660-669.
53. Wen, A.Y., K.M. Sakamoto, and L.S. Miller, The role of the transcription factor CREB in immune function. J Immunol, 2010. 185(11): p. 6413-9.
54. von Euw, E., et al., CTLA4 blockade increases Th17 cells in patients with metastatic melanoma. J Transl Med, 2009. 7: p. 35.
55. Abad, C., et al., Vasoactive intestinal peptide loss leads to impaired CNS parenchymal T-cell infiltration and resistance to experimental autoimmune encephalomyelitis. Proc Natl Acad Sci U S A, 2010. 107(45): p. 19555-60.
56. Colwell, C.S., et al., Disrupted circadian rhythms in VIP- and PHI-deficient mice. Am J Physiol Regul Integr Comp Physiol, 2003. 285(5): p. R939-49.
57. Chaudhury, D., et al., Select cognitive deficits in vasoactive intestinal peptide deficient mice. BMC Neurosci, 2008. 9: p. 63.
58. Pham, T.N.D., et al., Preclinical Models of Pancreatic Ductal Adenocarcinoma and Their Utility in Immunotherapy Studies. Cancers (Basel), 2021. 13(3).
59. Dow, S., A Role for Dogs in Advancing Cancer Immunotherapy Research. Front Immunol, 2019. 10: p. 2935.
60. Abad, C., et al., VPAC1 receptor (Viprl)-deficient mice exhibit ameliorated experimental autoimmune encephalomyelitis, with specific deficits in the effector stage. J Neuroinflammation, 2016. 13(1): p. 169.
61. Moody, T.W., et al., A vasoactive intestinal peptide antagonist inhibits non-small cell lung cancer growth. Proc Natl Acad Sci U S A, 1993. 90(10): p. 4345-9.
62. Sharma, A., et al., A vasoactive intestinal peptide antagonist inhibits the growth of glioblastoma cells. J Mol Neurosci, 2001. 17(3): p. 331-9.
63. Moody, T.W., et al., VIP receptor antagonists inhibit mammary carcinogenesis in C3(1)SV40T antigen mice. Life Sci, 2004. 74(11): p. 1345-57.
64. Moody, T.W., et al., Vasoactive intestinal peptide-camptothecin conjugates inhibit the proliferation of breast cancer cells. Peptides, 2007. 28(9): p. 1883-90.
65. Plonowski, A., et al., Inhibition of PC-3 human prostate cancers by analogs of growth hormone-releasing hormone (GH-RH) endowed with vasoactive intestinal peptide (VIP) antagonistic activity. Int J Cancer, 2002. 98(4): p. 624-9.
66. Zia, H., et al., (N-stearyl, norleucine17) VIP hybrid inhibits the growth of pancreatic cancer cell lines. Life Sci, 2000. 66(5): p. 379-87.
67. Jiang, S., et al., Vasoactive intestinal peptide (VIP) stimulates in vitro growth of VIP-1 receptor-bearing human pancreatic adenocarcinoma-derived cells. Cancer Res, 1997. 57(8): p. 1475-80.
68. Said, S.I. and V. Mutt, Polypeptide with broad biological activity: isolation from small intestine. Science, 1970. 169(3951): p. 1217-8.
69. Delgado, M., et al., VIP and PACAP inhibit IL-12 production in LPS-stimulated macrophages. Subsequent effect on IFN gamma synthesis by T cells. Journal of Neuroimmunology, 1999. 96(2): p. 167-181.
70. Delgado, M., et al., Vasoactive intestinal peptide (VIP) and pituitary adenylate cyclase-activating polypeptide (PACAP) inhibit IL-12 production in LPS-stimulated macrophages. Faseb Journal, 1999. 13(4): p. A320-A320.
71. Smalley, S.G., P.A. Barrow, and N. Foster, Immunomodulation of innate immune responses by vasoactive intestinal peptide (VIP): its therapeutic potential in inflammatory disease. Clin Exp Immunol, 2009. 157(2): p. 225-34.
72. Chorny, A., et al., Vasoactive intestinal peptide induces regulatory dendritic cells that prevent acute graft-versus-host disease while maintaining the graft-versus-tumor response. Blood, 2006. 107(9): p. 3787-3794.
73. Delgado, M., E. Gonzalez-Rey, and D. Ganea, The neuropeptide vasoactive intestinal peptide generates tolerogenic dendritic cells. J Immunol, 2005. 175(11): p. 7311-24.
74. Tsukamoto, M., et al., PD-L1 expression enhancement by infiltrating macrophage-derived tumor necrosis factor-alpha leads to poor pancreatic cancer prognosis. Cancer Sci, 2019. 110(1): p. 310-320.
75. Jansen, C.S., et al., An intra-tumoral niche maintains and differentiates stem-like CD8 T cells. Nature, 2019. 576(7787): p. 465-470.
76. Adams, S., et al., Preoperative concurrent paclitaxel-radiation in locally advanced breast cancer: pathologic response correlates with five-year overall survival. Breast Cancer Res Treat, 2010. 124(3): p. 723-32.
77. Sena, M., et al., High conservation of upstream regulatory sequences on the human and mouse vasoactive intestinal peptide (VIP) genes. DNA Seq, 1994. 5(1): p. 25-9.
78. Komar, H.M., et al., Inhibition of Jak/STAT signaling reduces the activation of pancreatic stellate cells in vitro and limits caerulein-induced chronic pancreatitis in vivo. Sci Rep, 2017. 7(1): p. 1787.
79. Mace, T.A., et al., Pancreatic cancer-associated stellate cells promote differentiation of myeloid-derived suppressor cells in a STAT3-dependent manner. Cancer Res, 2013. 73(10): p. 3007-18.
80. Thavasu, P.W., et al., Measuring cytokine levels in blood. Importance of anticoagulants, processing, and storage conditions. J Immunol Methods, 1992. 153(1-2): p. 115-24.
81. Petersen, C.T., et al., Improving T-cell expansion and function for adoptive T-cell therapy using ex vivo treatment with PI3Kdelta inhibitors and VIP antagonists. Blood Adv, 2018. 2(3): p. 210-223.
82. Nakanishi, T., et al., FISH analysis of 142 EGFP transgene integration sites into the mouse genome. Genomics, 2002. 80(6): p. 564-74.

### B. Sequences

**SEQ ID NO: 1 amino acid sequence for VIPhyb**
   KPRRPYTDNYTRLRKQMAVKKYLNSILN
**SEQ ID NO: 2 amino acid sequence for native vasoactive intestinal peptide (VIP)**
   HSDAVFTDNYTRLRKQMAVKKYLNSILN
**SEQ ID NO: 3 amino acid VIP-R antagonist consensus sequence**
   KPRRPYX¹X²X³X⁴TX⁵LRKQX⁶AVX⁷X⁸KYLX⁹X¹⁰ILN
**SEQ ID NO: 4 amino acid sequence for antagonistic peptide ANT005**
   KPRRPYTDNCTRLRKQMAVKKYLNSILN
**SEQ ID NO: 5 amino acid sequence for antagonistic peptide ANT008**
   KPRRPYTDNYTRLRKQMAVKKYLNLILN
**SEQ ID NO: 6 amino acid sequence for antagonistic peptide ANT058**
   KPRRPYADNYTRLRKQMAVNKYLNLILN
**SEQ ID NO: 7 amino acid sequence for antagonistic peptide ANT105**
   KPRRPYAVNYTRLRKQIAVKKYLMSILN
**SEQ ID NO: 8 amino acid sequence for antagonistic peptide ANT107**
   KPRRPYAVNYTRLRKQMAVNKYLMSILN
**SEQ ID NO: 9 amino acid sequence for antagonistic peptide ANT114**
   KPRRPYADNCTRLRKQIAVNKKYLNSILN
**SEQ ID NO: 10 amino acid sequence for antagonistic peptide ANT195**
   KPRRPYTVNYTSLRKQIAVKKYLMLILN
**SEQ ID NO: 11 amino acid sequence for antagonistic peptide ANT197**
   KPRRPYTDNCTSLRKQIAVNKYLNLILN
**SEQ ID NO: 12 amino acid sequence for antagonistic peptide ANT202**
   KPRRPYAVNCTSLRKQIAVNKYLNSILN
**SEQ ID NO: 13 amino acid sequence for antagonistic peptide ANT203**
   KPRRPYAVNCTSLRKQIAVKKYLMSILN
**SEQ ID NO: 14 amino acid sequence for antagonistic peptide ANT219**
   KKPRRPYTVNCTSLRKQIAVKKYLMLILN
**SEQ ID NO: 15 amino acid sequence for antagonistic peptide ANT300**
   KPRRPYTSDYTRLRKQMAVKKYLNSILN
**SEQ ID NO: 16 amino acid sequence for antagonistic peptide ANT308**
   KPRRPYTSDYTRLRKQMAVKKYLNLILN
**SEQ ID NO: 17 amino acid sequence for scrambled peptide SCRAM1**
   HSDAVFINTKLDKLNTRLVSAQNYMKYR
**SEQ ID NO: 18 amino acid sequence for scrambled peptide SCRAM2**
   KPRRPYINTKLDKLNTRLVSAQNYMKYR
**SEQ ID NO: 19 human CMA1 Accession number GenBank: AAI03975.1:** 
MLLKLKEKASLTLAVGTLPFPSQFNFVPPGRMCRVAGWGRTGVLKPGSDTLQEVKLRL
MDPQACSHFRDFDHNLQLCVGNPRKTKSAFKGDSGGPLLCAGVAQGIVSYGRSDAKPP
AVFTRISHYRPWINQILQAN
**SEQ ID NO: 20 amino acid sequence of human recombinant enkephalinase (neutral endopeptidase, EC 3.4.24.11)** 
DGICKSSDCIKSAARLIQNMDATTEPCTDFFKYACGGWLKRNVIPETSSRYGNFDILRDE
LEVVLKDVLQEPKTEDIVAVQKAKALYRSCINESAIDSRGGEPLLKLLPDIYGWPVATE
NWEQKYGASWTAEKAIAQLNSKYGKKVLINLFVGTDDKNSVNHVIHIDQPRLGLPSRD
YYECTGIYKEACTAYVDFMISVARLIRQEERLPIDENQLALEMNKVMELEKEIANATAK
PEDRNDPMLLYNKMTLAQIQNNFSLEINGKPFSWLNFTNEIMSTVNISITNEEDVVVYAP
 
EYLTKLKPILTKYSARDLQNLMSWRFIMDLVSSLSRTYKESRNAFRKALYGTTSETATW
RRCANYVNGNMENAVGRLYVEAAFAGESKHVVEDLIAQIREVFIQTLDDLTWMDAET
KKRAEEKALAIKERIGYPDDIVSNDNKLNNEYLELNYKEDEYFENIIQNLKFSQSKQLKK
LREKVDKDEWISGAAVVNAFYSSGRNQIVFPAGILQPPFFSAQQSNSLNYGGIGMVIGHE
ITHGFDDNGRNFNKDGDLVDWWTQQSASNFKEQSQCMVYQYGNFSWDLAGGQHLNG
INTLGENIADNGGLGQAYRAYQNYIKKNGEEKLLPGLDLNHKQLFFLNFAQVWCGTYR
PEYAVNSIKTDVESPGNFRIIGTLQNSAEFSEAFHCRKNSYMNPEKKCRVW
SEQ ID NO: 21
   KPRRPYX¹X²X³X⁴TX⁵LRKQX⁶AVX⁷KYLX⁸X⁹ILN
SEQ ID NO: 22
   GGGGSC

## Claims

1. A vasoactive intestinal peptide receptor (VIP-R) antagonist comprising the amino acid sequence: KPRRPYTSDYTRLRKQMAVKKYLNLILN (SEQ ID NO: 16),
optionally wherein an amino, carboxyl, hydroxyl, or thiol group in the peptide is substituted and/or wherein the peptide is conjugated to and/or encapsulated within a nanoparticle.

2. A pharmaceutical composition comprising the vasoactive intestinal peptide receptor (VIP-R) antagonist of Claim 1 and a pharmaceutically acceptable carrier.

3. A nucleic acid encoding the amino acid sequence of a vasoactive intestinal peptide receptor (VIP-R) antagonist of Claim 1.

4. A method of *ex vivo* augmenting T cell activation and/or expansion, comprising mixing one or more T cells with the vasoactive intestinal peptide receptor (VIP-R) antagonist of Claim 1 or the pharmaceutical composition of Claim 2;
optionally wherein mixing the one or more T cells is in combination with an anti-CD3 antibody, an anti-CD28 antibody, a phosphatidylinositol 3-kinase (PI3K) inhibitor, and/or an immune checkpoint blockade.

5. An *in vitro* cell culture composition, comprising
one or more T cells, and
the vasoactive intestinal peptide receptor (VIP-R) antagonist of Claim 1 or the pharmaceutical composition of Claim 2;
optionally wherein the *in vitro* cell culture composition further comprises an anti-CD3 antibody, an anti-CD28 antibody, a phosphatidylinositol 3-kinase (PI3K) inhibitor, and/or an immune checkpoint blockade.

6. The method of *ex vivo* augmenting T cell activation and/or expansion of Claim 4 or *in vitro* cell culture composition of Claim 5, wherein the PI3K inhibitor is a PI3Kα inhibitor, a PI3Kβ inhibitor, a PI3Kδ inhibitor, or a PI3Kγ inhibitor;
preferably wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.

7. The method of *ex vivo* augmenting T cell activation and/or expansion of Claim 4 or 6, or the *in vitro* cell culture composition of Claim 5 or 6, wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-1-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor.

8. A therapeutically effective amount of the vasoactive intestinal peptide receptor (VIP-R) antagonist of Claim 1 or the pharmaceutical composition of Claim 2 for use in treating a microbial infection in a subject;
preferably wherein the microbial infection is a viral infection, a bacterial infection, a fungal infection, and/or a parasitic infection.

9. A therapeutically effective amount of the vasoactive intestinal peptide receptor (VIP-R) antagonist of Claim 1 or the pharmaceutical composition of Claim 2 for use in treating a cancer and/or metastasis;
preferably wherein the cancer comprises pancreatic cancer, colon cancer, leukemia, liver cancer, lung cancer, or melanoma.

10. The therapeutically effective amount of the VIP-R antagonist or the pharmaceutical composition for use of Claim 8 or 9, wherein a therapeutically effective amount of a phosphatidylinositol 3-kinase (PI3K) inhibitor is for administration to the subject;
preferably wherein the PI3K inhibitor is a PI3Kα inhibitor, a PI3Kβ inhibitor, a PI3Kδ inhibitor, or a PI3Kγ inhibitor;
most preferably wherein the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.

11. The therapeutically effective amount of the VIP-R antagonist or the pharmaceutical composition for use of any one of Claims 8-10, wherein a therapeutically effective amount of an immune checkpoint blockade is for administration to the subject;
preferably wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-1-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor;
most preferably wherein the immune checkpoint blockade comprises pembrolizumab, nivolumab, cemiplimab, dostarlimab, atezolizumab, avelumab, durvalumab, or ipilimumab.

12. A therapeutically effective amount of the vasoactive intestinal peptide receptor (VIP-R) antagonist of Claim 1 or the pharmaceutical composition of Claim 2 for use in enhancing an immune response to a cancer and/or metastasis;
preferably wherein the cancer comprises pancreatic cancer, colon cancer, leukemia, liver cancer, lung cancer, or melanoma.

13. The therapeutically effective amount of the VIP-R antagonist or the pharmaceutical composition for use of Claim 12, wherein a therapeutically effective amount of a phosphatidylinositol 3-kinase (PI3K) inhibitor is for administration to the subject;
preferably wherein the PI3K inhibitor is a PI3Kα inhibitor, a PI3Kβ inhibitor, a PI3Kδ inhibitor, or a PI3Kγ inhibitor;
most preferably wherein the the PI3K inhibitor comprises idelalisib, copanlisib, duvelisib, alpelisib, umbralisib, buparlisib, copanlisib, dactolisib, leniolisib, parsaclisib, paxalisib, taselisib, zandelisib, inavolisib, apitolisib, bimiralisib, eganelisib, fimepinostat, gedatolisib, linperlisib, nemiralisib, pilaralisib, samotolisib, seletalisib, serabelisib, sonolisib, tenalisib, voxtalisib, AMG 319, AZD8186, GSK2636771, SF1126, acalisib, omipalisib, AZD8835, CAL263, GSK1059615, MEN1611, PWT33597, TG100-115, or ZSTK474.

14. The therapeutically effective amount of the VIP-R antagonist or the pharmaceutical composition for use of Claim 12 or 13, wherein a therapeutically effective amount of an immune checkpoint blockade is for administration to the subject;
preferably wherein the immune checkpoint blockade comprises a PD-1 inhibitor, a PD-L1 inhibitor, a CTLA-4 inhibitor, an anti-TIM3 inhibitor, an anti-LAG3 inhibitor, an anti-CD47 inhibitor, imiquimod, polyinosinic-polycytidylic acid-poly-1-lysine carboxymethylcellulose (poly-ICLC) , pexidartinib, an anti-TIGIT inhibitor, an anti-B7-H3 inhibitor, an anti-B7-H4 inhibitor, an anti-A2aR inhibitor, an anti-CD73 inhibitor, an anti-NKG2A inhibitor, an anti-PVRIG/PVRL2 inhibitor, an anti-CEACAM1 inhibitor, an anti-CEACAM5 inhibitor, an anti-CEACAM6 inhibitor, an focal adhesion kinase (FAK) inhibitor, a CCL2/CCR2 inhibitor, an anti-leukemia inhibitory factor (LIF) inhibitor, an anti-CD47/SIRPα inhibitor, an anti-colony-stimulating factor (CSF)-1 inhibitor, an anti-IL-1 inhibitor, an anti-IL-1R3 inhibitor, an anti-IL-8 inhibitor, an anti-semaphorin 4D (Sema4D) inhibitor, an angiopoietin(Ang)-2 inhibitor, a CLEVER-1 inhibitor, Axl-targeted enapotamab vedotin (EnaV), or an anti-phosphatidylserine inhibitor;
most preferably wherein the immune checkpoint blockade comprises pembrolizumab, nivolumab, cemiplimab, dostarlimab, atezolizumab, avelumab, durvalumab, or ipilimumab.
